(19) Europäisches Patentamt
European Patent Office
Office européen des brevets

(11) EP 3 225 590 A1

(12) EUROPEAN PATENT APPLICATION

(43) Date of publication:
**04.10.2017 Bulletin 2017/40**

(51) Int Cl.:
***C01B 39/02*** (2006.01) ***G01N 21/63*** (2006.01)
***G01N 33/531*** (2006.01) ***G01N 33/58*** (2006.01)

(21) Application number: **16305378.8**

(22) Date of filing: **31.03.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Université de Strasbourg**
  **67000 Strasbourg (FR)**
- **CENTRE NATIONAL DE LA RECHERCHE SCIENTIFIQUE -CNRS-**
  **75794 Paris Cedex 16 (FR)**

(72) Inventors:
- **BIEDERMANN, Frank**
  **76137 KARLSRUHE (DE)**
- **SEPTIADI, Dedy**
  **1700 FRIBOURG (CH)**
- **PRASETYANTO, Eko Adi**
  **67000 STRASBOURG (FR)**
- **CHEN, Pengkun**
  **67000 STRASBOURG (FR)**
- **DE COLA, Luisa**
  **67000 STRASBOURG (FR)**

(74) Representative: **Novagraaf Technologies**
**Bâtiment O2**
**2, rue Sarah Bernhardt**
**CS90017**
**92665 Asnières-sur-Seine Cedex (FR)**

# (54) HIGHLY SELECTIVE ARTIFICIAL NEUROTRANSMITTER RECEPTORS

(57) The present invention relates to a conjugate comprising a nanoporous crystalline three-dimensional framework material bearing a permament negative framework charge and having an average pore size ranging from 1 nm to 2 nm, selected from a zeolite, an organosilica, a metal organic framework (MOF) and a covalent organic framework (COF) material, containing within its pores an electrophilic UV-visible or fluoresecent analyte-responsive reporter dye, a method for producing the same, and uses thereof.

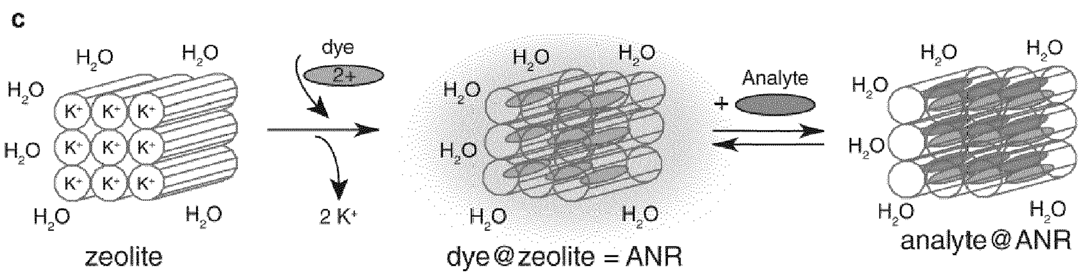


**FIGURE 3**

EP 3 225 590 A1

## Description

### Field of the Invention

The present invention relates to a conjugate comprising a nanoporous crystalline three-dimensional framework material bearing a permament negative framework charge and having an average pore size ranging from 1 nm to 2 nm, selected from a zeolite, an organosilica, a metal organic framework (MOF) and a covalent organic framework (COF) material, containing within its pores an electrophilic UV-visible or fluoresecent analyte-responsive reporter dye, a method for producing the same, and uses thereof.

### Background of the Invention

Despite decades of extensive efforts, the design of artificial receptors still remains as an open challenge in the field of supramolecular chemistry. Hence, alternative strategies are needed to complement protein-based receptors with robust and cheap artificial counterparts. Recent applications of nanoscopic scaffolds and sensitive spectroscopic techniques have enabled the conception of sensing devices for neurotransmitters.[1-4] Unfortunately, the problem of the selectivity and affinity, in particular for the use in water or complex biological media, persists.

An ideal artificial receptor should operate in a fast and reversible binding mode in order to allow for non-destructive *in situ* detection of neurotransmitters. However, the non-covalent complexation of small organic molecules in aqueous media remains extremely challenging on account of the attenuation of directional non-covalent interactions such as hydrogen bonds by solvent water molecules. Nature has overcome these obstacles by ensuring a high binding-affinity through release of energetically frustrated water molecules from hydrophobic and confined protein cavities, while simultaneously defining the selectivity for a particular ligand through well positioned ligand-binding units (Fig. 1a). For instance, the binding pocket of the human D3-type dopamine-receptor is confined, hydrophobic and decorated with negatively charged amino acid residues in order to form salt-bridges with the positively charged ammonium moiety of dopamine.

At present, there is therefore a need for the development of functional, robust, and readily applicable artificial neurotransmitter receptor analogues that show outstanding affinities and selectivities.

### BRIEF DESCRIPTION OF THE DRAWING

**Figure 1** | Binding and signal transduction strategies for natural neurotransmitter receptors and the herein presented artificial receptor analogue. **a,** Schematic concept illustrating the neurotransmitter binding to protein-receptors. The process is favoured by release of cavity water and the formation of salt-bridges between the protein and the ligand. The binding event is transcribed into a biological signal through conformational changes of the receptor. **b,** The biomimetic approach investigated: inorganic nanoporous material contain high-energy water whose displacement by an analyte is energetically favourable. The negatively charged channels of zeolite materials cause a charge-mediated binding preference for cationic analytes, e.g. aromatic amine neurotransmitters. A sensitive signal transduction is accomplished through co-inclusion of an aryl-moiety-responsive, fluorescent reporter dye into zeolite L or Y. **c-d,** Selected chemical structures of aromatic amine neurotransmitters/drugs that can be detected by the ANRs and representative dyes employed. **e,** Intensity coded-confocal micrographs of an aqueous suspension of, tailored made for this experiments, large **ANR-L1** crystals, prior (left) and after (right) addition of an excess of dopamine. Binding of the neurotransmitter clearly quenches the fluorescence intensity. The scale bar is 5 μm.

**Figure 2** | Emission response of ANR-L2 and ANR-Y2 towards the addition of neurotransmitters and other analytes. **a,** Normalized emission and excitation spectra of dye **D2** and of its corresponding receptors **ANR-L2** and **ANR-Y2** in water. **b,** Emission spectra of **ANR-L2** (25 μg/mL) upon addition of serotonin. **c**, Enhancement of the fluorescence emission of **ANR-Y2** (250 μg/mL) upon addition of histamine. **d-e,** Binding isotherms for **ANR-L2** (250 μg/mL) and indole-derivatives **(d)** or catechol amines **(e).** See Fig. 1c for their chemical structures. All titration experiments were carried out in aqueous HEPES buffer (10 mM, pH 7.3) and with an excitation wavelength of $\lambda_{exc}$ = 475 nm. ANRs were prepared from commercial, small-size zeolites (L-type 50 nm; Y-type 900 nm).

**Figure 3** | Chemical structures of a, the dyes and b, the analytes tested in this study. c, Schematic representation of a, ANR preparation through dye-sorption of zeolite crystals and b, subsequent emission quenching through binding of an electron-rich, aromatic amine analyte.

Excitation and Emission Spectra of the ANRs

**Figure 4** | Normalized emission and excitation spectra of a, dye D1 and its corresponding receptors ANR-L1 (based on zeolite LTL), and ANR-Y1 (based on zeolite Y), and b, for dye D2 and its corresponding receptors ANR-L2 (based on zeolite LTL), and ANR-Y2 (based on zeolite Y).

**Figure 5** | Normalized emission and excitation spectra of a, dye D1 and b, dye D3 and of their corresponding receptors ANR-L1 and ANR-L3 when dispersed in water or in chloroform.

**Figure 6** | Normalized emission and excitation spectra of a, dye D4 and of its corresponding receptor ANR-L4 in water, and b, of dye D5 and of its corresponding receptor ANR-L5 in aqueous HEPES buffer. Fluorescence Titration Experiments and Binding Curves

**Figure 7** | Emission spectra and binding curve for the titration of ANR-Y1 (250 μg/mL) with serotonin in 10 mM HEPES buffer (pH 7.3). a, Addition of a solution of serotonin to a suspension of ANR-Y1 results in a quenching of the emission of ANR-Y1. b, Fit of the relative emission intensity with a 1:1 binding site model yields a single site dissociation constant $K_d$ = 70·nM. The excitation wavelength $\lambda_{exc}$ = 395 nm was used.

**Figure 8** | Emission spectra and binding curve for the titration of ANR-L1 (250 μg/mL) with serotonin in 10 mM HEPES buffer (pH 7.3). a, Addition of a solution of serotonin to a suspension of ANR-L1 results in a quenching of the emission of ANR-L1. b, Fit of the relative emission intensity with a 1:1 binding site model yields a single site dissociation constant $K_d$ = 3.3·μM. The excitation wavelength $\lambda_{exc}$ = 395 nm was used.

**Figure 9** | Emission spectra and binding curve for the titration of ANR-Y2 (250 μg/mL) with dopamine in 10 mM HEPES buffer (pH 7.3). a, Addition of a solution of dopamine to a suspension of ANR-Y2 results in a quenching of the emission of ANR-Y2. b, Fit of the relative emission intensity with a 1:1 binding site model yields a single site dissociation constant $K_d$ = 9.5·μM. The excitation wavelength $\lambda_{exc}$ = 450 nm was used.

**Figure 10** | Emission spectra for the titration of ANR-L4 (250 μg/mL) with a, tyramine and b, tyrosine. The experiments were carried out in 10 mM HEPES buffer with the excitation wavelength $\lambda_{exc}$ = 300 nm.

**Figure 11** | Emission spectra for the titration of ANR-L4 (250 μg/mL) with a, dopamine and b, catechol. The experiments were carried out in 10 mM HEPES buffer with the excitation wavelength $\lambda_{exc}$ = 300 nm.

**Figure 1** | Response of ANR-L3 (250 μg/mL) and ANR-L5 (250 μg/mL) to the presence of a PET-quencher and a non-quencher. a, ANR-L3 ($\lambda_{exc}$ = 445 nm) after addition of 85 μM dopamine and b, after addition of 120 μM histamine. c, ANR-L5 ($\lambda_{exc}$ = 350 nm) after addition of 500 μM dopamine and d, after addition of 500 μM histamine. The experiments were carried out in 10 mM HEPES buffer.

**Figure 13** | Emission spectra for the titration of ANR-Y2 (250 μg/mL) with a, phenethylamine and b, nicotine in 10 mM HEPES buffer (pH 7.3). The excitation wavelength $\lambda_{exc}$ = 395 nm was used.

**Figure 14** | Emission response of ANR-L2 (2.5 μg/mL) upon addition of serotonin. Error bars were calculated from 3 repetition experiments. The experiments were carried out in aqueous HEPES buffer (10 mM, pH 7.3) and with an excitation wavelength of $\lambda_{exc}$ = 475 nm.

**Figure 15** | Binding curves for the titration of ANR-Y1 (250 μg/mL) with indole, melatonin, tryptophane, tryptamine, serotonin, $TrpNH_2$, and TrpGly in 10 mM HEPES buffer (pH 7.3). The solid lines represent the least-square fits to a single-site 1:1 binding site model. The excitation wavelength $\lambda_{exc}$ = 395 nm was used.

**Figure 16** | Binding curves for the titration of ANR-L1 (250 μg/mL) with indole, melatonin, tryptophan, tryptamine, serotonin, $TrpNH_2$, and TrpGly in 10 mM HEPES buffer (pH 7.3). The solid lines represent the least-square fits to a single-site 1:1 binding site model. The excitation wavelength $\lambda_{exc}$ = 395 nm was used.

**Figure 17** | Binding curves for the titration of ANR-Y2 and ANR-L2 (each at 250 μg/mL) with indole, tryptophan, $TrpNH_2$, tryptamine and serotonin in 10 mM HEPES buffer (pH 7.3). The solid lines represent the least-square fits

to a single-site 1:1 binding site model. The excitation wavelength $\lambda_{exc}$ = 395 nm was used.

**Figure 18** | Binding curves for the titration of ANR-L3 (250 μg/mL) with indole, tryptophan, $TrpNH_2$, tryptamine and serotonin in 10 mM HEPES buffer (pH 7.3). The solid lines represent the least-square fits to a single-site 1:1 binding site model. The excitation wavelength $\lambda_{exc}$ = 445 nm was used.

**Figure 19** | Binding curves for the titration of ANR-L1 (250 μg/mL) with a, serotonin and b, tryptmaine, each in 10 mM HEPES buffer (pH 7.3) to which were added 50 mM tryptophan. The excitation wavelength $\lambda_{exc}$ = 400 nm was used. The solid lines represent the least-square fits to a single-site 1:1 binding site model. shows The binding strength ($K_d$ = 14 μM) of serotonin in the presence of 50 mM Trp is only by a factor ~4 weaker than in the absence of the potential competitor Trp. Similarly, the also the binding of tryptamine ($K_d$ = 27 μM) and dopamine ($K_d$ = 48 μM) is still very strong in the presence of 50 mM Trp, and only by a factor ~3 weaker than in the absence of the potential competitor Trp (See Table 1 for comparison).

**Figure 20** | Binding curves for the titration of a, ANR-Y1 and b, ANR-Y2 (each at 250 μg/mL) with phenylephrine, epinephrine, norepinephrine and dopamine in 10 mM HEPES buffer (pH 7.3). The solid lines represent the least-square fits to a single-site 1:1 binding site model. The excitation wavelength a, $\lambda_{exc}$ = 395 nm and b, $\lambda_{exc}$ = 442 nm was used.

**Figure 21** | Binding curves for the titration of a, ANR-L1 and b, ANR-L2 (each at 250 μg/mL) with phenylephrine, epinephrine, norepinephrine and dopamine in 10 mM HEPES buffer (pH 7.3). The solid lines represent the least-square fits to a single-site 1:1 binding site model. The excitation wavelength a, $\lambda_{exc}$ = 395 nm and b, $\lambda_{exc}$ = 442 nm was used.

**Figure 22** | Binding curves for the titration of a, ANR-L3 and b, ANR-L4 (each 250 μg/mL) with phenylephrine, epinephrine, norepinephrine and dopamine in 10 mM HEPES buffer (pH 7.3). The solid lines represent the least-square fits to a single-site 1:1 binding site model. The excitation wavelength a, $\lambda_{exc}$ = 445 nm and b, $\lambda_{exc}$ = 300 nm was used.

**Figure 23** | Binding curves for the titration of a, ANR-Y1 and b, ANR-Y2 (each at 250 μg/mL) with catechol, octopamine, tyramine and dopamine in 10 mM HEPES buffer (pH 7.3). The solid lines represent the least-square fits to a single-site 1:1 binding site model. The excitation wavelength a, $\lambda_{exc}$ = 395 nm and b, $\lambda_{exc}$ = 442 nm was used.

**Figure 24** | Binding curves for the titration of a, ANR-L1 and b, ANR-L2 (each at 250 μg/mL) with octopamine, tyramine, dopamine and catechol in 10 mM HEPES buffer (pH 7.3). The solid lines represent the least-square fits to a single-site 1:1 binding site model. The excitation wavelength a, $\lambda_{exc}$ = 395 nm and b, $\lambda_{exc}$ = 442 nm was used.

**Figure 25** | Binding curves for the titration of a, ANR-L3 and b, ANR-L4 (each 250 μg/mL) with octopamine, tyramine, dopamine and catechol in 10 mM HEPES buffer (pH 7.3). The solid lines represent the least-square fits to a single-site 1:1 binding site model. The excitation wavelength a, $\lambda_{exc}$ = 445 nm and b, $\lambda_{exc}$ = 300 nm was used.

Ratiometric Sensing Experiments

**Figure 26** | Ratiometric sensing with ANR-L1 that contains a co-encapsulated, analyte-non-responsive reference dye DXP. a, Emission spectra of ANR-L1-DXP (=DXP loaded Linde-type zeolite L that was subsequently loaded with D1) (250 μg/mL) upon addition of serotonin. b, Control: Emission spectra of DXP loaded Linde-type zeolite L (250 μg/mL) upon addition of serotonin. c, Ratiometric plot for the titration experiment shown in part b of this figure. d, Ratiometric plot for titration experiments at lower ANR-L1-DXP concentration ((25 μg/mL), allowing for detection of lower concentrations of serotonin. These experiments were carried out in triple-fold and the error bars are given. All experiments were carried out in 10 mM HEPES buffer and the excitation wavelength $\lambda_{exc}$ = 360 nm was used.

Pattern Recognition Analysis

**Figure 27** | Principal component analysis (PCA) of the binding constants ($K_a = K_d^{-1}$) of aromatic amine neurotrans-

mitters and trace amines with ANR-L1, ANR-Y1, ANR-L2, ANR-Y2 and ANR-L3. a, The red dots represent the scores generated from the actual binding constants values (shown in b), while the crosses show typical statistic variations if to each binding constant a random error of up to ±20% was addded, i.e. $-0.2*K_a \leq$ error $\leq 0.2*K_a$. Despite this liberal assumption on the error, most analytes can be readily distinguished from each other through the principal component analysis. See also Figure for the further distinction of all catechol amine neurotransmitters and corresponding trace amines. For the blank samples, which is representative for weakly binding analytes, an upper-limit $K_a$ value of 1000 $M^{-1}$ was used for the interaction with each of the receptors.

**Figure 28** | Principal component analysis (PCA) of the binding constants ($K_a = K_d^{-1}$) of catecholamine neurotransmitters and trace amines with ANR-L1, ANR-Y1, ANR-L2 and ANR-Y2. a, The red dots represent the scores generated from the actual binding constants values (shown in b), while the crosses show typical statistic variations if to each binding constant a random error of up to ±20% was addded, i.e. $-0.2*K_a \leq$ error $\leq 0.2*K_a$. Despite this liberal assumption on the error, most analytes can be readily distinguished from each other through the principal component analysis. For the blank samples, which is representative for weakly binding analytes, an upper-limit $K_a$ value of 1000 $M^{-1}$ was used for the interaction with each of the receptors.

**Figure 29** | Principal component analysis (PCA) for the differentiation of dopamine, norepinephrine, serotonin and histamine by changes in the intensity of ANR-L1, ANR-L2 and ANR-L3 (each at 125 μg / mL in 10 mM HEPES buffer). a, Normalized changes in the emission intensity, $I_{rel} = (I_0 - I_{analyte}) / I_0$, in the presence of 39 μM neurotransmitter. The experiments were carried out in triple fold, but only average $I_{rel}$ values are shown. b, Principal component analysis of the $I_{rel}$ taking into consideration the addition of 8 μM ($I_{rel}$ values not shown) and of 39 μM ($I_{rel}$ values shown in a) neurotransmitter. The experiments were carried out in triple repetition. The typical excitation and emission wavelengths for the ANRs were used, see the section "Fluorescence Titration Experiments and Binding Curves" above for representative examples.

Confocal Microscopy Experiments

**Figure 30** | Fluorescence, brightfield and intensity coded-confocal micrographs showing self-agglomerated ANR-Y1 in culture media solution. a, Before, b, after addition of serotonin, and c, after subsequent addition of histamine. Images were acquired at $\lambda_{exc}$ = 405 nm. Comparison of the intensities in b and c shows, that a partial displacement of serotonin from the ANR-L1 by histamine is possible.

**Figure 31** | Fluorescence, brightfield and intensity coded-confocal micrographs showing self-agglomerated ANR-Y2 in culture media solution. a, Before, b, after addition of serotonin. Images were acquired at $\lambda_{exc}$ = 488 nm.

**Figure 32** | a, Intensity coded-confocal micrographs showing self-agglomerated ANR-L1 before (a), after addition of serotonin (b), and after subsequent addition of histamine (c). Comparison of the intensities in b and c shows, that a partial displacement of serotonin from the ANR-L1 by histamine is possible.

**Figure 33** | Fluorescence, brightfield and overlayed micrographs showing Rattus norvegicus C6 glioma cells with internalized poly(lysine)-coated ANR-L1 after 24h of incubation. Note that the ANR remains emissive, e.g. is not quenched by biological components occurring in the cell. Note further, that no dye-leakage of dicationic dye D1 from the ANR into the nucleus of the cells was witnessed, indicating that the ANRs are stable. Images were acquired at $\lambda_{exc}$ = 405 nm.

**Figure 34** | Fluorescence-based monitoring of the intracellular release of serotonin as a model drug. a) Cellular uptake of drug-loaded and surface-coated ANRs results after release of the drug in an increase in the fluorescence emission of the ANR. b) Intensity coded-confocal micrographs showing cellular uptake experiment of the serotonin-loaded and poly(lysine)-coated ANR-L1 with Rattus norvegicus C6 glioma cells at different incubation times 1h (left), 4h (center), and 24h (right panel), and c) their corresponding brightfield images. Images were acquired at $\lambda_{exc}$ = 405 nm. The scale bar is 20 μm.

Monitoring of a Label-free Enzymatic Reaction in Real time

**Figure 35** | Real-time monitoring of serotonin oxidation with $O_2$ catalyzed by the enzyme laccase through changes in the emission intensity of PEGylated ANR-L1 (250 μg/mL). Note that the small drift of $I_{rel}$ in the control experiments without enzyme or substrate (red and black traces) are caused by the slow sedimentation of ANR-L1-PEG over the time course of several minutes in a standing cuvette. If required, this drift can be avoided by a constantly stirring the reaction mixture. The experiments were carried out in 10 mM HEPES buffer that was adjusted with HCl to pH 4 and the excitation wavelength $\lambda_{exc}$ = 405 nm was used.

Fluorescence Titration Experiments for ANRs in Organic Solvents

**Figure 36** | Emission spectra and binding curves for the titration of ANR-L1 (250 μg/mL) with tryptamine in the organic solvent mixture chloroform:acetic acid (99:1). a, Addition of a solution of tryptamine in chloroform to a suspension of ANR-L1 in the chloroform:acetic acid (99:1) mixture results in the typical quenching of the emission of ANR-L1. Note that acetic acid was added in order to ensure a full protonation of the tryptamine analyte in the presence of the ANR (For solubility reasons, the free base and not the hydrochloride was used for preparing a 1mM stock solution of tryptamine in chloroform). b, Fit of the relative emission intensity with a 1:1 binding site model yields a single site dissociation constant $K_d$ = 3·μM. The excitation wavelength $\lambda_{exc}$ = 395 nm was used.

**Figure 37** | Emission spectra and binding curves for the titration of ANR-L3 (250 μg/mL) with tryptamine in the organic solvent mixture chloroform:acetic acid (99:1). a, Addition of a solution of tryptamine in chloroform to a suspension of ANR-L3 in the chloroform:acetic acid (99:1) mixture results in the typical quenching of the emission of ANR-L3. Note that acetic acid was added in order to ensure a full protonation of the tryptamine analyte in the presence of the ANR (For solubility reasons, the free base and not the hydrochloride was used for preparing a 1mM stock solution of tryptamine in chloroform. ). b, Fit of the relative emission intensity with a 1:1 binding site model yields a single site dissociation constant $K_d$ = 4·μM. A slightly weaker binding occurs ($K_d$ = 15·μM) when the experiments were conducted in neat chloroform, i.e. the absence of acetic acid (data not shown). The excitation wavelength $\lambda_{exc}$ = 445 nm was used in all experiments.

**Figure 38** | Emission spectra and binding curves for serotonin and ANR-L1 (250 μg/mL) in aqueous and ethanolic media. a, Addition of a solution of serotonin*HCl to a suspension of ANR-L1, both in ethanol with 0.2 mM acetic acid. b, Fit of the relative emission intensity with a 1: 1 binding site model yields a single site dissociation constant $K_d$ ~ 3 μM in water and aqueous buffer, and $K_d$ ~ 25·μM in ethanolic media. The excitation wavelength $\lambda_{exc}$ = 396 nm was used in all experiments.

UV/vis Spectra of the ANRs

**Figure 39** | Solid state UV/vis spectra for ANR-L1 in the absence of analyte and in the presence of a, dopamine, or b, serotonin, against a background of $BaSO_4$. The spectra of ANR-L1 and analyte@ANR-L1 were normalized to equal absorbance at 425 nm, which was found to be a near isosbestic point for D1 in the presence of electron-rich aromatic guests and an organic host. **[32]**

**Figure 40** | Solid state UV/vis spectra for ANR-Y1 in the absence of analyte and in the presence of a, dopamine, or b, serotonin, against a background of $BaSO_4$. The spectra of ANR-Y1 and analyte@ANR-Y1 were normalized to equal absorbance at 425 nm.

**Figure 41** | Solid state UV/vis spectra for a, ANR-L2 and b, ANR-Y2 in the absence and presence of dopamine against a background of $BaSO_4$. The spectra of ANR-L2, ANR-Y2, dopamine@ANR-Y2 and dopamine@ANR-Y2 were normalized to equal absorbance at 420 nm.

**Figure 42** | Solid state UV/vis spectra for ANR-L3 in the absence of analyte and in the presence of a, dopamine, or b, serotonin, against a background of $BaSO_4$. The spectra were normalized to equal absorbance at 426 nm, which was found to be a near isosbestic point for D3 in the presence of electron-rich aromatic guests and an organic host. **[23]**

**Figure 43** | SEM measurement (left) and XRD analysis (right) of custom-made, large, barrel-shaped zeolite L crystals.

**Figure 44** | top: SEM images of zeolite L before (left) and after (right) uptake of dye D1. bottom: TEM image of zeolite L prior to dye uptake.

**Figure 45** | SEM images of zeolite Y before (left) and after (right) uptake of dye D1.

**Figure 46** | SEM image of ANR-L1-PEG (left), and of TGA analysis of this material in comparison to that of the parent zeolite L and derived ANR-L1.

**Figure 47** | Stern-Volmer plot for a, ANR-Y1 with dopamine and b, ANR-Y2 with dopamine. The approximated dissociation constants ($K_d$ = 1/slope) of 4 μM and 10 μM, respectively, are qualitatively in good agreement with the ones obtained by the preferred non-linear least square fit, see Tables 1 and 3.

**Figure 48** | Stern-Volmer plot for a, ANR-L1 with serotonin and b, ANR-L2 with serotonin. The approximated dissociation constants ($K_d$ = 1/slope) of 4 μM and 2 μM, respectively, are qualitatively in good agreement with the ones obtained by the preferred non-linear least square fit, see Tables 1 and 3.

**Figure 49** | Stern-Volmer plot for a, ANR-L3 with dopamine and b, ANR-L4 with dopamine. The approximated dissociation constants ($K_d$ = 1/slope) of 15 μM and 7 μM, respectively, are qualitatively in good agreement with the ones obtained by the preferred non-linear least square fit, see Tables 1 and 3.

Sensing through covalent binding

**Figure 50** | Response of a thiol-reactive dye to the presence of **a)** cysteamine, and **b)** L-cysteine.

**Table 3** | Fitted single site dissociation constants ($K_d$) for the binding of zeolite L-based ANR-L2 and zeolite Y-based ANR-Y2 (each at 250 $\mu$g/mL in 10 mM HEPES buffer, pH 7.3) with representative analytes

$K_d$ values with the >> sign denote less than 10% of emission quenching at >50 molar excess of analyte. Errors in $K_d$, determined by repetitive experiments and considering concentrations errors are expected to be not larger than $\pm$20%.

**[0016]** * The electron-poor analyte phenethylamine and histamine do not quench the emission of ANR-L2 and ANR-Y2. Conversely, the addition of phenethylamine and histamine to ANR-Y2, ANR-L3 and ANR-L5 causes an increase in the emission intensity through dye-aggregation, while nicotine gave no appreciable effect at 100 $\mu$M concentration. The $K_d$ values for ANR-L3 (250 $\mu$g/mL in 10 mM HEPES buffer, pH 7.3) with phenethylamine and histamine are 190 $\mu$M and 360 $\mu$M, respectively. Furthermore, phenethylamine partially quenches the emission of ANR-L4 (residual emission int. 75% at 25 $\mu$M of analyte, $K_d$ = 19 $\mu$M). All other herein shown aryl-moiety containing analytes are known to quench the emission of D2 when held in close spatial proximity to the dye. **[Ref 23]** Biedermann et al., Angew. Chem. Int. Ed., 5694-5699 (2014)

**Table 4** | Fitted single site dissociation constants ($K_d$) for the binding of zeolite LTL-based ANR-L2 (250 $\mu$g/mL in 10 mM HEPES buffer, pH 7.3) with representative analytes.

$K_d$ values with the >> sign denote less than 10% of emission quenching at >100 molar excess of analyte. Errors in $K_d$, determined by repetitive experiments and considering concentrations errors are expected to be not larger than $\pm$20%.

**[0019]** * Tyramine quenches the emission of ANR-L3 by only 30%, but the titration curve shows saturation behaviour.

** The electron-poor analytes phenethylamine and histamine cause an increase in the emission intensity of ANR-L3 through dye-aggregation.

**Table 5** | Response of the emission ANR-L4 (250 $\mu$g/mL in 10 mM HEPES buffer, pH 7.3) to representative analytes.

$K_d$ values with the >> sign denote less than 10% of emission quenching at >100 molar excess of analyte. Errors in $K_d$, determined by repetitive experiments and considering concentrations errors are expected to be not larger than $\pm$20%.

**[0023]** * The electron-poor analyte histamine cannot quench the emission of ANR-L4. All other herein shown aryl-moieties are known to quench the emission of D4 when held in close spatial proximity to the dye. **[ref 7]**

The autofluorescence of serotonin and tryptamine in the spectral region of the emission of ANR-L4 has prevented the investigation of their binding to ANR-L4.

**Table 6** | Emission quantum yields (QY) for ANRs and corresponding dyes in water. Emission quantum yields were determined at the excitation wavelength given by adjusting the concentration of dye or ANR to yield absorbance values of approx. 0.3 to 0.5 a.u. Solid dopamine was subsequently added to yield a total concentration of about 1 mM.

**Table 7** | Zeta potential of ANRs in PBS buffer (IX, ChemCruz) and their average size, determined by SEM.

**DEFINITIONS**

To facilitate an understanding of the present invention, a number of terms and phrases are defined below:

As used herein other than the claims, the terms "a," "an," "the," and/or "said" means one or more. As used herein in the claim(s), when used in conjunction with the words "comprise," "comprises" and/or "comprising," the words "a," "an," "the," and/or "said" may mean one or more than one. As used herein and in the claims, the terms "having," "has," "is," "have," "including," "includes," and/or "include" has the same meaning as "comprising," "comprises," and "comprise." As used herein and in the claims "another" may mean at least a second or more. As used herein and in the claims, "about" refers to any inherent measurement error or a rounding of digits for a value (e.g., a measured value, calculated value such as a ratio), and thus the term "about" may be used with any value and/or range.

The phrase "a combination thereof' "a mixture thereof" and such like following a listing, the use of "and/or" as part of a listing, a listing in a table, the use of "etc" as part of a listing, the phrase "such as," and/or a listing within brackets with "e.g.," or i.e., refers to any combination (e.g., any sub-set) of a set of listed components, and combinations and/or mixtures of related species and/or embodiments described herein though not directly placed in such a listing are also contemplated. Such related and/or like genera(s), sub-genera(s), specie(s), and/or embodiment(s) described herein are

contemplated both in the form of an individual component that may be claimed, as well as a mixture and/or a combination that may be described in the claims as "at least one selected from," "a mixture thereof" and/or "a combination thereof."

In general, the term "substituted" whether preceded by the term "optionally" or not, and substituents contained in formulae of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds.

As used herein, the term "alkyl", refers to straight and branched alkyl groups. An analogous convention applies to other generic terms such as "alkenyl", "alkynyl" and the like. In certain embodiments, as used herein, "lower alkyl" is used to indicate those alkyl groups (substituted, unsubstituted, branched or unbranched) having about 1-6 carbon atoms. Illustrative alkyl groups include, but are not limited to, for example, methyl, ethyl, n-propyl, isopropyl, allyl, n-butyl, sec-butyl, isobutyl, tert-butyl, n-pentyl, sec-pentyl, isopentyl, tert-pentyl, n-hexyl, sec-hexyl, moieties and the like, which again, may bear one or more substituents. Alkenyl groups include, but are not limited to, for example, ethenyl, propenyl, butenyl, 1-methyl-2-buten-1-yl, and the like. Representative alkynyl groups include, but are not limited to, ethynyl, 2-propynyl (propargyl), 1-propynyl and the like.

As used herein, the term "independently" refers to the fact that the substituents, atoms or moieties to which these terms refer, are selected from the list of variables independently from each other (i.e., they may be identical or the same).

As used herein, the term "about" can refer to a variation of ±5%, ±10%,±20%, or ±25%, of the value specified. For example, "about 50" percent can in some embodiments carry a variation from 45 to 55 percent. For integer ranges, the term "about" can include one or two integers greater than and/or less than a recited integer. Unless indicated otherwise herein, the term "about" is intended to include values, e.g., concentration values, proximate to the recited range that are equivalent in terms of the functionality of the individual ingredient, the composition, or the embodiment.

As used herein, the term "and/or" means any one of the items, any combination of the items, or all of the items with which this term is associated.

As will be understood by the skilled artisan, all numbers, including those expressing quantities of ingredients, properties such as molecular weight, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements.

As will be understood by one skilled in the art, for any and all purposes, particularly in terms of providing a written description, all ranges recited herein also encompass any and all possible subranges and combinations of subranges thereof, as well as the individual values making up the range, particularly integer values. A recited range includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc.

As will also be understood by one skilled in the art, all language such as "up to," "at least," "greater than," "less than," "more than," "or more," and the like, include the number recited and such terms refer to ranges that can be subsequently broken down into subranges as discussed above. In the same manner, all ratios recited herein also include all subratios falling within the broader ratio. Accordingly, specific values recited for radicals, substituents, and ranges, are for illustration only; they do not exclude other defined values or other values within defined ranges for radicals and substituents.

One skilled in the art will also readily recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of any one or more of members of a recited group. Accordingly, provisos may apply to any of the disclosed categories or embodiments whereby any one or more of the recited elements, species, or embodiments, may be excluded from such categories or embodiments, for example, as used in an explicit negative limitation.

## DETAILED DESCRIPTION OF CERTAIN PREFERRED EMBODIMENTS OF THE INVENTION

The present invention provides the first successful biomimetic strategy for functional, robust, and readily applicable artificial neurotransmitter receptor analogues that show outstanding affinities and selectivities.

**1) General Description of conjugates of the Invention**

In this context, there is provided a conjugate comprising a nanoporous crystalline three-dimensional framework material bearing a permanent negative framework charge and having an average pore size ranging from 1 nm to 5 nm, selected from a zeolite, an organosilica, a metal organic framework (MOF) and a covalent organic framework (COF) material, containing within its pores an electrophilic UV-visible or fluoresecent analyte-responsive reporter dye, wherein the reporter dye is non-covalently bound to the zeolite inner pore surface.

Advantageously, the nanoporous crystalline three-dimensional framework material bearing a permanent negative framework charge has an average pore size ranging from 1 nm to 5 nm, preferably 1 nm to 4 nm, more preferably 1 nm to 3 nm, most preferably 1 nm to 2 nm.

Advantageously, when the nanoporous crystalline three-dimensional framework material is a zeolite, the material does not comprise a stop-cock moiety and an affinity binding agent covalently bound thereto. The stop-cock molecule may be located near a channel entrance of the zeolite material, and is typically a molecule having a head group a and a tail, comprising for example a hydrophilic anchor group, such as methoxysilane, and a spacer. The anchor group and the spacer enter the channel allowing the anchor to attach to the porous material. The bulky head group, e.g. a fluorenylmethylcarbamate group, remains outside the channel entrance due to size restriction imposed by the channel dimensions. Examples of stopcock molecules or stopcock moieties are described in detail in EP 1 335 879 and EP 2 089 320 the disclosure thereof is hereby explicitly incorporated herein by reference. The affinity binding agent, under appropriate conditions, binds to another biological moiety or chemical moiety. Affinity binding agents may comprise any of the following: an antigen, a protein, an antibody, biotin or biotin analogue and avidin or streptavidin, sugar and lectin, an enzyme, a polypeptide, an amino group, a nucleic acid or nucleic acid analogue and complementary nucleic acid, a nucleotide, a polynucleotide, a peptide nucleic acid (PNA), a polysaccharide, a metal-ion sequestering agent, receptor agonist, receptor antagonist, or any combination thereof. For example, the affinity binding agent can be one partner of a specific binding pair, where the other partner of said binding pair is associated with or is the target on a cell surface or an intracellular structure. Preferably an affinity binding agent is, a partner or member of an affinity binding pair, or as it is also called by the skilled artisan, a partner or member of a specific binding pair.

As used herein, the term "permament negative framework charge" refers to the presence of a localized negative charge at atomic sites over the entire atomic framework of the nanoporous material. Such is the case for example of zeolites which are minerals that are the result of a very low-grade metamorphism, typically found in the cavities or vesicles of volcanic rock. They are framework aluminosilicates consisting of interlocking tetrahedra of $SiO_4$ and $AlO_4$, wherein the corner-sharing $SiO_4$ and $AlO_4$ tetrahedra of the crystalline alumino silicate give rise to very defined channels. Each aluminum entity in their framework contributes a negative charge that is compensated by an exchange of cations such as sodium, calcium and the like that reside in the pore structure (see **[16, 17]**). Each zeolite type may have different channel arrangement. Some of them have one-dimensional, and other can have 3D or interconnected, channel arrangement. For example, several types of zeolite, such as Zeolite L, have hexagonal pore channel. Others, such as Zeolite Y, have a cubic and 3D inter connected pore system. Both types of zeolites may be used in the context of the present invention, and are illustrated in the Examples.

As used herein , the term "analyte-responsive reporter dye" refers to the property of a UV-visible or fluoresecence dye compound to respond to the presence of small molecule analytes bearing at least one aromatic or heteroaromatic moiety and/or at least one free thiol moiety, and an overall positive charge, wherein the small molecule has a rigid section smaller than the framework material average pore size, when the analyte comes in the vicinity of the reporter dye, or when the analyte covalently binds to the reporter dye molecule, depending on the nature of the reporter dye. The analyte presence (vicinity ot binding) triggers a signal response of the dye. The signal can be, for example, a change of UV-visible or fluoresecence emission signal. Generally speaking, the presence of a reporter dye within the pores of the nanoporous material, confers to the conjugate its high affinity and selectivity properties. As used herein , the term "analyte-responsive reporter dye" excludes DXP (N,N'-bis(2,6-dimethylphenyl)-perylene-3,4,9,10-tetracarboxylic diimide):

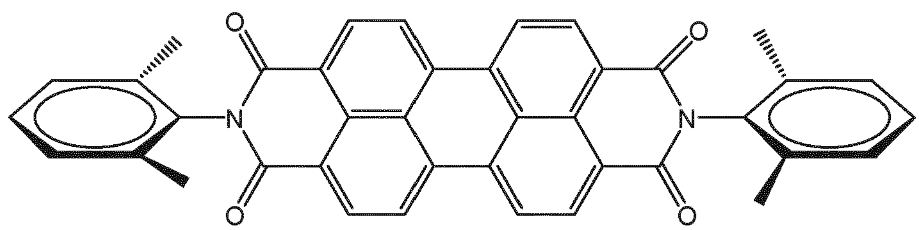
;

which is an analyte-non-responsive reference dye (i.e., it does not respond to the presence of positively charged analytes

bearing at least one aromatic or heteroaromatic moiety and/or at least one free thiol moiety). Thus, conjugates comprising a nanoporous crystalline zeolite having an average pore size ranging from 1 nm to 2 nm, containing within its pores DXP as sole UV-visible or fluorescent dye, wherein DXP is non-covalently bound to the zeolite inner pore surface, are specifically excluded from the scope of the present invention.

Advantageously, the nanoporous crystalline three-dimensional framework material may be a zeolite, for example zeolite L or Y.

Many forms of Zeolite are known, each form exhibiting different geometrical and chemical characteristics. For example, Zeolite L exhibits one-dimensional channels running through the whole crystal, with an opening of 0.71 nm, a largest free diameter of 1.26 nm and a unit cell length of 0.75 nm. The centre-to centre distance between two channels is 1.84 nm. As an example a crystal with a diameter of 550 nm consists of about 80,000 parallel channels. Zeolite L channels can be filled with suitable organic guest molecules, although only guests that can pass through the opening are able to enter the channels. Due to the channel entrances, the chemical and physical properties of the base and coat of the cylindrical crystals are different. Exemplary guest molecules include certain dyes possessing desired emission properties (see Calzaferri et al., Angew Chem Int Ed 42:3732, 2003). Zeolite L crystals can also be prepared in different sizes (diameter and length). For example the length of a zeolite L crystal can be from 30nm to several thousand nanometers (see Ruiz et al., Monatshefte Fur Chemie 136:77, 2005). Pure zeolite L crystals with lengths between 30 and 7000 nm have been synthesized previously (Megelski and Calzaferri, Adv Funct Mater 11:277, 2001). In exemplary embodiments, the nanoporous crystalline three-dimensional framework material may be an organic/inorganic hybrid organosilica material, such as $SO_3^-$ or COO- functionalized silica. See for example **[24, 25]** for $SO_3^-$ functionalized silica: S. Saravanamurugan et al. Microporous Mesoporous Mater., 2008, 112, 97 ; and Y. Satoh et al. Industrial & Engineering Chemistry Research, 2013, 52, 15293-15297; and **[26]** for $CO_2^-$ functionalized silica: Z. Chen et al. Nanoscale Research Letters, 2008, 4, 204-209.

Organic/inorganic hybrid Si-based framework materials are well-known in the literature, as well as methods for their preparation. (See for example, **[18-22]**) These methods may be readily adapted to the present invention by using a suitable Si precursor in the sol-gel synthetic process to achieve a material with the desired porosity and permament framewok negative charge.

In exemplary embodiments, the nanoporous crystalline three-dimensional framework material may be a MOF, such as MOFs comprising sulfonate linkers or other anionic MOFs. Examples of such MOF materials suitable in the present invention include anionic MOF described in the literature See for example **[27-29]:** J. Tian et al. J. Am. Chem. Soc., 2012, 134, 9581-9584; A. Karmakar et al., Coord. Chem. Rev., 2016, 307, Part 2, 313-341. For a review of ionic MOFs in general see **[29]:** A. Karmakar et al., Coord. Chem. Rev., 2016, 307, Part 2, 313-341.

In exemplary embodiments, the nanoporous crystalline three-dimensional hybrid organic-inorganic framework material may be a COF. Covalent organic frameworks and methods for making same are known in the art **[31].** The person of ordinary skill in the art will know how to adapt the known methods for preparing COFs suitable in the context of the present invention. For example, the skilled person may adapt the teachings for negatively charged MOFs and silica (cf. above) to the preparation of COFs bearing a permanent negative framework charge. Chemical modification of the nanoporous material crystal may direct the binding of the crystal to targets structures on biological moieties, such as the surface of cells, or to targets provided inside cells that are associated with cellular structures. As such, the framework material may be further functionalized at the outer surface with an organic surface agent selected from:

- a polymer, for instance polyethylene glycol (PEG), polyvinyl alcohol, polylysine or polyethyleneimine;
- an oligosaccharide, for instance cyclodextrins monomers, oligomers or polymers;
    - a polysaccharide, for instance chitosan, dextran, fucoidan, alginate, pectin, amylose, starch, cellulose or xylan; or
    - a targeting molecule such as a biotin, avidin, folic acid, lipoic acid, ascorbic acid, an antibody or antibody fragment, a peptide, a protein or a DNA, RNA or PNA moiety.

Advantageously, the framework material may be functionalized at its outer surface with a polyethylene glycol (PEG) polymer.

The dye used for conjugating with the hybrid organic-inorganic framework material may be selected by the skilled artisan according to the intended application (i.e., the analyte of interest). Preferably, the dye is an electrophilic UV-visible or fluoresecent dye, most preferably a fluorescent dye. Advantageously, the UV-visible or fluorescent dye will be selected to be capable of entering into the channels of the framework material crystal.

<u>Sensing through reversible binding:</u>

Advantageously, the reporter dye can reversibly bind small molecule analytes bearing at least one aromatic or heteroaromatic moiety and an overall positive charge, wherein the small molecule has a rigid section smaller than the

framework material average pore size.

As used herein, the term "rigid section" when referring to a small molecule analyte, refers to the dimension of the minor axis of the rigid volume occupied by the molecule in a given medium, advantageously a liquid medium (e.g., water, a physiological fluid, an organic solvent or a water/organic solvent mixture). In other words, the small molecule analyte has at least one dimension ("rigid section") that is small enough to allow it to enter the channels/pores of the framework material, to be loaded within its pores/channel, and to come in the vicinity or covalently bind the reporter dye loaded within the pores/channels of the framework material.

Small molecule analytes suitable within the scope of the present invention may be any molecule having at least one aromatic or heteroaromatic moiety and an overall positive charge, wherein the small molecule has a rigid section smaller than the framework material average pore size. For example, when the sensing is carried out at physiological pH, the overall positive charge may be contributed by the presence of at least one functional group that is protonated under physiological conditions. For example, such functional group may be a primary, secondary or tertiary, preferably primary amino group. Examples of suitable small molecule analytes meeting these features include serotonin, tryptamine, dopamine, norepinephrine, tyramine, octopamine, $TrpNH_2$, epinephrine, phenylepinephrine, phenethylamine, histamine, nicotine and propranolol. See, for example, Table 1. Alternatively, if the sensing is carried out at pH other than physiological pH, the overall positive charge may be contributed by the presence of at least one functional group that is protonated at a suitably adjusted pH (other than physiological pH). For example, the zwitterionic amino acid tryptophan at physiological pH bears an overall positive charge when the pH is adjusted <3. Accordingly, at pH<3, tryptophan which is not binding under physiological pH, becomes (reversibly) binding with a dye according to the present invention, and is therefore detectable in the context of the present invention when the sensing is carried out at suitable pH. For example, ANR1 was found to bind trytophane at pH<3 (data not shown).

Advantageously, reporter dyes that can be used in the context of the invention for reversibly binding small molecule analytes bearing at least one aromatic or heteroaromatic moiety and an overall positive charge, may be a dicationic moiety. For example, it may be a dicationic dye such as one of the following:

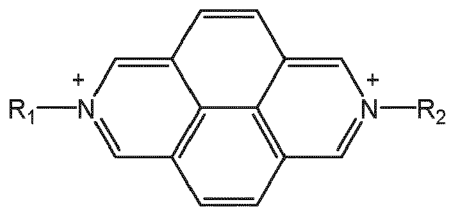

;

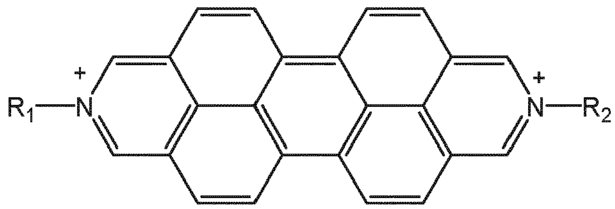

;

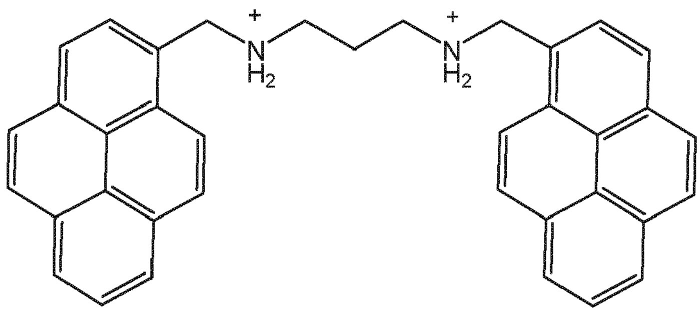

;

or

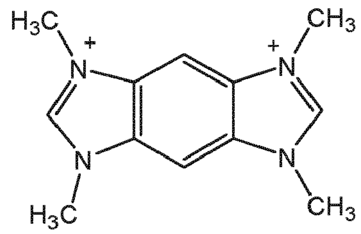

wherein $R_1$ and $R_2$ independently represent a C1-6alkyl moiety or a phenylC1-3alkyl moiety, preferably methyl or benzyl.

Examples of suitable UV-visible reporter dyes for carrying out the present invention include any dye which, upon non-covalent interaction with the analyte when the latter comes in proximity of the dye ("reversible binding"), exhibits characteristic UV/Vis bands (or Cicrular Dichroism bands). Examples of such dyes include:

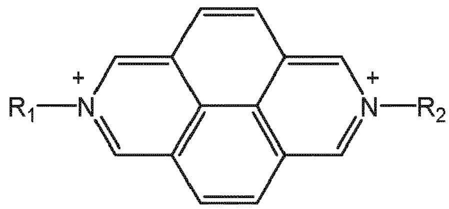

;

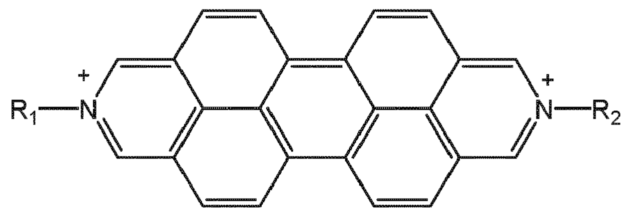

wherein $R_1$ and $R_2$ independently represent a C1-6alkyl moiety or a phenylC1-3alkyl moiety, preferably methyl or benzyl. See also **[23].**

<u>Sensing through covalent bond formation:</u>

In other exemplary embodiments, the reporter dye may be selected among dyes that can covalently bind small molecule analytes bearing at least one free thiol moiety and an overall positive charge, wherein the small molecule has a rigid section smaller than the framework material average pore size. Small molecule analytes suitable for this marticular embodiment may be any molecule having at least one free thiol moiety and an overall positive charge, wherein the small molecule has a rigid section smaller than the framework material average pore size. For example, when the sensing is carried out at physiological pH, the overall positive charge may be contributed by the presence of at least one functional group that is protonated under physiological conditions. For example, such functional group may be a primary, secondary or tertiary, preferably primary amino group. Examples of suitable small molecule analytes meeting these features include cysteamine. See Figure 50. Alternatively, if the sensing is carried out at pH other than physiological pH, the overall positive charge may be contributed by the presence of at least one functional group that is protonated at a suitably adjusted pH (other than physiological pH). For example, cysteine and cysteine-containing peptides meeting the rigid section criterion also fall within the scope of the present invention when the pH is adjusted such that all COO- groups are protonated, thus not anymore negatively charged. In this context, bovine serum albumin (too large), cysteine (zwitter ion) and ethanthiol (not charged) are not suitable analytes for this embodiment when sensing is arried out at physiological pH, as they do not meet the requisite size and charge criteria at physiological pH ((i) rigid section small enough to fit within the framework material's pores, and (ii) overall positive charge on the analyte).

We found for instance that with the ANR1, the zwitterionic amino acid tryptophan was binding when the pH chosen to be <3.

Advantageously, reporter dyes that can be used in the context of the invention for covalently binding small molecule analytes bearing at least one free thiol moiety and an overall positive charge, may be a thiol-reactive dye, selected from coumarin maleimides and iodoacetamides, pyrene maleimides and iodoacetamides, naphthalene-based dyes, bimane-based dyes.

For example, the following dyes may be used:

Coumarin maleimides :

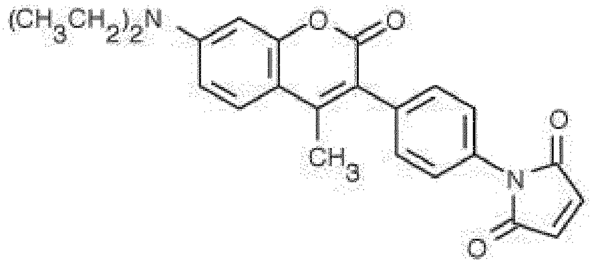

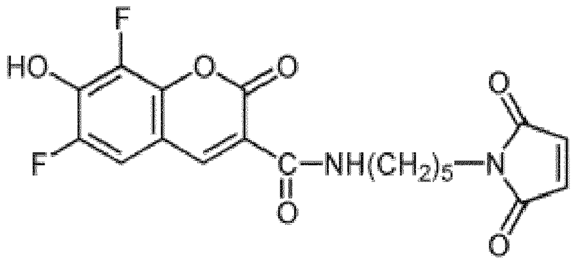

Coumarine iodoacetamides :

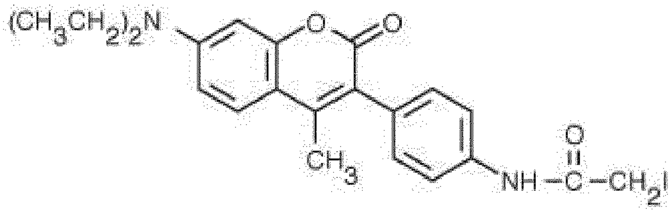

Pyrene iodoacetamides:

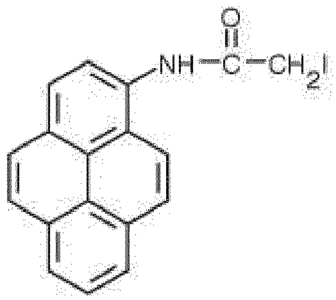

Naphthalene-based dyes:

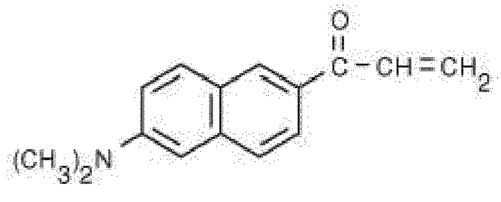

Bimane-based dyes :

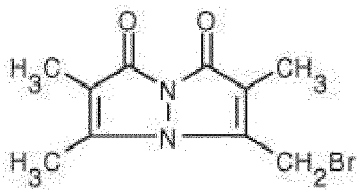

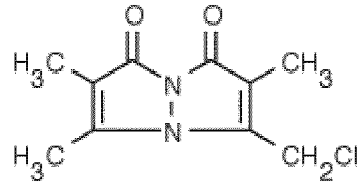

Adavantageously, the reporter dye may be a thiol-reactive dye having the structure:

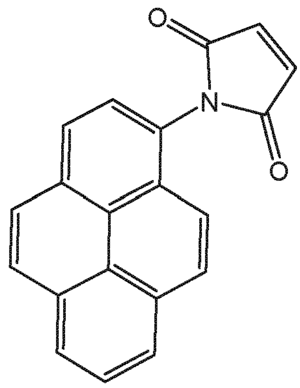

.

Advantageously, for both "reversible binding" and "covalent binding" embodiments, the nanoporous framework material may further comprise an analyte-non-responsive reference dye bearing a perylene-bisdiimide moiety encapsulated within its pores, such as DXP (N,N'-bis(2,6-dimethylphenyl)-perylene-3,4,9,10-tetracarboxylic diimide):

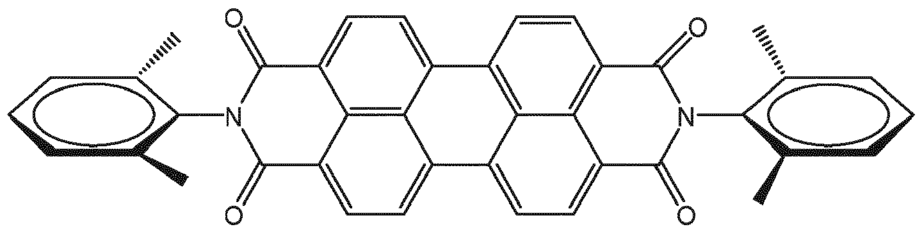

.

As used herein, the term "analyte-non-responsive reference dye" refers to a UV-visible or fluoresecence dye compound that does not respond to the presence of the small molecule analyte of interest, i.e., small molecule analytes having a rigid section smaller than the nanoporous framework material average pore size, bearing an overall positive charge, and comprising at least one aromatic or heteroaromatic moiety and/or at least one free thiol moiety.

**2) Synthetic Overview:**

In yet another aspect, there is provided a method for preparing the dye/ framework material conjugates of the invention.

Thus, in one aspect, there is provided a method of preparing a conjugate according to the invention, in all

variants described herein, the method comprising the step of immersing a nanoporous framework material having an average pore size ranging from 1 nm to 2 nm, selected from a zeolite, an organosilica, a metal organic framework ’MOF) and covalent organic framework (COF) material, in a solution of an electrophilic UV-visible or fluorescent analyte-responsive reporter dye, wherein the reporter dye has a rigid section smaller than the nanoporous framework material average pore size.

Thus, conjugates of the invention may be readily prepared by immersion of nanoporous crystalline framework material, such as commercial zeolite nanocrystals, with a solution of the corresponding reporter dyes, followed by simple washing steps (Fig. 3c). The uptake of dye by the framework materials may be readily witnessed for example by the vanishing of the colour of the supernatant as well as by quantifiable changes of the physical properties of the dye (*e.g.* absorption, excitation and emission spectra, quantum yield and anisotropy).

Encapsultation of the reporter dye into the channels of the framework material crystal may also be achieved by ion exchange, particularly when the reporter dye is a cation, or by crystallization inclusion or by a gas phase procedure using the double or the single ampoule method as described by Calzaferri et al, Chem. Eur. J. 2000, 6, 3456.

Advantageously, the reporter dye may be as defined in any of the variants above.

In exemplary embodiments, the solution in which the nanoporous framework material is immersed, may further comprise a non analyte-responsive dye bearing a perylene-bisdiimide moiety as defined above.

Preferably, the nanoporous crystalline three-dimensional framework material is a zeolite, such as zeolite Y or L. Zeolite L materials may be synthesized as described in A. Zabala Ruiz, D. Bruhwiler, T. Ban, G. Calzaferri, Monatshefte fur Chemie 136 (2005) 77 **[14],** or S. Megelski, G. Calzaferri Adv. Funct. Mater. 2001, 11, 277. **[15]** Zeolite Y materials may be synthesized according to known literature (cf. **[30]**) or can be obtained from commercial sourcesuch as ZEOLYST or SIGMA ALDRICH.

The crystalline three-dimensional framework materials used within the scope of the present invention, such as zeolite L and Y, carry a natural negative charge within their channel. Preferably, any chemical modifications/reactions applied to the material crystals are such that they do <u>not</u> lead to de-alumination that reduce or eliminate the negative charge of the channel.

In addition, the outer surface of the material may be functionalized with a surface agent.

As used herein, the term "surface agent" refers to a molecule that partly or totally covers the outer surface of the material, allowing the surface properties of the material to be modified, for example:

- modifying its biodistribution, for example to avoid its recognition by the reticulo-endothelial system ("furtiveness"), and/or
- giving it advantageous bioadhesion properties during oral, ocular or nasal administration, and/or
- enabling it to specifically target certain sick organs/tissues, etc.

According to the invention, several surface agents may be used to combine the abovementioned properties. For example, a surface agent combining at least two of the abovementioned properties may be used. For example, the organic surface agent may be chosen from:

- a polymer, for instance polyethylene glycol (PEG), polyvinyl alcohol, polylysine or polyethyleneimine, or a polypeptide,
    - an oligosaccharide, for instance cyclodextrins,
    - a polysaccharide, for instance chitosan, dextran, fucoidan, alginate, pectin, amylose, starch, cellulose or xylan,
    - a glycosaminoglycan, for instance hyaluronic acid or heparin,
    - a surface active agent, such as pluronic or lecithin,
    - vitamins, such as biotin,
    - coenzymes, such as lipoic acid,
    - antibodies or antibody fragments,
    - amino acids or peptides.

A chemical linker may be used to bind the surface agent to the outer surface of the nanoporous crystal. Such linker may preferably have a first functional group and a second functional group at each end thereof. The first functional group is capable of binding to the nanoporous material crystal (for example, binding to the silanol groups on the outer surface of an organsilica nanoporous crystal) and the second functional group is capable of binding to the selected surface agent.

In the case where the nanoporous material is an organosilica material, outer surface functionalization may be effected for example by using a function group-containing trialkoxysilane, such as a PEG group linked to a trialkoxysilane. Likewise, marking of the outer surface of the organosilica material (for example for medical purposes) may be achieved by condensation of a marker-containing trialkoxysilane. The marker may be selected from a contrast agent, a tracer, a

radioactive marker, any commercial dye, such as a fluorescent marker or a phosphorescent marker, a magnetic resonance imaging agent or a positron emission tomography agent, such as pyrene, rhodamine, IR783, Gd-EDTA or $^{64}$Cu-EDTA. The marker may be a fluorescent molecule selected from rhodamines, fluorescein, luciferase, pyrene-containing markers, aminopyrrolidino-7-nitrobenzofurazan, or indocyanine green (ICG) for NIR emission.

**3) Compositions and uses**

The conjugates of the invention are useful for any application where the detection or monitoring of small molecules is desired.

Accordingly, there is provided compositions comprising a conjugate according to the invention and any compound and/or additive suitable for any one or more of the conjugate's intended use/purpose described herein.

For example, there is provided a pharmaceutical composition comprising a conjugate according to the invention, and a pharmaceutically acceptable carrier, adjuvant or vehicle. In exemplary embodiments, these compositions optionally further comprise one or more additional therapeutic agents.

In a preferred aspect the present invention relates to the use of a conjugate according to the present invention for selectively detecting molecules in a fluid sample, such as bio fluids like blood or fruit/vegetable juices; wherein the molecule has a rigid section smaller than the nanoporous framework material average pore size, bears an overall positive charge, and comprises an aryl moiety and/or a free thiol moiety.

Advantageously, the molecule (analyte) may be an electron-rich aryl-containing neurotransmitter, drug/pharmaceutically active compound, or trace amine. For example, the molecule may be serotonin, dopamine, amidated tryptophan (TrpNH2), epinephrine, norepinephrine, tryptamine, tyramine, octopamine, or propranolol.

In a further aspect the present invention relates to the use of a conjugate according to the present invention for selectively detecting molecules in fluid samples, such as biofluids like blood; wherein the conjugate further comprises a non analyte-responsive dye bearing a perylene-bisdiimide moiety encapsulated within its pores, such as:

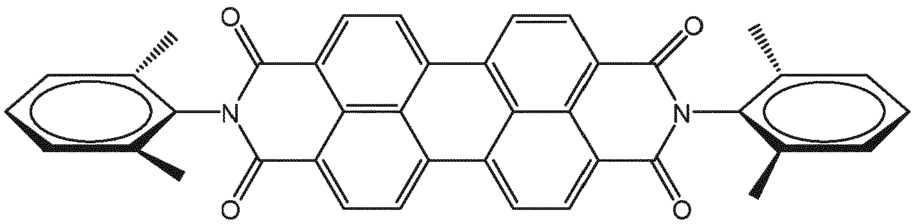
;

and the molecule is an electron-poor positively charged aryl-containing compound having a rigid section smaller than the nanoporous framework material average pore size, such as phenethylamine, histamine or nicotine. It is surmised that the electron-poor aryl-containing compound favors dye-aggregation within the nanoporous framework material pores, for example via $\pi$-$\pi$ stacking.

In a further aspect the present invention relates to the use of a conjugate according to the present invention as drug-capture/release monitoring system; wherein the drug has a rigid section smaller than the nanoporous framework material average pore size, bears an overall positive charge, and comprises an aryl moiety and/or a free thiol moiety.

In a further aspect the present invention relates to the use of a conjugate according to the present invention for real-time minotoring of chemical or enzymatic reactions. For example the chemical reaction may be the degradation of serotonin, and the enzymatic reaction may be the enzymatic conversion of Parkinson's pro-drug L-DOPA into dopamine by L-DOPA decarboxylase.

In a further aspect the present invention relates to a method for detecting an analyte, the method comprising the steps of

a) providing a fluid sample suspected or known to comprise the analyte;
b) contacting said sample with a conjugate according to the invention under conditions appropriate for interaction of the analyte and the reporter dye within the nanoporous framework material pores;
c) measuring any UV-visible or fluorescence emission-quenching response of the reporter dye; thereby detecting the presence of the analyte;

wherein the analyte has a rigid section smaller than the nanoporous framework material average pore size, bears an overall positive charge, and comprises an aryl moiety and/or a free thiol moiety.

The conditions appropriate for interaction of the analyte and the reporter dye within the nanoporous framework material pores need not be specified, since the skilled artisan without any inventive effort can easily identify such

appropriate incubation conditions. As the skilled artisan will appreciate there are numerous methods to measure such analyte-dye interaction, all described in detail in relevant textbooks (cf, e.g., Christopoulos, T.K. (eds.), Immunoassay, Academic Press, Boston (1996)).

Advantageously, the interaction in step (b) may be covalent binding, or reversible non-covalent binding (π-π stacking interaction). For example, the contacting step (b) may be carried out in an aqueous or organic solvent, for example water, buffered aqueous medium, physiological fluid, alcohol solvent, ether solvent, alkane solvent or chlorinates solvents, for example $CHCl_3$ or ethanol.

The fluid sample may comprise a biofluid, wherein the biofluid comprises whole blood, serum, plasma, cerebral spinal fluid, urine, saliva, semen, joint aspirate, wound drainage, fruit juice, or vegetable juice. Indeed, many fruits and vegetables contain serotonin (e.g. banana, pineapple). Therefore, the conjugates of the present invention also find applications to food analysis.

The fluid sample may be prefiltered or treated to remove impurities prior to contacting with the conjugate.

The fluid sample may further comprise a buffer, a salt, an enzyme, a surfactant, or a combination thereof.

The analyte may be an electron-rich aryl-containing neurotransmitter, drug/pharmaceutically active compound, or trace amine, such as one defined herein.

In exemplary embodiments, the analyte may be an electron-poor aryl-containing compound having a rigid section smaller than the nanoporous framework material average pore size, such as phenethylamine, histamine or nicotine, and the reporter dye is a dicationic moiety having one of the following structures:

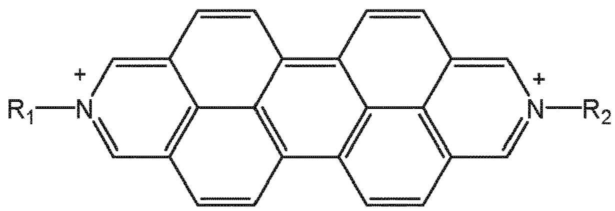

;

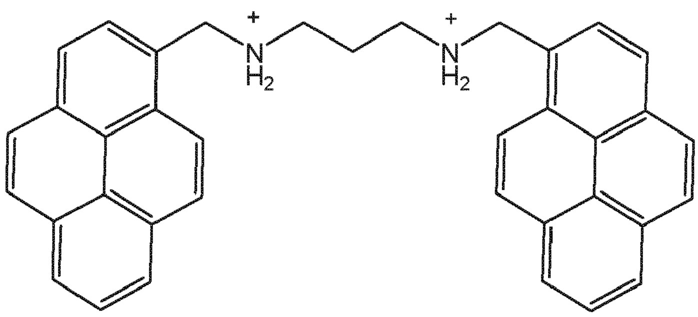

.

In exemplary embodiments, the analyte may be an electron-rich drug/pharmaceutically active compound having a rigid section smaller than the nanoporous framework material average pore size, bearing an overall positive charge, and comprising an aryl moiety and/or a free thiol moiety; and the method allows to monitor drug capture and/or release.

In exemplary embodiments, the analyte may be a substrate of a chemical or enzymatic reaction, such as serotonin and Parkinson's pro-drug L-DOPA.

In exemplary embodiments, the analyte may comprise a free thiol moiety and the reporter dye is a thiol-reactive dye, such as one defined herein. For example, the analyte may be cysteamine.

Avantageously, in any one of the above-described method or use, the nanoporous crystalline three-dimensional framework material may be a zeolite, such as zeolite Y or L.

Evidence for the spatial proximity of analytes (e.g., serotonin or dopamine) and the reporter dye in the cavities of the porous material is provided by the changes in the absorption spectrum, e.g. appearance of characteristic charge transfer (CT) bands, upon analyte addition (see Examples). Accordingly, the conjugates may be used for the spectroscopic investigation of analytes concentration in a liquid medium, such as a biofluid.

Specifically, the conjugates of the invention may be used to the detection of low μM concentrations of aromatic-amine analytes (e.g., neurotransmitters) in aqueous media, owing to the high binding affinity and the sensitivity of the spectrometric response (e.g., fluorescence response) of the reporter dye.

The conjugates of the invention may also be used for the detection of electron-poor analytes (e.g., histamine) owing to the modularity of the reporter dye- material conjugate approach of the present invention, allowing also the detection of non-emission-quenching analytes by changing the signal generation strategy to a dye-deaggregation approach.
The conjugates of the invention may also be used for analyte-differentiation and ratiometric sensing (e.g., neurotransmitter-differentiation and ratiometric sensing). The modularity of the conjugates according to the invention allows for obtaining differently selective conjugates with ease, by changing the reporter dye and/or material making up the conjugate. Upon changing the reporter dye, the magnitudes $\pi$-$\pi$-, cation-$\pi$ and CT-interactions between the dye and analyte can be altered, resulting in relative affinity differences on account of electronic effects. In addition, when using different material frameworks, the steric contributions to binding are changed. By modulating both of these features, analyte differential sensing is rendered possible.

The conjugates of the invention may also be used for real-time monitoring of changes in the analyte (e.g., neurotransmitter) concentration. Apart from the simple detection of analytes, non-covalently binding conjugates according to the invention have great potential for applications in monitoring of chemical and enzymatic reactions in real time because of their fast response time and reversible, *i.e.* non-destructive, binding of the substrate/product pair. Hence, non-covalently binding conjugates according to the invention could be used to follow of the release and re-uptake, or the enzymatic production and degradation of neurotransmitters in real time, which are important dynamic biological processes, *e.g.* in the synaptic cleft, for allergic responses, or the enzymatic conversion of the Parkinson drug L-DOPA to dopamine.
The conjugates of the invention may also be used for monitoring of intracellular release from a nanoporous material-drug-delivery material. Porous materials, such as zeolites, MOFs, organosilica and COFs, which are apable of storing gases or delivering drugs have found wide ranges of industrial and medical use, respectively. However *in situ* monitoring strategies for their cargo-loading and - deloading process remain rare, such that fluorophore-labelled drug-analogues are commonly used. Here, conjugates according to the invention can serve as a porous drug carrier with a built-in fluorescent signal transducer, allowing to monitor the loading and release of the drug.

The conjugates of the invention may be used in a versatile way: in both aqueous and organic media, without loosing their functionality (high affinity bonding and selectivity).
The dye/ material conjugates according to the invention therefore can find applications in in vitro and in vivo diagnostics, in drug delivery, and in any other application where a high affinity and selective binding to small molecule analytes can be envisaged.
Other advantages may also emerge to those skilled in the art upon reading the examples below, with reference to the attached figures, which are provided as nonlimiting illustrations.

## EQUIVALENTS

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.
The following examples contain important additional information, exemplification and guidance that can be adapted to the practice of this invention in its various embodiments and the equivalents thereof.

## EXEMPLIFICATION

The reporter dye conjugates according to the present invention and their preparation can be understood further by the examples that illustrate some of the processes by which these conjugates are prepared or used. It will be appreciated, however, that these examples should not be construed to limit the invention. Variations of the invention, now known or further developed, are considered to fall within the scope of the present invention as described herein and as hereinafter claimed.
The present invention will now be exemplified using a zeolite as nanoporous crystalline three-dimensional framework material but it will be understood this is not meant to limit the invention.
The Examples described herein can be transposed to other nanoporous materials such as organosilicas, metal organic frameworks (MOFs) or covalent organic frameworks (COFs), which, - depending on their charge and atomic composition - are also adapted for carrying out the present invention.
Throughout the present document, the acronym "ANR" stands for "artificial neuroreceptors" and corresponds to a nanoporous material/reporter dye conjugate according to the present invention, wherein the nanoporous material is a zeolite. **ANR-L1 - ANR-L5** designate conjugates according to the present invention of (i) zeolite LTL and (ii) dicationic reporter dyes D1-D5, respectively (see Fig. 1d). Likewise, **ANR-Y1** and **ANR-Y2** referred to in the present document, designate conjugates according to the present invention of (i) zeolite Y and (ii) reporter dicationic dye D1-D2, respectively.

## Materials and methods

*Materials Characterizations*

SEM measurements were carried out using a FEI Quanta FEG 250 instrument. DLS and zeta-potential analysis were performed on a Delsa Nano C Particle Analyzer (Beckman Coulter, Brea, CA, USA); all DLS measurements were conducted in water, while zeta-potential analysis in phosphate buffered saline (PBS), pH = 7. Thermo-gravimetric analyses (TGA) were carried out by using a STA 449 F1 Jupiter (Netzsch, Selb, Germany).

*Photophysical Characterisations*

Absorption spectra were measured on a Shimadzu UV-3600 spectrophotometer double-beam UV-VIS-NIR spectrometer and baseline corrected.
Steady-state emission spectra were recorded on a Horiba Jobin-Yvon IBH FL-322 Fluorolog 3 spectrometer equipped with a 450 W xenon arc lamp, double-grating excitation, and emission monochromators (2.1 nm $mm^{-1}$ of dispersion; 1200 grooves $mm^{-1}$) and a TBX-04 single photoncounting detector. Emission and excitation spectra were corrected for source intensity (lamp and grating) and emission spectral response (detector and grating) by standard correction curves.
Confocal fluorescence microscopy experiments were acquired using Zeiss LSM 710 confocal microscope system equipped with 63x magnification, numerical aperture 1.3 of Zeiss LCI Plan-NEOFLUAR water immersion objective lens (Zeiss GmbH). The particles (e.g. **ANR-L1)** were excited by a continuous wave (cw) laser at 405 nm while the emission signal was collected from 412 to 723 nm, respectively. For z-stack cellular experiment, the samples that were counterstained priory with DAPI and Alexa Fluor® 647 Phalloidin were excited at 405 and 633 nm, respectively. All image processing was done by ZEN software (Zeiss GmbH) and false colour images were adjusted.

**Materials Preparation**

The zeolite host (Lucidot® NZL 40) was provided by Clariant. Lucidot® NZL 40 is a zeolite with an L structure with crystallite sizes around 50 nm. Zeolite Y was purchased as its sodium salt from Alfa Aesar and shows a size around of 900 nm (see Table 7).
**D1** was synthesized from 1,3,6,8-tetrahydro-2,7-dimethyl-2,7-diazapyrene, following the DDQ oxidation procedure given in references **[5]** and **[6].** Similarly, **D2** and **D3** were prepared by DDQ oxidation procedures shown in the aforementioned references. **D4** was prepared according to literature procedures. **[7]** Analytes (e.g. neurotransmitters) were purchased from Sigma Aldrich with the highest purity grade available, typically as analytical standard grade, but in all cases at least 99%, and used as received.

## Example 1

*Modified literature procedures for the synthesis of dye **D1-D3** by DDQ oxidation:*

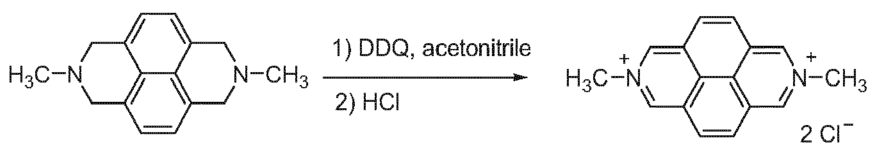


**D1**

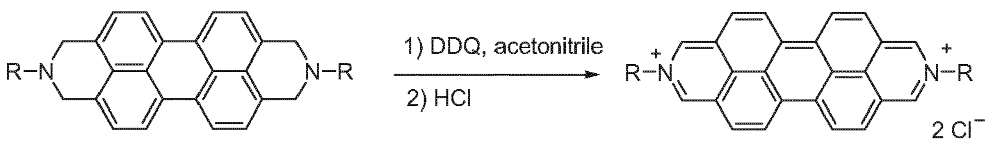


**D2**: R = $CH_2C_6H_5$
**D3**: R = $CH_3$

A 100 ml RBF with connected reflux condenser was charged with the amine (0.84 mmol) and 2,3-dichloro-5,6-dicyano-1,4-benzoquinone (DDQ) (2.0 g, 6 mmol). Then 100 mL of dry acetonitrile was added, the apparatus was and

the mixture was stirred overnight at r.t. and then another 7 days at reflux. The solution was cooled to r.t. and 5 mL of concentrated HCl and 150 mL of acetone were added. A yellow-brown (**D1**) or red **(D2, D3)** precipitate formed and the solution was kept in a fridge overnight. The solid was collected by filtration and washed with copious amount of acetone. The solid was redisolved in 20 mL of 1M HCl and filtered. Upon addition of acetone (300 mL) a yellow precipitate formed. The flask was left stand in the fridge overnight and the solid was collected by filtration and washed with acetone. If still starting material, as its hydrochloride salt, can be detected in the sample (e.g. by NMR or by emission spectroscopy), then the solid can be dissolved in a minimal amount of saturated $NaHCO_3$ - the solution turns brown - filtered and then conc. HCl can be added until a pH ~ 3 - the solution turns yellow again. Then acetone is added (300 mL) and the forming yellow precipitate is collected and washed with acetone. The title compound was yielded as a yellow solid **(D1)** or red solid (**D2** and **D3**) in 40%, 30% and 40% yield, respectively. The analytical properties of the materials (NMR, ESI-MS, fluorescence excitation and emission spectra, Fig. 2-5) matched that of the literature reports.

**D1:** $^1H$ NMR (400 MHz, $D_2O$) δ 10.04 (s, 4H), 8.86 (s, 4H), 4.98 (s, 6H) ppm. ESI-MS: m/z calcd for [$M^{2+}$] 117.0573 Da, found 117.0569 Da. Fluorescence excitation and emission spectra are shown in Fig. 4a.
**D2:** $^1H$ NMR (400 MHz, $D_2O$) δ 9.57 (s, 4H), 8.10-7.55 (m, 18H), 6.25 (s, 4H). (Note: Dye stacking occurs in aqueous media). $^{13}C$ NMR (101 MHz, $D_2O$) δ 159.16, 137.52, 133.82, 133.73, 130.76, 130.21, 129.66, 125.55, 65.83 ppm. (Note: Due to low solubility and stacking, not all quarterny carbons appear after 10,000 scans). ESI-MS: m/z calcd for [$M^{2+}$] 255.1043 Da, found 255.1041 Da. Fluorescence excitation and emission spectra are shown in Fig. 4b.
**D3:** $^1H$ NMR (400 MHz, $D_2O$) δ 9.13 (s, 4H), 7.20 - 6.90 (m, 8H), 4.63 (s, 6H). (Note: Dye stacking occurs in aqueous media). $^{13}C$ NMR (101 MHz, $D_2O$) δ 138.57, 125.57, 125.17, 124.77, 124.53, 116.80, 49.08. ppm. ESI-MS: m/z calcd for [$M^{2+}$] 179.0730 Da, found 179.0728 Da. Fluorescence excitation and emission spectra are shown in Fig. 5b.

**Example 2**

*Preparation of $N^1$,$N^3$-bis(pyren-1-ylmethyl)propane-1,3-diaminium acetate (**D5**)*

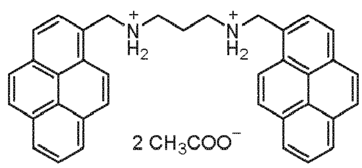

Synthesis of **D5:** 250 mg of pyridine-carboxaldehyde (2.2 equiv) was dissolved in acetonitrile (10 mL) and diamino-propane (37 mg, 1equiv) was added. The solution was cooled in an ice bath. Sodium cyanoborohydride (150 mg, 5 equiv) was slowly added and the reaction mixture was stirred for 1h by allowing it to warm up to r.t. Then two drops of acetic acid (glacial) were added, and then another 2 drops after 2h total reaction time. The reaction mixture was stirred overnight and the reaction progress was followed by TLC. Diethyl ether (200 mL) was added and the organic phase was washed with aqueous sat. sodium carbonate (2x 100 mL) and with water (100 mL). The organic phase was dried over sodium sulfate and the solvent was evaporated under reduced pressure. The residue was taken up in a minimal amount of chloroform and reprecipitated upon addition of a few drops of acetic acid. The title compound was obtained by suction filtration, followed by washing with diethyl ether and drying under reduced pressure as an off-white solid.
$^1H$ NMR (400 MHz, $d^6$-DMSO) δ 8.41 (d, 2H), 8.27 - 8.01 (m, 20H), 4.36 (s, 4H), 2.77 (t, 4H), 1.74 (s, 6H, -$CH_3$ of acetate) ppm. $^{13}C$ NMR (101 MHz, $d^6$-DMSO) δ 174.17 (-COO of acetate), 135.41, 131.27, 130.82, 130.23, 128.99, 127.88, 127.48, 127.44, 127.17, 126.54, 125.42, 125.34, 125.02, 124.53, 124.48, 124.23, 51.55, 48.45, 23.95 (-$CH_3$ of acetate) ppm. ESI-MS: m/z calcd for [M-H]+ 503.25 Da, found 503.25 Da. For the emission spectra, see Fig. 6.

**Example 3**

*Preparation of large, barrel-shaped zeolite L crystals*

3.11 g KOH was dissolved into 22 g dionized water, and the solution was stirred at 0°C for 5min. Then 0.58 g Al powder was added in to the solution under nitrogen atmosphere. The solution was stirred at 0°C for 15 min, then at room temperature for 1.5 h. The obtained solution with suspended impurities from the Al-source was filtered. After stirring for 5-10min, it was added under strong stirring into a suspension of 14.34 g LUDOX HS-40 collodial silica. The suspension

was transferred into pressure vessel and heated to 175°C for 72 h at static condition. Then the vessel was cooled in ice bath for 1 h, centrifuged at 4000 rpm for 8 min and washed until the supernatant solution became pH. The counter-cations were then exchanged for proton as follows: The material was suspended in 70 mL water containing 2.0 g ammonium chloride. The suspension was heated to 50°C for 5h, and was then centriguged 4000 rpm for 8 min and washed. The dried solids where then calcinated at 550°C for 6 h. The material was characterized by XRD and SEM measurements. These confirmed its monodisperse and crystalline nature and verified that its framework belongs to the class of zeolite L.

**Example 4**

*Preparation of the ANRs.*

A dicationic dye (**D1**-**D5**) was solubilized in 10 mL deionized water to reach a concentration of 5 mM. Then, commercial zeolite L powder (500 mg) was added to the solution. After 5 min of sonication, the suspension was shacked overnight. Then, the sample was centrifuged, decanted and washed copious time with water to remove surface-physisorbed dye. This sequence was repeated until the supernatant became colourless and non-emissive. Finally, the solids were dried in vacuum to yield the corresponding **ANR-LX** (X = 1 ... 5). The same procedure was applied for the preparation of **ANR**-**Y1** and **ANR-Y2** starting from zeolite Y. See Table 7 for information about the size and surface charges of the ANRs, and see Table 6 the Figures 4-6 and 40 for the photophysical properties. The included dye was on the order of 0.5% per weight, see the section "Discussion of the number of binding sites ($c_B$)", below. Table EM measurements confirmed that no significant change in the morphology occurred upon dye uptake, as was expected. These small-particle ANR materials are ideally suited for fluorescence titration experiments and for the investigation of their behaviour in biological media and cells. The large, custom-made barrel-shaped zeolite L (see above) was also transferred into a functional ANR by uptake of dye D1. This material was then used for the confocal microscopy studies presented in Fig. 1e.

**Example 5**

*Preparation of **ANR-L1-DXP***

*1. Insertion of reference dye DXP by sublimation*

The DXP dye (0.1 mg) is mixed with commercial zeolite-L (100 mg) in a glass ampoule. **[8]** The amount of DXP loading is calculated by considering that one DXP molecule occupies 3 unit cells and a loading of 20% corresponds to the highest loading possible. We aimed for a loading of 1%. The ampoule was dehydrated at about $4.0x10^{-6}$ mbar for 12 hours and sealed. The ampoule was then heated at 300 °C for 12 hours in the rotating oven. Afterwards the zeolite-L crystals were washed with n-butanol until the supernatant showed no absorbance anymore and the zeolites were finally dried in vacuum.

*2. Loadihg of DXP-zeolite with **D1***

To a suspension of DXP-loaded zeolite L in water (200 mg in 10 mL) was added dye **D1** to achieve a concentration of 1 mM. The suspension was shacked overnight at room temperature, centrifuged and the solids where treated 4x with a washing-centrifugation-decanting sequence. The so prepared **ANR-L1-DXP,** suitable for ratiometric sensing, (Fig. 28), was then dried in vacuum.

**Example 6**

*Preparation of PEGylated **ANR-L1-PEG***

To a suspension of **ANR-L1** (40 mg) in 1.5 mL of anhydrous chloroform and the suspension was sonicated with a tip sonicator for 2 min. Then 40 mg of MeO-PEG-silane ($M_w$ = 5 kDa) were added and the suspension was stirred with a magnetic stirrer bar at 40°C overnight and then centrifuged. The solids were washed 2x with chloroform and 2x with ethanol (each 1.5 mL) and then dried under vacuum. The presence of surface-grafted PEG on the ANR was confirmed by TGA (weight loss 6.8%) and SEM experiments, showing substantially less particle agglomeration than for the parent **ANR-L1.**

**Example 7**

*Preparation of polylysine-coated **ANR-L1***

To a suspension of **ANR-L1** (40 mg) in 1 mL of deionized water was added 500 μL of an aqueous solution of polylysine (0.1% (w/v), Santa Cruz Biotechnology). The suspension was sonicated in a sonication bath for 2 min and then shacked for 2 hours and centrifuged. The solids were washed 3x with deionized water and then dried under vacuum. The presence of a polylysine coating layer on the ANR was confirmed by the positive zeta potential of the coated, and negative zeta potential of the parent **ANR-L1** (Table 7).

The polylysine-coated **ANR-L1** can be readily loaded with serotonin as a model drug by suspending the material in a 1 mM serotonin solution in water, followed by a centrifugation and washing step.

**Example 8: Titration Experiments for Analyte-ANR interactions and Data Analysis**

*Preparation of the suspension of the ANRs and solutions of the analytes.*

A suspension of an ANR in a buffer (approx. 40 mL in total volume, suitable for 15 experiments) was prepared by weighing in the ANR (3 significant figures) followed by addition of the buffer and treatment with a tip sonicator (UP200S Hieschler) on the strongest setting for 5 min. The slightly warmed-up suspension was allowed to cool to room temperature and used as such.

The concentration of the analyte stock solution, typically 100 μM to 10 mM, was adjusted that less than 10% dilution will occur upon addition of 3-10 equivalents of the analyte to the cuvette containing the ANR suspension (as compared to the concentration of the dye in the ANR). The concentration of the analyte stock solution was independently assessed by their absorbances, utilizing reported extinction coefficients of many neurotransmitter, amino acids and their derivatives. In this way, it was confirmed that the intended concentrations by weighing in (3 significant figures) and the actual concentration of the analyte stock solution where always within 5% error.

*Fluorescence Titration Experiments*

Analyte-ANR response experiments were carried out in a fluorescence quartz cuvette (3.5 mL) by titrating a suspension of the ANR in buffer with a stock solution of the analyte. The excitation and emission wavelengths were chosen outside the absorbance window of the analyte while simultaneously avoiding the contribution of the Raman scattering peak to spectral area of interest. It was found that repetitive measurements of the ANR suspension gave essentially identical spectra and intensities, confirming that the ANR suspensions are stable. It was also confirmed that repetitive addition of buffer to the ANR suspension did not affect the measurable emission intensity beyond dilution effects. After addition of aliquots of the stock solution of the analyte to the ANR suspension, the cuvette was shaken for about 3 seconds and the emission spectra was recorded. By comparison with longer waiting times (e.g. 10 s, 5 min, 30 min) it is clear that the binding equilibrium was already established after 3 seconds (possibly faster), *i.e.* within manual mixing time.

Stern-Volmer analysis of the emission data shows the expected linear behaviour between the concentration of the quencher and the ratio of the emission intensities $F_0/F$.

The binding isothermes were obtained then by plotting relative emission intensity at a suitable wavelength against the analyte concentration and were then fitted by equation (1), see below.

This fitting procedure was used in favour of a Stern-Volmer analysis, because linearized binding plots are known to produce errors in $K_d$.

*Data analysis and extraction of the affinity constants $K_d$*

The binding isotherms were fitted by a least square fit through a binding equation for a single site 1:1 binding model (A+B→AB) under the assumption that only the components B and AB are emissive.

$$\frac{F_A}{F_0} = 1 + \frac{\Delta F[(c_A+c_B+K_d)-\sqrt{(c_A+c_B+K_d)^2-4\cdot c_A\cdot c_B}]}{2\cdot c_B} \qquad (1)$$

Herein, $F_A$ is the intensity at a given analyte concentration and $F_0$ is the emission intensity before analyte addition. $\Delta F$ is a measure of the relative emission increase or decrease caused by the analyte. For fully non emissive AB complexes, *i.e.* when the analyte A is an efficient quencher, $\Delta F$ reaches -1. The quantity $c_A$ denotes the concentration

of the analyte A and $c_B$ denotes the concentration of the "binding stations" in the ANR.

The values $K_d$ and $\Delta F$ result from the non-least square fit given the input $F_A$, $F_0$, $c_A$ and $c_B$.

The concentration $c_A$ of the analyte follows directly from the concentration of the stock solution and the added volume to the cuvette with the suspension of the ANR. The concentration $c_B$ of the single binding sites per ANR suspension is a priori not known. However, $c_B$ is directly and unambiguously obtained from the fitting of the fluorescence binding isotherms for the case of strongly binding analytes, e.g. serotonin and tryptamine. This so obtained value $c_B$ was then used as a constant for the fitting of the binding isotherms of the weaker binding analytes. Notably, in all cases were fits observed with an adjusted R-square value > 0.98, which cooperates that all binding sites inside the zeolite crystal can be treated as independent and equal. In addition, it is reassuring to note that the value of the affinity constant $K_d$ is rather insensitive to the concentration $c_B$. The so determined $c_B$ values are tabulated below.

| ANR suspension (250 μg/mL) | $c_B$ from fitting / μM | $c_{dye}$ from rel. $I_{em}$ / μM |
|---|---|---|
| **ANR-Y1** | 1.1 | 1.1 (0.13 *wt%*) |
| **ANR-Y2** | 1.0 | n.d. |
| **ANR-L1** | 6.6 | 0.4 (0.05 *wt%*) |
| **ANR-L2** | 2.6 | n.d. |
| **ANR-L3** | 3.2 | n.d. |
| **ANR-L4** | 5.5 | n.d. |
| **ANR-L1-DXP** | 4.0 | n.d. |

*Discussion of the number of binding sites ($c_B$)*

Based on the simplified schematic description in Fig. 1b, one would expect that the number of binding sites in the ANRs is closely related to the number of included dye molecules. This assumption was tested for **ANR-L1** and **ANR-Y1** by determining the dye concentration independently. In particular **D1** shows similar photophysical properties (*e.g.* emission quantum yield (Table 6), and emission and excitation spectra (Fig. 4) as its aqueous solution and inside the zeolite L and Y crystal. Thus, a stock solution of **D1** in 10 mM HEPES buffer was diluted as such that the recorded fluorescence emission intensity nearly equals that of an **ANR-L1** suspension (250 μg/mL) or that of an **ANR-Y1** suspension (250 μg/mL) in the same buffer. Because the concentration of the stock of **D1**, the quantum yields and the dilution factors are known, we could arrive at an approximate concentration of the dye component in the ANRs. For **ANR-Y1** (250 μg/mL), the dye concentration was $c_{dye}$ = 1.1 μM, which coincides with the concentration of the binding sites, $c_B$, see the table above. This suggests that indeed every cavity-bound dye molecule can act as an independent binding station within the zeolite Y crystals. For **ANR-L1** (250 μg/mL), the dye concentration was $c_{dye}$ = 0.37 μM, which is substantially lower than that of the binding stations, $c_B$. In this case, it is therefore plausible, that analytes can also bind in other parts of the zeolite L channels and do not only form face-to-face stacking complexes with the dye.

*Principal Component Analysis*

Principal component analysis was carried using the built-in tools in the Origin 8.6 software package by using a correlation matrix. The raw data was found to be highly correlated (> 0.9) such that principal component analysis is applicable to the data treatment. The number of extracted components were set to 2, which accounted for >90% of the variance. Figure - Figure display the so obtained score-plots.

**Example 9: Experiments in Biological Media and with Cells.**

*Stability and Functionality of **ANR-L1** particles in blood serum and culture media*

50 μl of dispersion of **ANR-L1** particles in blood serum (at concentration 1 mg/ml) was placed on glass cover slips and analyzed directly by confocal microscope. Next, 50 μl of serotonin solution in HEPES buffer (at concentration

1mM) was mixed to the droplet and the confocal microscopy experiment was performed. Finally, 50 μl of histamine solution HEPES buffer (at concentration 10mM) was extra added to the sample and the sample further analyzed by microscope.

## Example 10: Detection of non-quenching neurotransmitters, *e.g.* histamine

An important feature of the confinement of the dye into the zeolite is the possibility to form dyes aggregates into the channels. Upon aggregation some of the employed dyes display an exciplex emission at low energy (**D5**) that is well separated from the monomeric fluorescence, others are strongly quenched in the aggregate form (*e.g.* **D2** and **D3).** These behaviors are enhanced inside the zeolite nanopores. Disaggregation of the dyes can be used for the detection of analytes that cannot quench the emission of the dyes. Addition of the non-quenching neurotransmitter histamine to **ANR-L3** and **ANR-L5** causes a dye-deaggregation that is signaled by a clear increase in the emission intensity (**ANR-L3**), and a shift in the intensity ratio at different wavelengths (**ANR-L5**), respectively (Supplementary Fig. 38). Besides, the larger dye **D2** shows signs for self-aggregation in the larger-cavity of zeolite Y, which analogously allows for the detection of histamine binding to **ANR-Y2,** see Fig. 2c in the main text. Similar phenomena were observed for the non-quenching trace amine phenethylamine while the toxin nicotine gave at low μM concentrations a comparably weak response with all ANRs *(e.g.,* see Fig. 12).

## Example 11: Functionality in organic solvents and importance of cavity water release

An interesting and potentially useful feature of the ANRs is their full functionality in organic solvents (Fig. 30-32). For instance, the affinity of **ANR-L3** for tryptamine in chloroform, or a 99:1 chloroform-acetic acid mixture is substantial, 15 μM and 4 μM, respectively, and comparable to that of protonated tryptamine in aqueous buffer ($K_d$ ~ 9 μM). Thus, analytical protocols that pre-extract the neurotransmitter in an organic solvent, *e.g.* as its free base, are combinable with our fluorescent neurotransmitter detection strategy.

Fundamentally important, a higher binding affinity for the protonated serotonin was observed in aqueous than in ethanolic media (10x stronger for **ANR-L1** and 4x stronger for **ANR-Y1**) Based on a lock-and-key argument, the opposite would have been expected because salt-bridges are stronger in a less polar environment. In addition, the energetic cost of desolvation of protonated serotonin from the bulk solvent is higher in water than in ethanol. That nevertheless the affinity is substantially higher in the more cohesive solvent water is a good indication for the importance of the non-classical hydrophobic effect *(i.e.* high-energy cavity water molecules) in the zeolite-based receptor system.

## Results and Discussion

### Design strategy for artificial neurotransmitter receptors

If the release of energetically frustrated cavity water molecules from protein binding pockets is (one of) the main driving forces for protein-ligand complexations, the inventors surmised that artificial receptor analogues should be constructed from synthetic hosts or materials that possess similarly sized, hydrophobic cavities. To test this hypothesis, zeolite materials were employed in this study as receptor scaffolds because of *a*) their porosity, shape-stability and crystallinity allows for the creation of robust and molecularly defined binding pockets, and *b*) their permanent negative framework charge mimics the electrostatic-signature of $D_3$-receptor cavity (Fig. 1b).

It is known that zeolite materials can be loaded with fluorescent dyes. However, the use of analyte-responsive reporter dyes in this context to indicate the presence of an aromatic amine neurotransmitter had never been considered nor explored prior to the inventors' work described herein.

Specifically, we show herein that the dicationic reporter dyes (**D1**-**D5**) shown in Fig. 1d and Fig. 3a are readily uptaken and strongly bound in the negatively charged zeolite channels. The employed zeolite L-based artificial neuroreceptors (**ANR-L1** - **ANR-L5**, with number indicating the dye code) and the zeolite Y-based counterparts **ANR-Y1** and **ANR-Y2** were prepared by immersion of zeolite nanocrystals with a solution of the corresponding dyes, followed by washing steps (Fig. 3). The uptake of dyes by zeolite materials (ca. 0.1 *wt%* dye loading, see Examples) can be readily witnessed by the vanishing of the colour of the supernatant as well as by quantifiable changes of the physical properties of the dye (e.g. absorption, excitation and emission spectra and quantum yield - see Fig. 2a & Fig. 4-6).

Zeolite L (max. accessible pore diameter 10.7 Å) and zeolite Y (max. accessible pore diameter 11.9 Å) were chosen because their pores provide sufficient space to bind a dye molecule and an aromatic amine neurotransmitter in close proximity.

By entrapping the reporter dye in the negatively charged zeolites (Table 6), the resulting conjugates respond selectively to positively charged species that carry an aromatic moiety. In fact, out of the wide variety of naturally occurring small molecules, these characteristics are simultaneously found only for aromatic amine neurotransmitters, such as

serotonin, dopamine and epinephrine, and some neuroactive trace amines (Fig. 1c and Fig. 3). Indeed, the herein presented conjugates are extremely selective for these species.

Having verified the stability of the ANRs in buffers, we investigated their spectroscopic response upon addition of the representative aromatic amine neurotransmitters. In agreement with the conjugates' design, the emission of **ANR-L1** is nearly fully quenched by dopamine (Fig. 1e). Emission quenching was generally found for all ANRs in the presence of electron-rich aromatic neurotransmitters such as serotonin, dopamine and norepinephrine (Fig. 2b & Fig. 7-11). The emerging spatial proximity of the neurotransmitters and the reporter dye in the cavities of the ANRs is also implied by characteristic CT-band features in the absorption spectrum (Fig. 5-8). A complementary detection strategy for the electron-poor, analytes was also developed, which enabled the detection of histamine and phenethylamine through an increase in the emission of **ANR-Y2** (Fig. 2c), **ANR-L3** and **ANR-L5.** (Fig. 12-13). The working principle is the deaggregation of self-aggregating dyes, followed by a concomitant enhancement of the fluorescence of the monomer.

Low concentration of the neurotransmitters can be readily detected by the ANRs owing to the high sensitivity of fluorescence emission, as is exemplified for the response to ultra-low concentration (50 nM) of serotonin (Fig. 2b and Fig. 14). In order to quantify the binding strengths, dissociation constants ($K_d$) were determined from fluorescence titration experiments for various combinations of representative analytes (see Fig. 3 for their chemical structures) and six ANRs. High affinities ($K_d$ ranges from 100 nM to 100 μM) were observed for the interactions of the ANRs with the neurotransmitters and trace amines in aqueous media (Tables 1, 3-5). Interestingly, a higher binding strength (~10x) was found in aqueous than in ethanolic media, which indicates the energetically favourable release of frustrated cavity water molecules from the ANR channels upon neurotransmitter binding (see Examples for more details).

**Table 1** : Fitted single site dissociation constants ($K_d$) for representative analytes. Zeolite L-system, ANR-L1, and zeolite Y-assemblies, ANR-Y1 (each receptor 250 μg/mL in 10 mM HEPES buffer, pH 7.3).

| Analyte | biological function | charge | aryl-unit | $K_d$(ANR-L1) µM | $K_d$(ANR-Y1) µM |
|---|---|---|---|---|---|
| serotonin (5-HT) | neurotransmitter | + | 5HO-indole | 3.3 | 0.07 |
| tryptamine | trace amine | + | indole | 10 | 0.15 |
| dopamine | neurotransmitter | + | catechol | 16 | 3.4 |
| norepinephrine | neurotransmitter | + | catechol | 31 | 11 |
| tyramine | trace amine | + | phenol | 27 | 5.7 |
| octopamine | trace amine | + | phenol | 40 | 11 |
| $TrpNH_2$ | - | + | indole | 32 | 0.11 |
| epinephrine | neurotransmitter | + | catechol | >600 | 26 |
| phenylephrine | drug (decongestant) | + | phenol | >600 | 55 |
| phenethylamine | trace amine | + | benzene | n.s. | n.s. |
| histamine | neurotransmitter | + | imidazole | n.s. | n.s. |
| nicotine | stimulant drug, toxin | + | pyridine | n.s. | n.s. |
| propranolol | drug (beta blocker) | + | 1-naphthol | >1000 | >1000 |
| L-DOPA | drug (dopamine precursor) | + – | catechol | >>1000 | >>1000 |
| phenylalanine | amino acid | + – | benzene | >>1000 | >>1000 |
| tyrosine | amino acid | + – | phenol | >>1000 | >>1000 |
| histidine | amino acid | + – | imidazole | >>1000 | >>1000 |

| | | | | | |
|---|---|---|---|---|---|
| tryptophan (Trp) | amino acid | + – | indole | >>1000 | >>1000 |
| 5-HTP | precursor to 5HT | + – | 5HO-indole | >>1000 | >>1000 |
| TrpGly | occurs in hypophysis | + – | indole | >>1000 | >>1000 |
| indole-3-acetic acid | plant hormone | – | indole | >>1000 | >>1000 |
| melatonin | hormone | no | 5MeO-indole | >>1000 | >>1000 |
| adenosine | neuromodulator | no | purine | >>1000 | >>1000 |
| estradiol | hormone | no | phenol | >>1000 | >>1000 |
| propanil | herbicide | no | benzamide | >>1000 | >>1000 |
| indole | - | no | indole | >>1000 | >>1000 |
| catechol | - | no | catechol | >>1000 | >>1000 |
| paracetamol | drug (analgesic) | no | phenol | >>1000 | >>1000 |
| raspberry ketone | natural aroma | no | phenol | >>1000 | >>1000 |
| acetylcholin | neurotransmitter | + | no | >>1000 | >>1000 |
| glycine (Gly) | neurotransmitter | + – | no | >>1000 | >>1000 |
| D-serine | neurotransmitter | + – | no | >>1000 | >>1000 |
| aspartate | neurotransmitter | + – | no | >>1000 | >>1000 |
| glutamate | neurotransmitter | + – | no | >>1000 | >>1000 |
| GABA | neurotransm | + – | no | >>1000 | >>1000 |

| | | | | | |
|---|---|---|---|---|---|
| | itter | | | | |
| cadaverine | toxin | ++ | no | >>1000 | >>1000 |
| ethanolamine | - | + | no | >>1000 | >>1000 |
| glucose | sugar | no | no | >>1000 | >>1000 |

ANRs were prepared from commercial, small-size zeolites (L-type 50 nm; Y-type 900 nm). $K_d$ values with a >> sign denote less than 10% change in emission intensity at >50 molar excess of the analyte. Errors in $K_d$, determined by repetitive experiments, are estimated less than ±20%. n. s.= Receptors **ANR-L1** and **ANR-Y1** are not suitable for the detection of the electron-poor analytes phenethylamine, histamine and nicotine. Their detection is possible with **ANR-Y2**, **ANR-L3** and **ANR-L5**, see Fig. 2c and the Examples.

While not yet achieving the record values of nature, $K_d$ ~ 1 nM, for serotonin binding to the natural 5-HT7 receptor protein, for serotonin binding to **ANR-Y1** we observed an impressive $K_d$~ 70 nM. Our zeolite-based ANRs are already superior compared to other *artificial* receptors, *e.g.* $K_d$(5-HT) ~ 15 μM with the high-affinity macrocycle cucurbit[7]uril.**[9]** Besides, unlike natural protein receptors or most synthetic hosts, the zeolite-based ANRs have a *built-in* spectroscopic response upon neurotransmitter binding, which facilitates their use as chemosensors.

In order to investigate into the selectivity of the ANRs towards the neurotransmitters and structural analogues, ANR-responses to 37 representative substances were determined (Fig. 3 and Table 1). A very strong charge-mediated selectivity was generally observed. For instance, the binding affinity of the net positively charged, unnatural, amidated tryptophan ($TrpNH_2$) with **ANR-Y1** and **ANR-Y2** is large, $K_d$ ~ 110 nM and 2.7 μM, respectively. For comparison, the artificial receptor **CB8-D1** that is self-assembled from the macrocycle cucurbit[8]uril (CB8; max. cavity diameter ~9 Å) and the dye **D1** possesses an affinity of 2.2 μM for $TrpNF_2$ in water. The similarity in binding affinity for **ANR-Y1** and **CB8-D1** can be rationalized by a comparable water release contribution from the similarly sized cavities of zeolite Y1 and CB8, and by equally strong aryl-aryl stacking interactions between the dye and indoyl-moiety of $TrpNH_2$. The latter are spectroscopically indicated by charge-transfer bands in the absorption spectrum (Examples). However, striking differences between the zeolite- and CB8-based sensors are observed with respect to the guest selectivity; The **CB8-D1** receptor is nearly equally strongly binding $TrpNH_2$ ($K_d$ ~ 2.2 μM) and its zwitterionic parent amino acid tryptophan (Trp, $K_d$ ~ 6.3 μM). In fact, comparably low selectivity difference (<100) are typically observed for the complexation of organic molecules by synthetic hosts in water. In stark contrast, the zeolite-based ANRs reach charge-selectivity factors in $K_d$ of about 10,000 (see Fig. 2d and Fig. 15-18). To exemplify, 5 μM of serotonin could be clearly detected by **ANR-L1** in the presence of even 50,000 μM Trp (Fig. 19). Moreover, also non-charged species, such as catechol, melatonin and indole, are only weakly bound by the ANRs, which is in stark contrast to the findings for their derived catecholamine neurotransmitters (dopamine, epinephrine and norepinephrine) and serotonin/tryptamine (Fig. 1d-e).

The pore-size of the ANRs has an important role in determining the size-related binding-selectivity. It was observed that dopamine and norepinephrine are strongly bound by zeolite-L based ARNs, e.g. $K_d$(norepinephrine) = 13 μM with **ANR-L2,** while the slightly larger epinephrine and phenylephrine are only weakly bound, e.g. $K_d$(epinephrine) = 240 μM with **ANR-L2** (Fig. 2e and Fig. 20-25). Strikingly, the binding selectivity factors (~20, see Table 2) of the zeolite L-type artificial receptors are thus higher than of the natural $\alpha_{2A}$-adrenergic receptor and the dopamine $D_4$-receptors (~4). Conversely, the larger-cavity zeolite-Y-based receptors are less size-selective, e.g. $K_d$(norepinephrine) = 26 μM and $K_d$(epinephrine) = 39 μM with **ANR-L2.**

**Table 2** | Relative binding selectivity for natural receptor proteins and the zeolite-based artificial receptors for dopamine, norepinephrine and epinephrine.

| receptor system | scaffold | Selectivity dopamine *vs.* (*R*)-epinephrine | Selectivity (*R*)-norepinephrine *vs.* (*R*)-epinephrine |
|---|---|---|---|
| **$\alpha_{2A}$-adrenergic receptor**[23] | protein | 13 – 25* | 3 – 4* |
| **dopamine $D_4$-receptor**[24] | protein | 15 | 3 – 4* |
| **ANR-L1** | zeolite L | ≥40 | ≥20 |
| **ANR-L2** | zeolite L | 52 | 18 |
| **ANR-L3** | zeolite L | ≥40 | ≥20 |
| **ANR-L4** | zeolite L | 32 | 25 |
| **ANR-Y1** | zeolite Y | 8 | <3 |
| **ANR-Y2** | zeolite Y | 4 | <2 |

The protein-based receptor studies were carried out in 50 mM $K^+$-phosphate and in Tris-buffer with added membrane components.**[10, 11]** The binding assays with the ANRs were performed in 10 mM HEPES buffer.

* range of selectivity values was reported by different assay types

In addition to charge- and size-mediated selectivity constraints, it should be noted that only analytes that carry an aromatic recognition motif are expected to cause significant changes in the emission intensity of the sensors. Thus, of all naturally occurring substances tested, only the aromatic amine neurotransmitters and aromatic trace amines gave rise to a strong spectroscopic response while other neurotransmitters, hormones, drugs, amino acids, peptides and alkyl amines showed small effects (Table 1 and Fig. 3).

The modular design of the ANRs allows to address additional requirements for practically applicable sensors. Firstly, co-inclusion of an analyte-responsive reporter dye with a non-responsive reference dye provides a simple access to ratiometric sensing schemes (Fig. 26). Secondly, neurotransmitters and trace amines can be distinguished from each other by utilizing differently selective receptors in combination with a principal component analysis **[12]** (Figure 27-29). Thirdly, covalent or non-covalent surface functionalization of the ANRs is possible, effectively providing sensors that are resistant to agglomeration and protein-adherence (*e.g.* through PEGylation or zwitterions)[**13**] or those, which are readily uptaken by cells (*e.g.* through coating with polylysine).

The monitoring of dynamic processes (*e.g.* uptake of drugs, enzymatic production and destruction of naturally occurring compounds) in complex media, such as inside living cells, is an emerging field of great biological and medicinal relevance. We were pleased to note that the ANRs remain functional in cell culture media and blood serum (Fig. 30-32) and even remain intact after cellular uptake by living Rattus norvegicus C6 glioma cells (Fig. 33). As result, we were able to follow the delivery of serotonin by surface-coated **ANR-L1** into living C6 glioma cells, and the subsequent dynamic intracellular release of the serotonin-cargo (Fig. 34). The monitoring of the delivery of other drugs and of enzymatic

reactions in real time is also a possible application of the conjugates/artificial receptors of the invention. As a proof of principle, we have successfully monitored the oxidative degradation of serotonin by laccase utilizing surface-PEGylated **ANR-L1** (Fig. 35). The extension to medicinally important enzymatic processes, such as the conversion of the Parkinson's disease prodrug L-DOPA into dopamine by aromatic L-amino acid decarboxylase, is also within the range of possible applications of the invention, given the very large affinity differences between the substrate and product pair (Table 1). Furthermore, use of additional reporter dyes, e.g. those that are responsive towards other functional groups such amines or thiols, also falls within the scope of the present invention.

Applying the lessons learnt for synthetic hosts and natural proteins about the energetic importance of the non-classical hydrophobic effect for binding of analytes/ligands in aqueous media, it has been demonstrated that porous inorganic materials provide a platform for high-affinity binding. The herein introduced strategy relies on the use of nanoporous inorganic frameworks (pore size ~ 1 nm) as opposed to the frequently employed mesoporous (pore size > 2 nm) or nonporous spherical nanoparticles. Specifically, highly selective, artificial receptors for positively charged, aromatic neurotransmitters were provided starting from negatively-charged, nanoporous zeolite L and zeolite Y that were loaded with an aryl-moiety selective reporter dyes. Other nanoporous materials such as silicas, metal organic frameworks (MOFs) or covalent organic frameworks (COFs), may be used as high-affinity binders for different analyte classes, depending on their charge and atomic composition. Moreover, the herein demonstrated convenient signal transduction strategy, through co-inclusion of an emissive reporter dye into the porous framework, can find important applications because of its sensitivity and practical ease.

While we have described a number of embodiments of this invention, it is apparent that our basic examples may be altered to provide other embodiments that utilize the catalysts and methods of this invention. Therefore, it will be appreciated that the scope of this invention is to be defined by the appended claims rather than by the specific embodiments that have been represented by way of example.

## LIST OF REFERENCES

[1] Schmidt, A.C., Wang, X., Zhu, Y. & Sombers, L.A. Carbon Nanotube Yarn Electrodes for Enhanced Detection of Neurotransmitter Dynamics in Live Brain Tissue. ACS Nano 7, 7864-7873 (2013).

[2] Boersma, A.J., Brain, K.L. & Bayley, H. Real-Time Stochastic Detection of Multiple Neurotransmitters with a Protein Nanopore. ACS Nano 6, 5304-5308 (2012).

[3] Kruss, S. et al. Neurotransmitter Detection Using Corona Phase Molecular Recognition on Fluorescent Single-Walled Carbon Nanotube Sensors. Journal of the American Chemical Society 136, 713-724 (2013).

[4] Kasera, S., Herrmann, L.O., Barrio, J.d., Baumberg, J.J. & Scherman, O.A. Quantitative multiplexing with nano-self-assemblies in SERS. Sci. Rep. 4 (2014).

[5] Basuray, A. N. et al. The Chameleonic Nature of Diazaperopyrenium Recognition Processes. Angew. Chem. Int. Ed. 51, 11872-11877, (2012).

[6] Hartlieb, K. J. et al. Anticancer Activity Expressed by a Library of 2,9-Diazaperopyrenium Dications. ACS Nano 9, 1461-1470, (2015).

[7] Biedermann, F. et al. Benzobis(imidazolium)-cucurbit[8]uril complexes for binding and sensing aromatic compounds in aqueous solution. Chem. Eur. J. 16, 13716-13722, (2010).

[8] Calzaferri, G., Huber, S., Maas, H. & Minkowski, C. Host-Guest Antenna Materials. Angew. Chem. Int. Ed. 42, 3732-3758, (2003).

[9] Kasera, S., Herrmann, L.O., Barrio, J.d., Baumberg, J.J. & Scherman, O.A. Quantitative multiplexing with nano-self-assemblies in SERS. Sci. Rep. 4 (2014).

[10] Nyrönen, T. et al. Molecular Mechanism for Agonist-Promoted $\alpha$2A-Adrenoceptor Activation by Norepinephrine and Epinephrine. Mol. Pharmacol. 59, 1343-1354 (2001).

[11] Lanau, F., Zenner, M.-T., Civelli, O. & Hartman, D.S. Epinephrine and Norepinephrine Act as Potent Agonists at the Recombinant Human Dopamine D4 Receptor. J. Neurochem. 68, 804-812 (1997).

[12] Stewart, S., Ivy, M.A. & Anslyn, E.V. The use of principal component analysis and discriminant analysis in differential sensing routines. Chemical Society Reviews (2013).

[13] Garcia, K.P. et al. Zwitterionic-Coated "Stealth" Nanoparticles for Biomedical Applications: Recent Advances in Countering Biomolecular Corona Formation and Uptake by the Mononuclear Phagocyte System. Small 10, 2516-2529 (2014).

[14] A. Zabala Ruiz, D. Bruhwiler, T. Ban, G. Calzaferri, Monatshefte fur Chemie 136 (2005) 77

[15] S. Megelski, G. Calzaferri Adv. Funct. Mater. 2001, 11, 277.

[16] Breck in Zeolite Molecular Sieves, 752, Wiley, New York, 1974.

[17] Baerlocher et al., in Atlas of Zeolite Framework Types, 19, Elsevier, Amsterdam, 5th edition, 2001.

[18] J. Pyun, S. Jia, T. Kowalewski, G.D. Patterson, K. Matyjaszewski, Macromolecules, 2003, 36, 5094-5104.
[19] C. Sanchez, B. Julian, P. Belleville, M.Popall, J. mater. Chem. 2005, 15, 3559-3592.
[20] S.R. Hall, S.S. Davis, S. Mann, Langmuir, 2000, 16, 1454-1456.
[21] Inagaki, S.; Guan, S.; Ohsuna, T.; Terasaki, O. Nature 2002, 416, 304-307.
[22] Asefa, T.; MacLachlan, M. J.; Coombs, N.; Ozin, G. A. Nature 1999, 402, 867-871.
[23] Biedermann, F. & Nau, W. M. Noncovalent Chirality Sensing Ensembles for the Detection and Reaction Monitoring of Amino Acids, Peptides, Proteins, and Aromatic Drugs. Angew. Chem. Int. Ed., 5694-5699 (2014).
[24] S. Saravanamurugan, Sujandi, E. A. Prasetyanto and S.-E. Park, "Liquid-phase reaction of 2'-hydroxyacetophenone and benzaldehyde over SO3H-SBA-15 catalysts: Influence of microwave and thermal effects" Microporous Mesoporous Mater., 2008, 112, 97.
[25] Y. Satoh, Y. Yokoyama, I. Ogino and S. R. Mukai, Industrial & Engineering Chemistry Research, 2013, 52, 15293-15297.
[26] Z. Chen, R. Xu, Y. Zhang and N. Gu, Nanoscale Research Letters, 2008, 4, 204-209.
[27] J. Tian, L. V. Saraf, B. Schwenzer, S. M. Taylor, E. K. Brechin, J. Liu, S. J. Dalgarno and P. K. Thallapally, J. Am. Chem. Soc., 2012, 134, 9581-9584.
[28] A. Karmakar, A. V. Desai and S. K. Ghosh, Coord. Chem. Rev., 2016,307, Part 2, 313-341.
[29] A. Karmakar, A. V. Desai and S. K. Ghosh, Coord. Chem. Rev., 2016,307, Part 2, 313-341.
[30] D. Karami and S. Rohani, Industrial & Engineering Chemistry Research, 2009, 48, 4837-4843.
[31] Ding, S.-Y. & Wang, W. Covalent organic frameworks (COFs): from design to applications. Chem. Soc. Rev. 42, 548-568 (2013).
[32] Sindelar, V. et al. Supramolecular Assembly of 2,7-Dimethyldiazapyrenium and Cucurbit[8]uril: A New Fluorescent Host for Detection of Catechol and Dopamine. Chemistry - A European Journal 11, 7054-7059 (2005).

All patent(s) and publication(s) mentioned in this specification are herein incorporated by reference to the same extent as if each individual publication was specifically and individually indicated to be incorporated by reference.

## Claims

1. A conjugate comprising a nanoporous crystalline three-dimensional material bearing a permament negative framework charge and having an average pore size ranging from 1 nm to 5 nm, preferably 1 nm to 2 nm, selected from a zeolite, an organosilica, a metal organic framework (MOF) and a covalent organic framework (COF) material, containing within its pores an electrophilic UV-visible or fluoresecent analyte-responsive reporter dye, wherein the reporter dye is non-covalently bound to the nanoporous framework material inner pore surface;
providing that when the nanoporous crystalline three-dimensional framework material is a zeolite, the material does not comprise a stop-cock moiety and an affinity binding agent covalently bound thereto.

2. The conjugate of claim 1, wherein the nanoporous framework material is further functionalized at the outer surface with an organic surface agent selected from:

    - a polymer, for instance polyethylene glycol (PEG), polyvinyl alcohol, polylysine or polyethyleneimine;
    - an oligosaccharide, for instance cyclodextrins monomers, oligomers or polymers;

        - a polysaccharide, for instance chitosan, dextran, fucoidan, alginate, pectin, amylose, starch, cellulose or xylan; or
        - a targeting molecule such as a biotin, avidin, folic acid, lipoic acid, ascorbic acid, an antibody or antibody fragment, a peptide, a protein or a DNA, RNA or PNA moiety.

3. The conjugate of any one of claims 1-2, wherein the reporter dye can reversibly bind small molecule analytes bearing at least one aromatic or heteroaromatic moiety and an overall positive charge, wherein the small molecule has a rigid section smaller than the nanoporous framework material average pore size.

4. The conjugate of any one of claims 1-3, wherein the small molecule analyte bears at least one functional group that is protonated under physiological conditions.

5. The conjugate of claim 4, wherein the functional group is a primary, secondary or tertiary amino group, preferably a primary amino group.

**6.** The conjugate of claim 5, wherein the small molecule analyte is serotonin, tryptamine, dopamine, norepinephrine, tyramine, octopamine, $TrpNH_2$, epinephrine, phenylepinephrine, phenethylamine, histamine, nicotine or propranolol.

**7.** The conjugate of any one of claims 3 to 6, wherein the reporter dye is a dicationic moiety, such as one of the following:

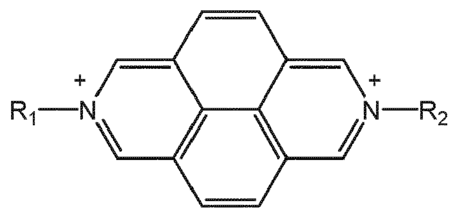

;

;

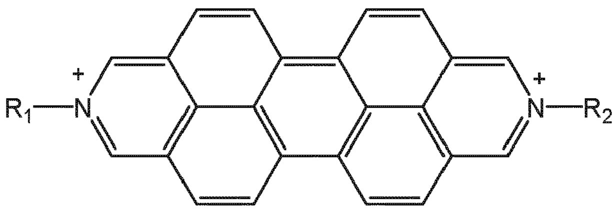

;

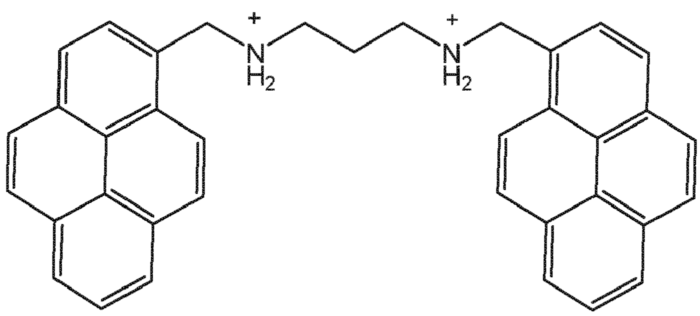

;

or

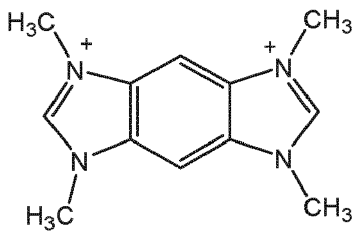

wherein $R_1$ and $R_2$ independently represent a C1-6alkyl moiety or a phenylC1-3alkyl moiety, preferably methyl or benzyl.

**8.** The conjugate of any one of claims 1-2, wherein the reporter dye can covalently bind small molecules bearing at least one free thiol moiety and an overall positive charge, wherein the small molecule has a rigid section smaller than the nanoporous framework material average pore size; such as cysteamine.

**9.** The conjugate of claim 8, wherein the reporter dye is a thiol-reactive dye, selected from coumarin maleimides and iodoacetamides, pyrene maleimides and iodoacetamides, naphthalene-based dyes, bimane-based dyes, such as:

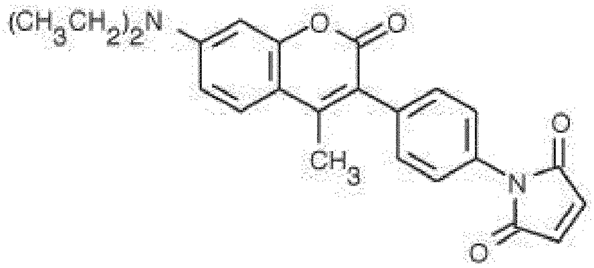

;

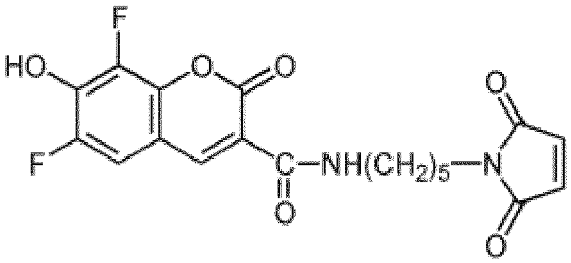

;

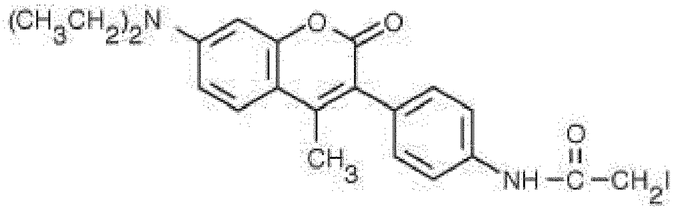

;

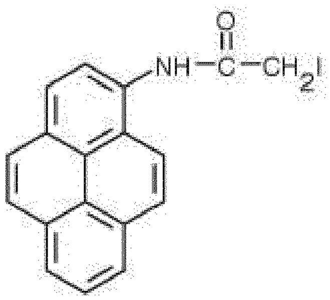

;

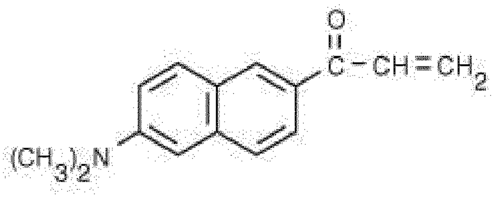

;

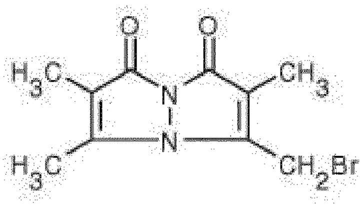

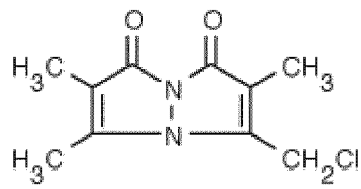

or

**10.** The conjugate of claim 8 or 9, wherein the reporter dye is a thiol-reactive dye having the structure:

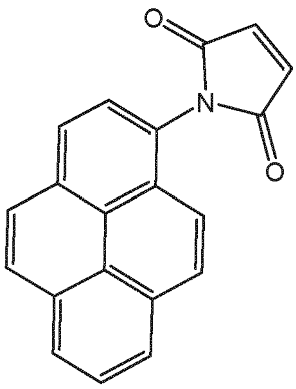

.

11. The conjugate of any one of claims 1-10, wherein the nanoporous framework material further comprises an analyte-non-responsive reference dye bearing a perylene-bisdiimide moiety encapsulated within its pores, such as:

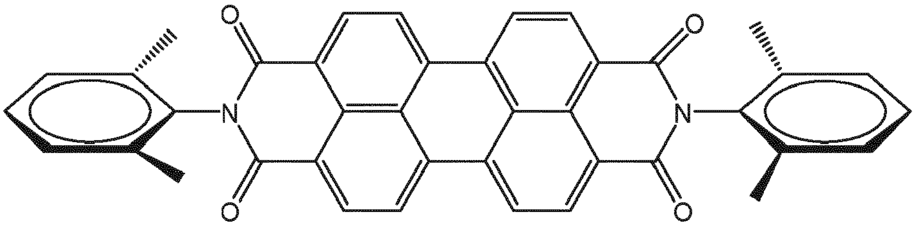

.

12. The conjugate of any one of the preceding claims wherein the nanoporous crystalline three-dimensional framework material is a zeolite.

13. The conjugate of claim 1, wherein the zeolite is zeolite Y or L.

14. A method for producing a conjugate according to any one of claims 1-11, the method comprising the step of immersing a nanoporous framework material having an average pore size ranging from 1 nm to 2 nm, selected from a zeolite, an organosilica, a metal organic framework (MOF) and a covalent organic framework (COF) material, in a solution of an electrophilic UV-visible or fluorescent analyte-responsive reporter dye, wherein the reporter dye has a rigid section smaller than the nanoporous framework material average pore size.

15. A method according to claim 14, wherein the reporter dye is as defined in any one of claims 3-10.

16. A method according to claim 14, wherein the solution in which the nanoporous framework material is immersed, further comprises an analyte-non-responsive reference dye bearing a perylene-bisdiimide moiety as defined in claim 11.

17. The method of any one of claims 14-16, wherein the nanoporous crystalline three-dimensional framework material is a zeolite.

18. The method of claim 17, wherein the zeolite is zeolite Y or L.

19. Use of a conjugate according to any of claims 1 to 11 for selectively detecting molecules in a fluid sample, such as biofluids like blood; wherein the molecule has a rigid section smaller than the nanoporous framework material average pore size, bears an overall positive charge, and comprises an aryl moiety and/or a free thiol moiety.

20. Use according to claim 19, wherein the molecule is an electron-rich aryl-containing neurotransmitter, drug/pharmaceutically active compound, or trace amine.

21. Use according to claim 19 or 20, wherein the molecule is serotonin, dopamine, amidated tryptophan (TrpNH2), epinephrine, norepinephrine, tryptamine, tyramine, octopamine, or propranolol.

**22.** Use of a conjugate according to claim 11 for selectively detecting molecules in fluid samples, such as biofluids like blood; wherein the conjugate further comprises a non analyte-responsive reference dye bearing a perylene-bisdiimide moiety encapsulated within its pores, such as:

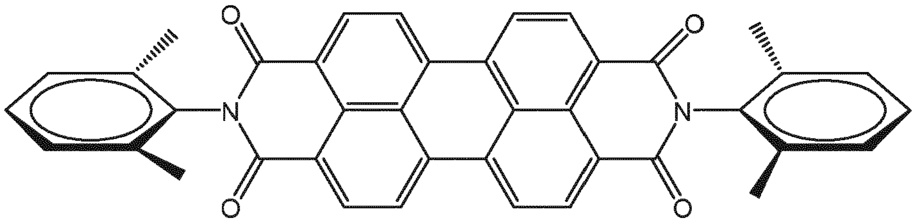
;

and the molecule is an electron-poor positively charged aryl-containing compound having a rigid section smaller than the nanoporous framework material average pore size, such as phenethylamine, histamine or nicotine.

**23.** Use according to claim 22, wherein the electron-poor aryl-containing compound favors dye-aggregation within the nanoporous framework material pores, for example via $\pi$-$\pi$ stacking.

**24.** Use of a conjugate according to any of claims 1 to 11 as drug-capture/release monitoring system; wherein the drug has a rigid section smaller than the nanoporous framework material pore size, bears an overall positive charge, and comprises an aryl moiety and/or a free thiol moiety.

**25.** Use of a conjugate according to any of claims 1 to 11 for real-time minotoring of chemical or enzymatic reactions.

**26.** Use according to claim 25, wherein the chemical reaction is the degradation of serotonin.

**27.** Use according to claim 25, wherein the enzymatic reaction is the enzymatic conversion of Parkinson's pro-drug L-DOPA into dopamine by L-DOPA decarboxylase.

**28.** A method for detecting an analyte, the method comprising the steps of

a) providing a fluid sample suspected or known to comprise the analyte;
b) contacting said sample with a conjugate according to any of claims 1 to 11 under conditions appropriate for interaction of the analyte and the reporter dye within the nanoporous framework material pores;
c) measuring any UV-visible or fluorescence emission-quenching response of the reporter dye; thereby detecting the presence of the analyte;

wherein the analyte has a rigid section smaller than the nanoporous framework material average pore size, bears an overall positive charge, and comprises an aryl moiety and/or a free thiol moiety.

**29.** The method of claim 28, wherein the interaction in step (b) is covalent binding, or reversible non-covalent binding ($\pi$-$\pi$ stacking interaction).

**30.** The method of claim 29, wherein the contacting step (b) is carried out in an aqueous or organic solvent, for example water, buffered aqueous medium, physiological fluid, alcohol solvent, ether solvent, alkane solvent or chlorinate solvent, for example $CHCl_3$ or ethanol.

**31.** The method of any one of claims 28-30, wherein the fluid sample comprises a biofluid, wherein the biofluid comprises whole blood, serum, plasma, cerebral spinal fluid, urine, saliva, semen, joint aspirate, wound drainage, fruit juice, or vegetable juice.

**32.** The method of claim 31, wherein the fluid sample is prefiltered or treated to remove impurities prior to contacting with the conjugate.

**33.** The method of claim 31 or 32, wherein the fluid sample further comprises a buffer, a salt, an enzyme, a surfactant, or a combination thereof.

**34.** The method of any one of claims 28-25, wherein the analyte is an electron-rich aryl-containing neurotransmitter, drug/pharmaceutically active compound, or trace amine, such as one defined in claim 21.

**35.** The method of any one of claims 28-34, wherein the analyte is an electron-poor aryl-containing compound having a rigid section smaller than the nanoporous framework material average pore size, such as phenethylamine, histamine or nicotine, and the reporter dye is a dicationic moiety having one of the following structures:

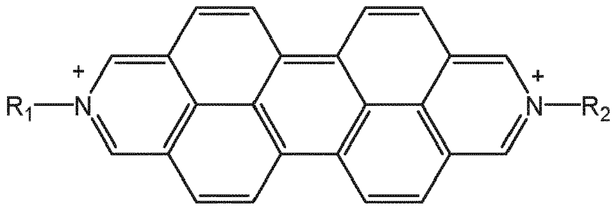

;

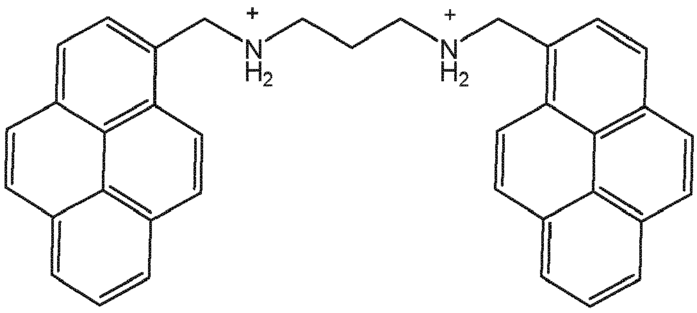

.

**36.** The method of any one of claims 28-34, wherein the analyte is an electron-rich drug/pharmaceutically active compound having a rigid section smaller than the nanoporous framework material average pore size, bearing an overall positive charge, and comprising an aryl moiety and/or a free thiol moiety; and the method allows to monitor drug capture and/or release.

**37.** The method of any one of claims 28-34, wherein the analyte is a substrate of a chemical or enzymatic reaction, such as serotonin and Parkinson's pro-drug L-DOPA.

**38.** The method of any one of claims 28-34, wherein the analyte comprises a free thiol moiety and the reporter dye is a thiol-reactive dye, such as one defined in any one of claims 8-10.

**39.** The method of claim 38, wherein the analyte is cysteamine.

**40.** The use or method of any one of claims 19-39, wherein the nanoporous crystalline three-dimensional framework material is a zeolite.

**41.** The use or method of claim 40, wherein the zeolite is zeolite Y or L.

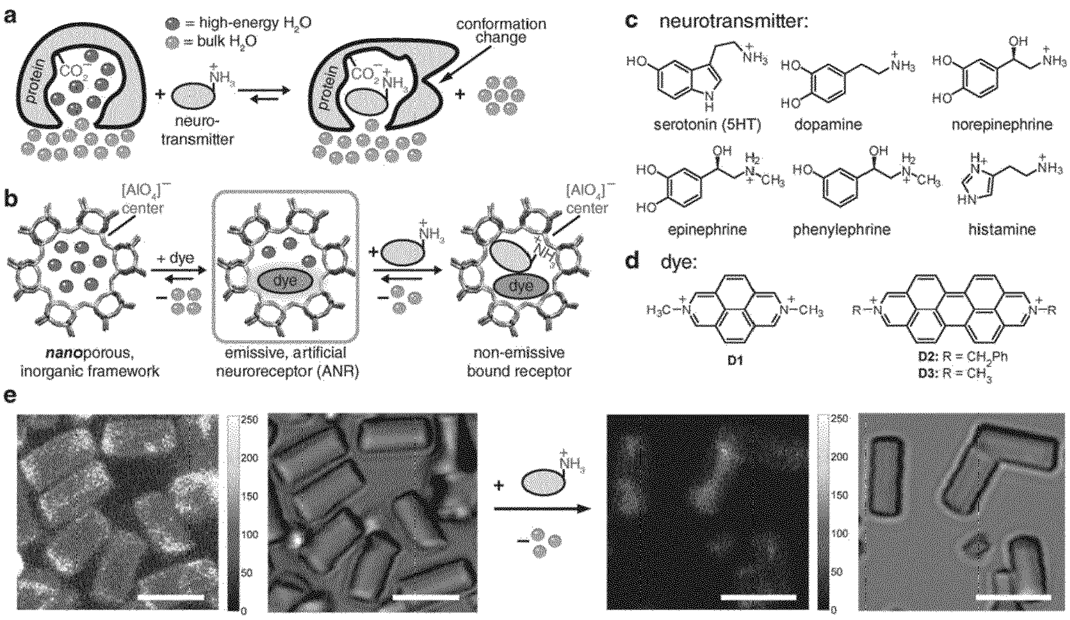


# FIGURE 1

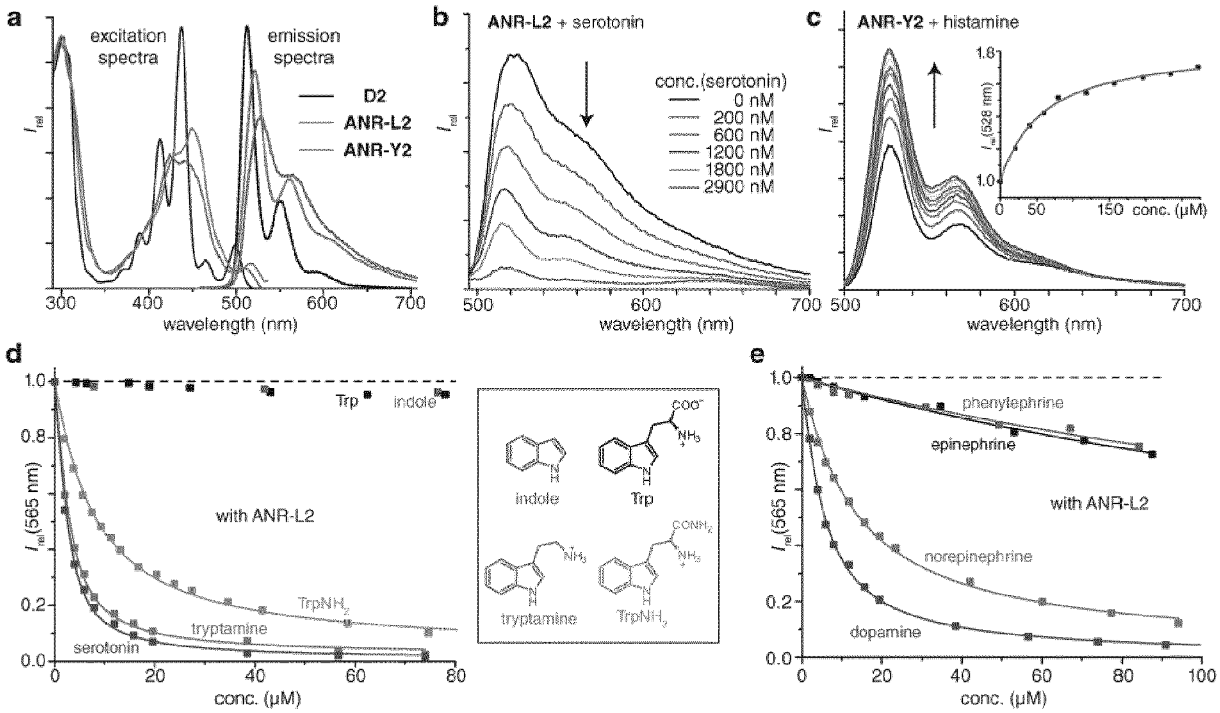


# FIGURE 2

a

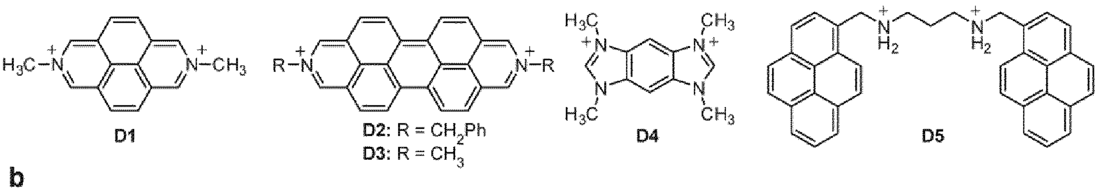

D1

D2: R = $CH_2Ph$

D3: R = $CH_3$

D4

D5

b

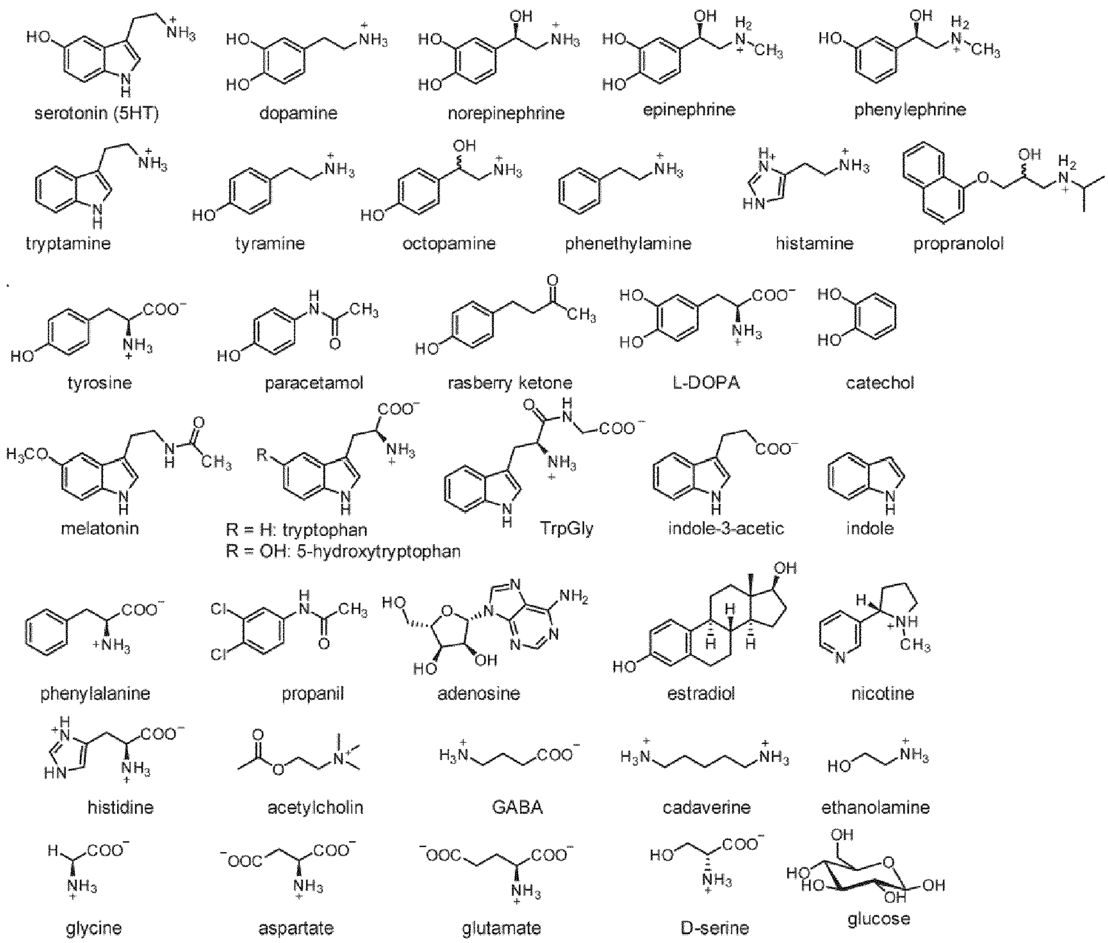

serotonin (5HT), dopamine, norepinephrine, epinephrine, phenylephrine

tryptamine, tyramine, octopamine, phenethylamine, histamine, propranolol

tyrosine, paracetamol, rasberry ketone, L-DOPA, catechol

melatonin, R = H: tryptophan, R = OH: 5-hydroxytryptophan, TrpGly, indole-3-acetic, indole

phenylalanine, propanil, adenosine, estradiol, nicotine

histidine, acetylcholin, GABA, cadaverine, ethanolamine

glycine, aspartate, glutamate, D-serine, glucose

c

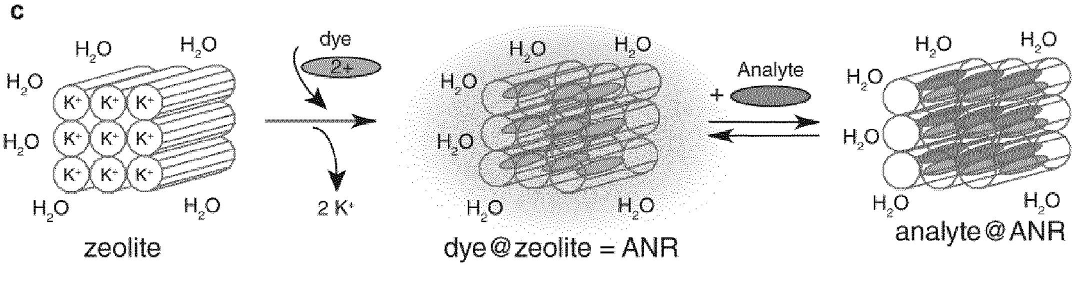


# FIGURE 3

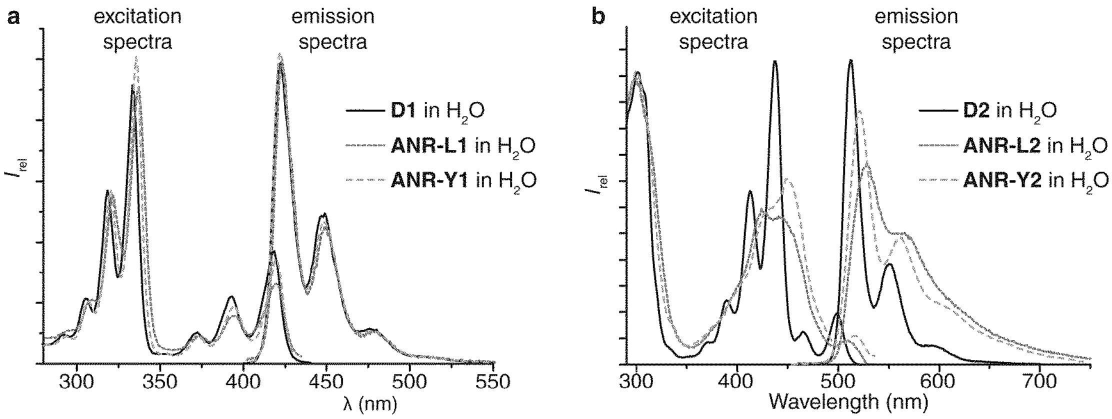


FIGURE 4

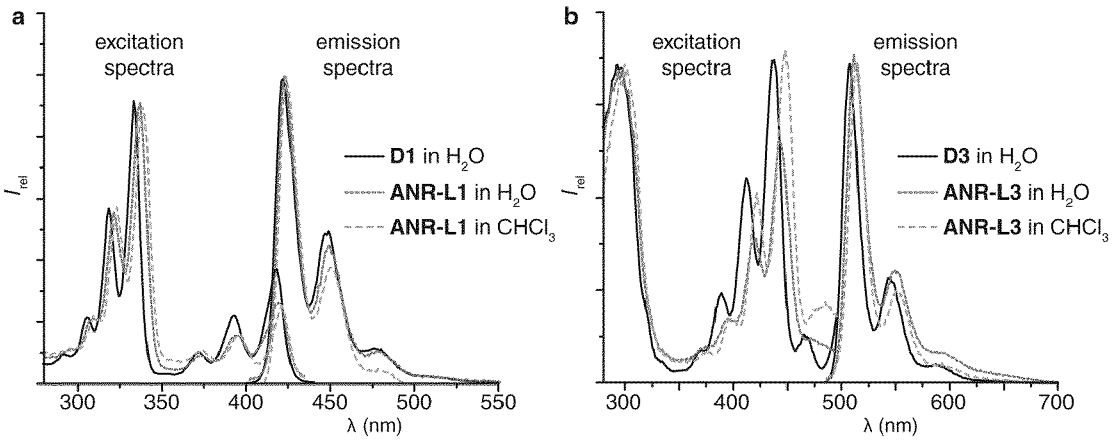


FIGURE 5

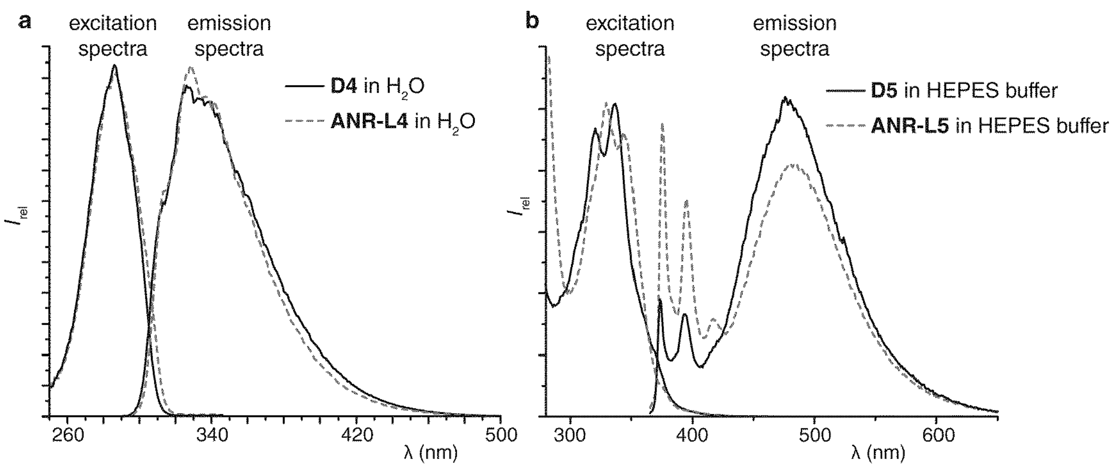


FIGURE 6

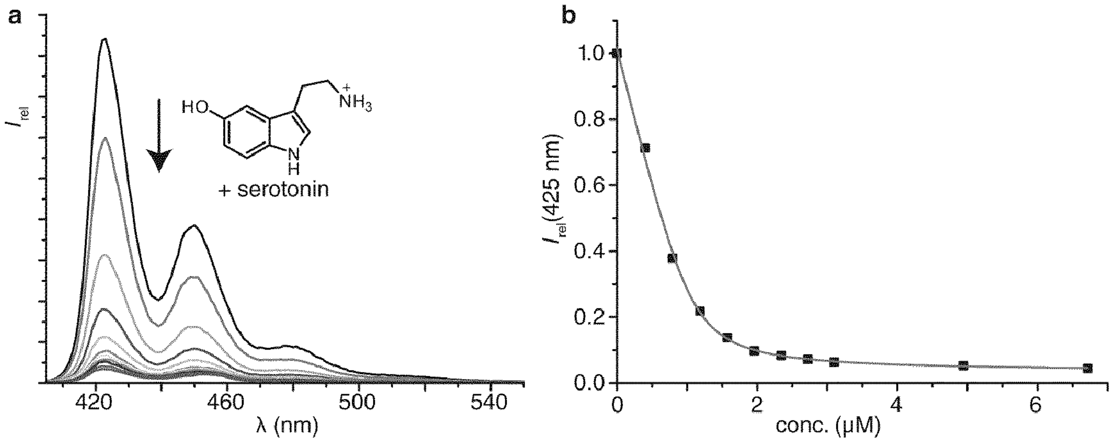


FIGURE 7

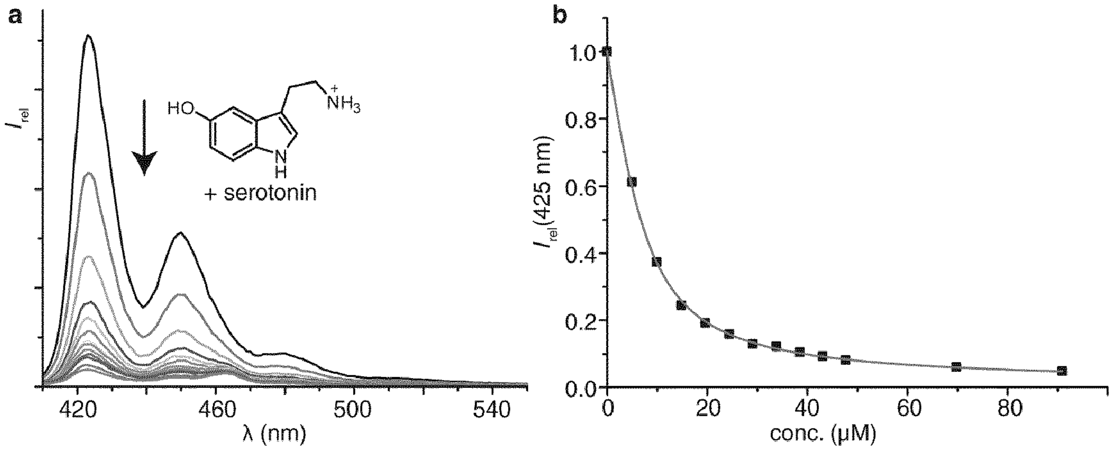


FIGURE 8

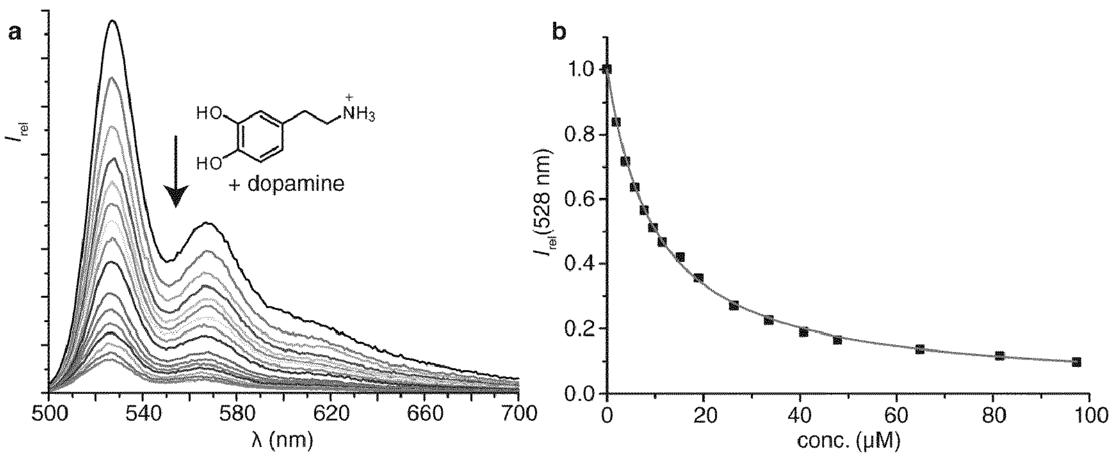


FIGURE 9

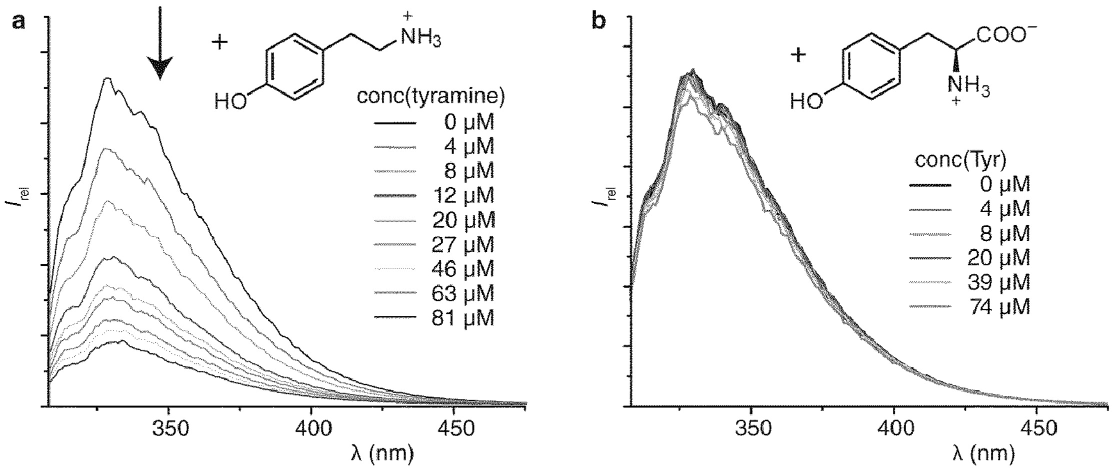


**FIGURE 10**

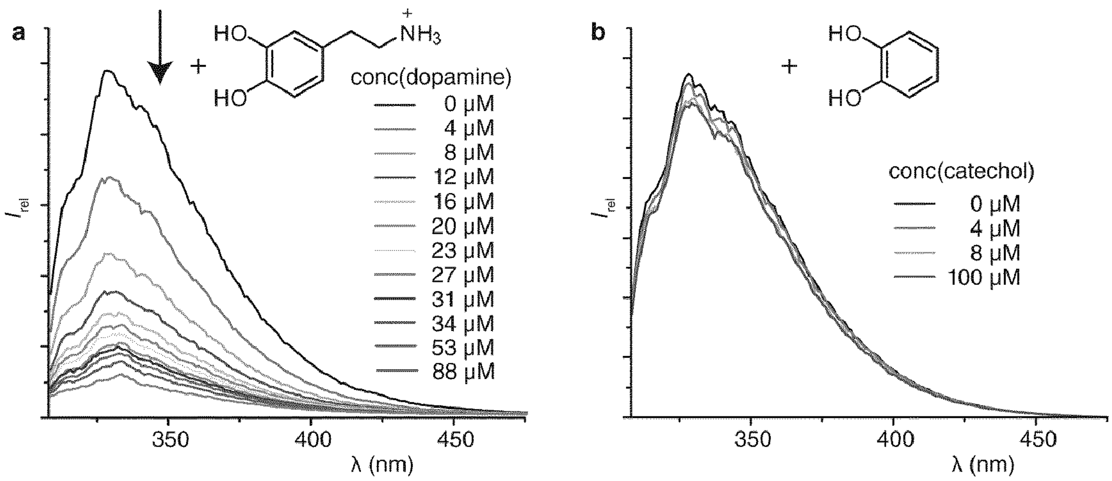


**FIGURE 11**

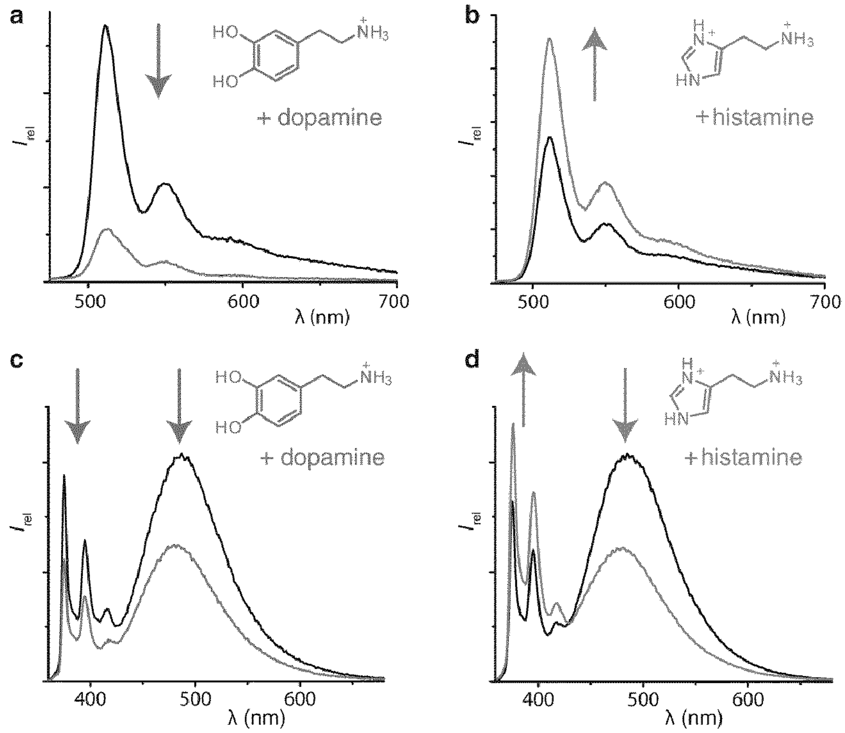


FIGURE 12

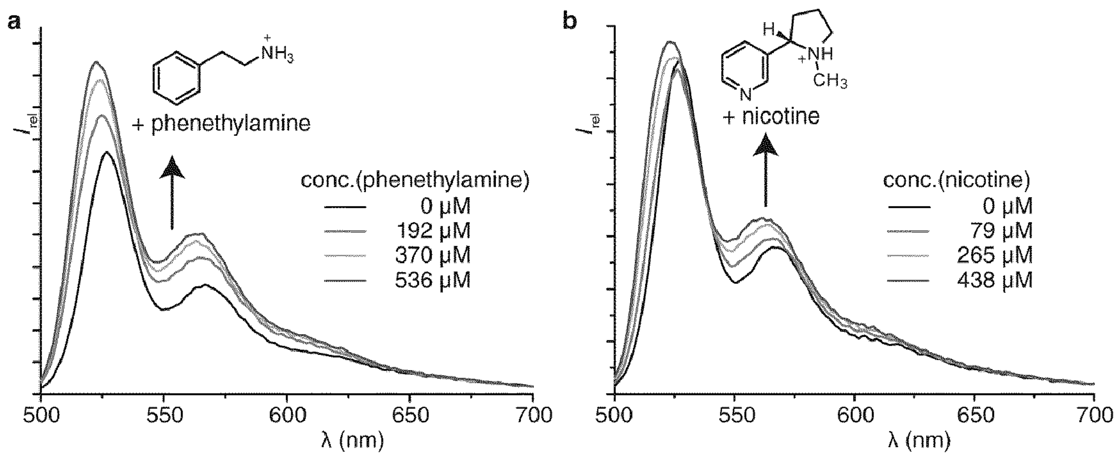


FIGURE 13

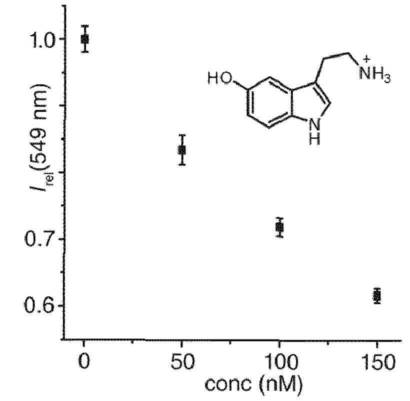


FIGURE 14

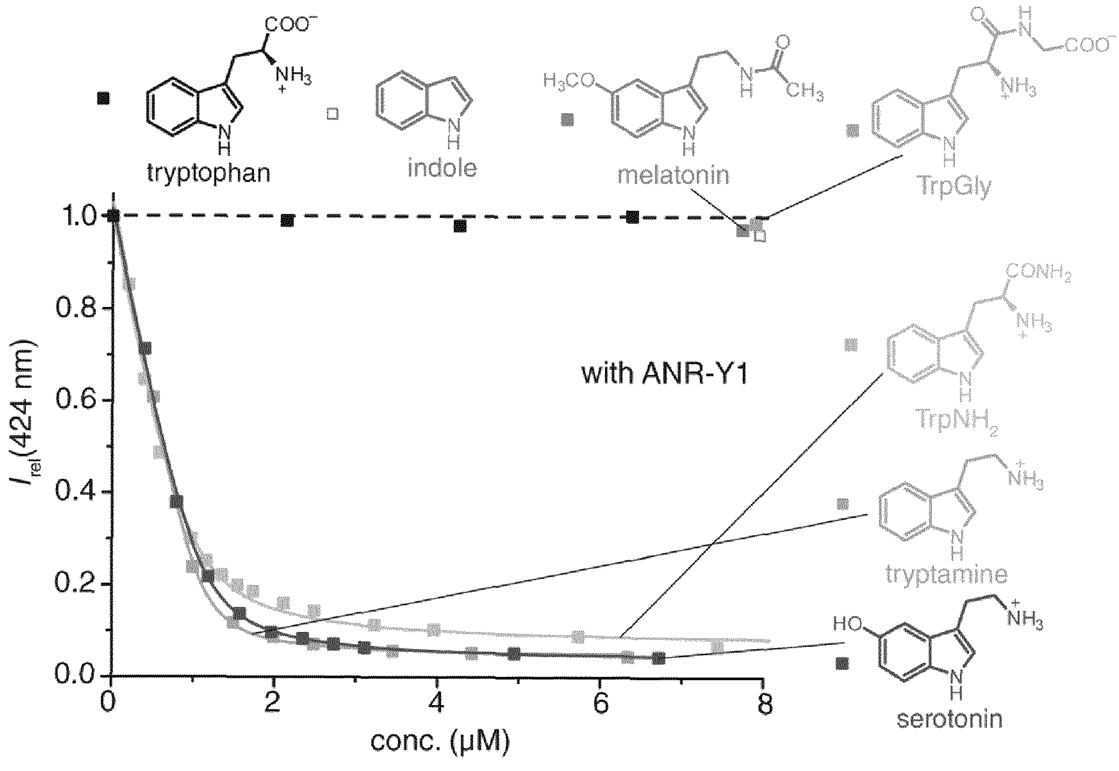


FIGURE 15

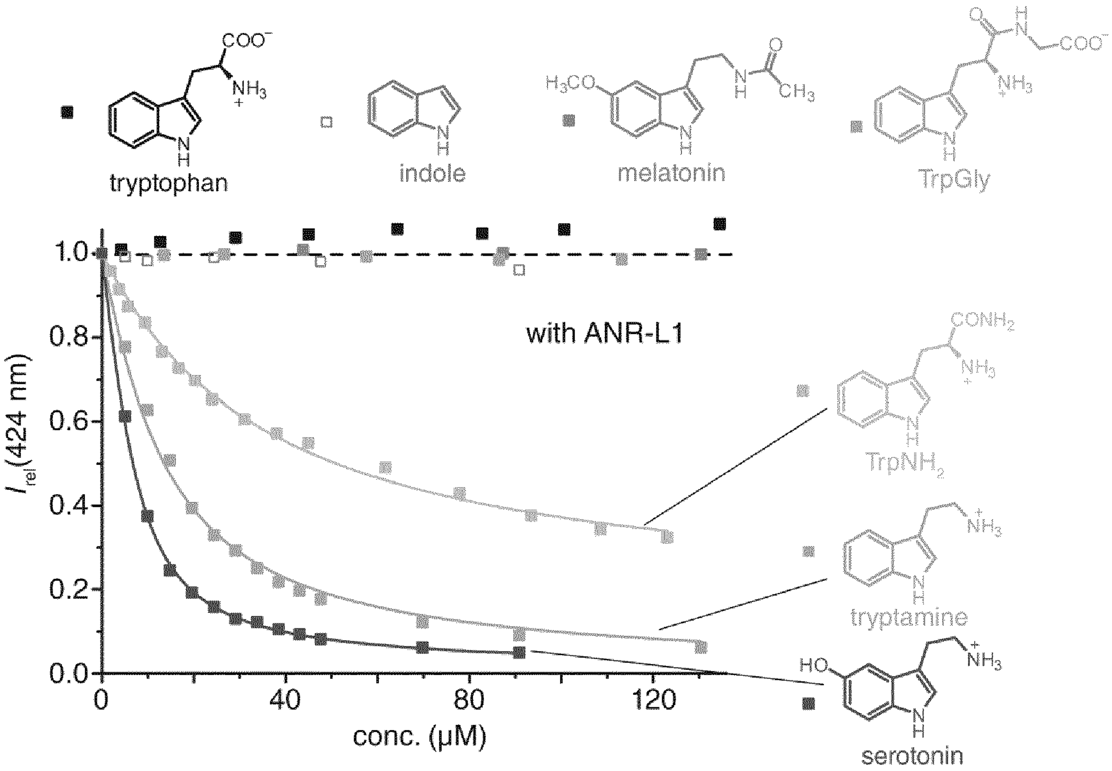


**FIGURE 16**

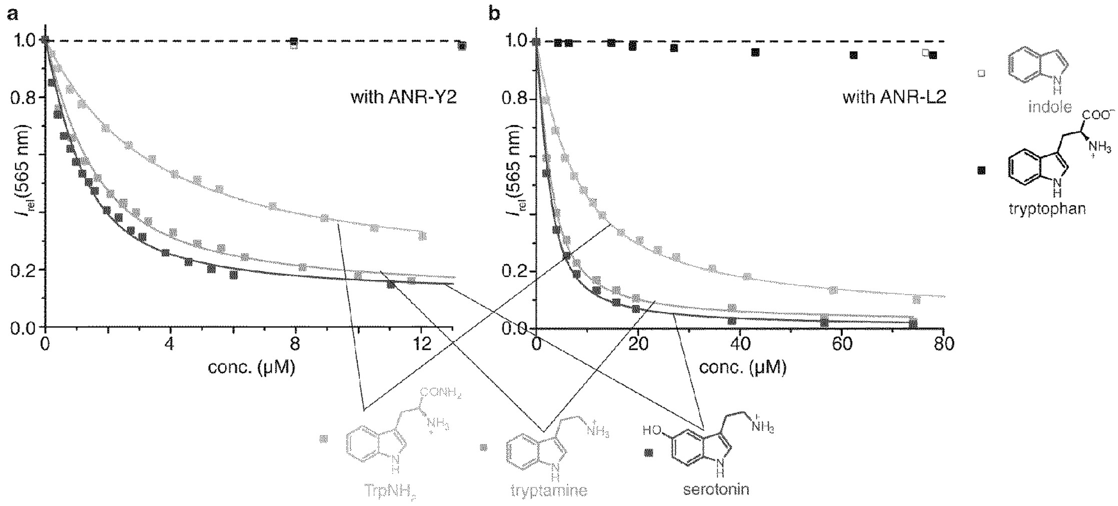


**FIGURE 17**

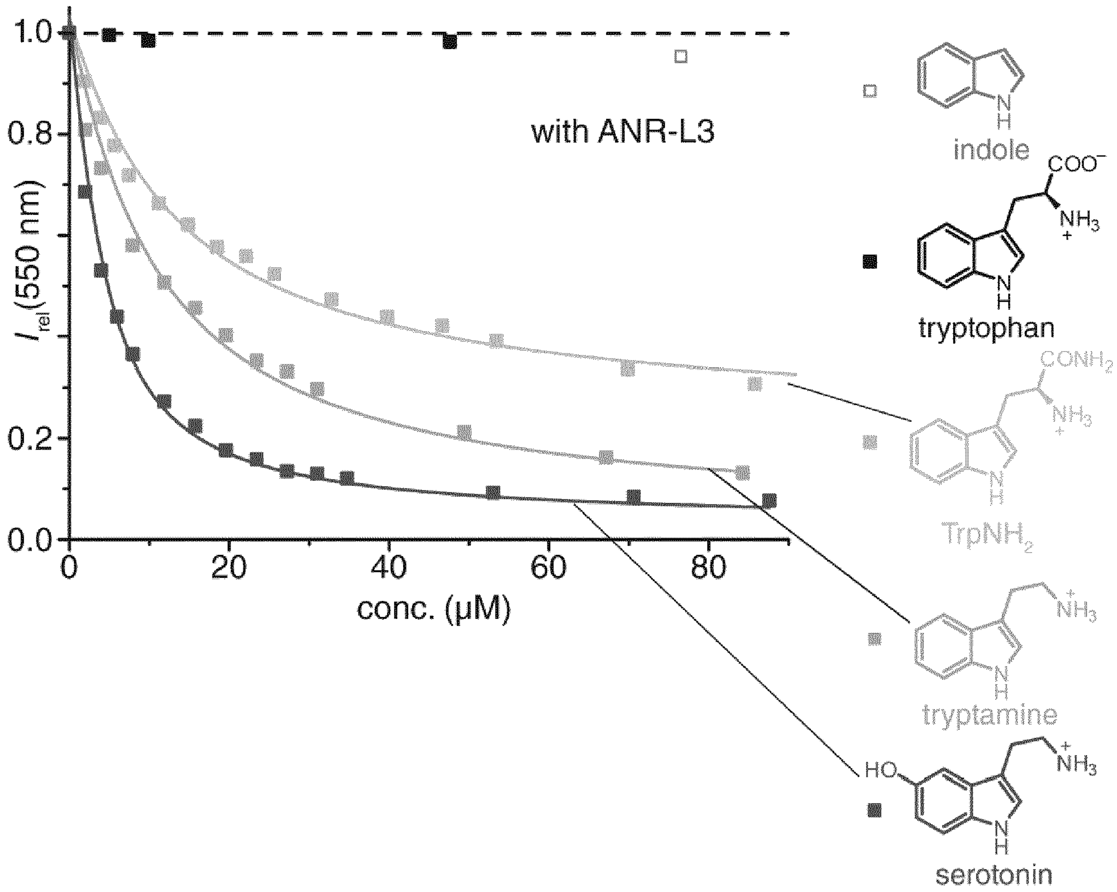


FIGURE 18

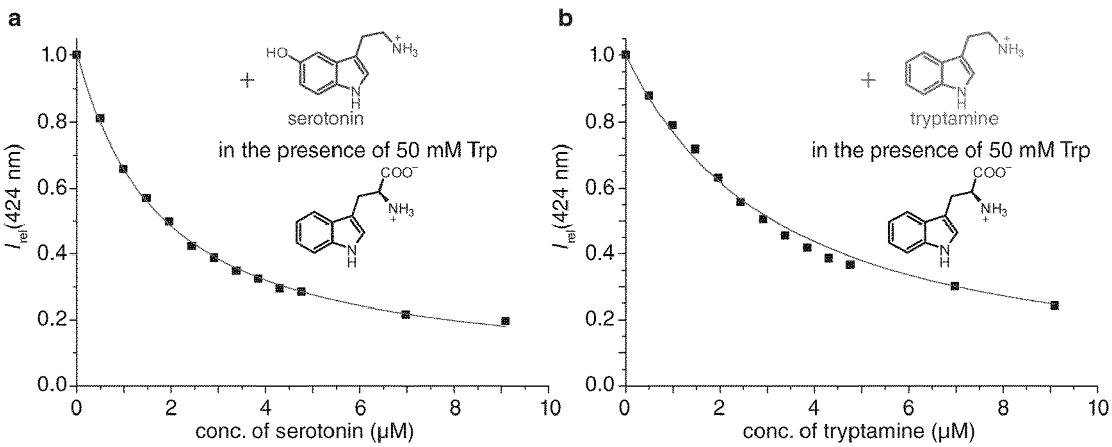


FIGURE 19

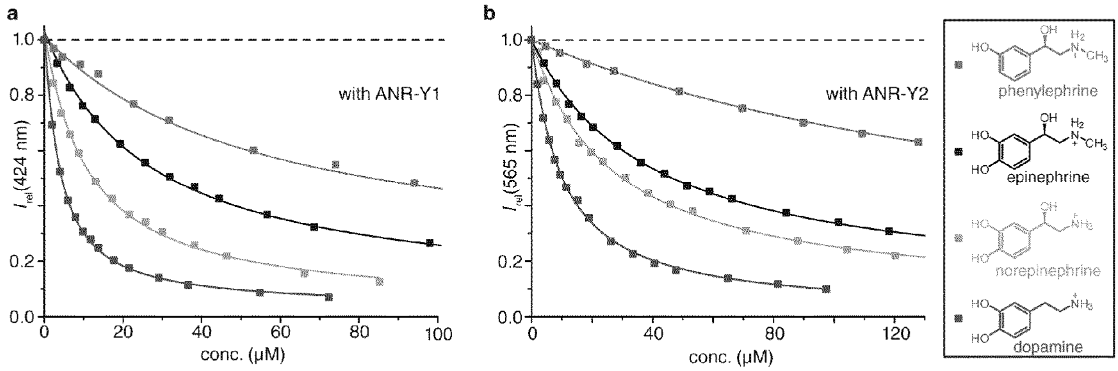


FIGURE 20

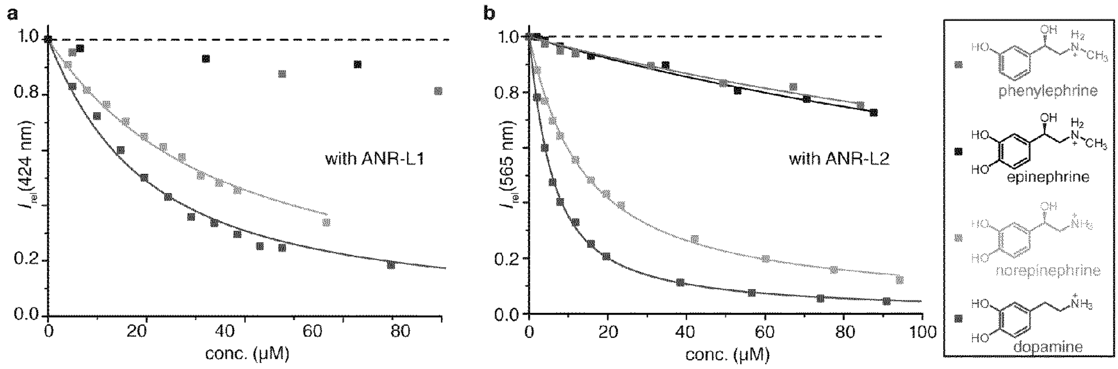


FIGURE 21

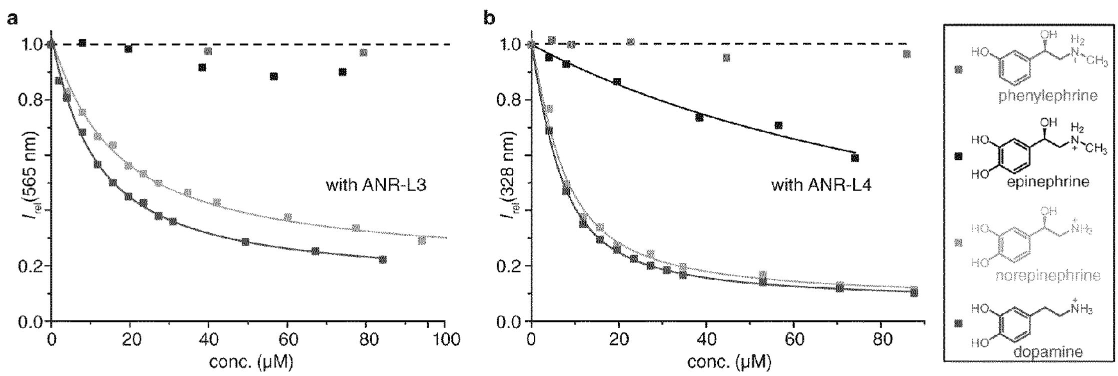


FIGURE 22

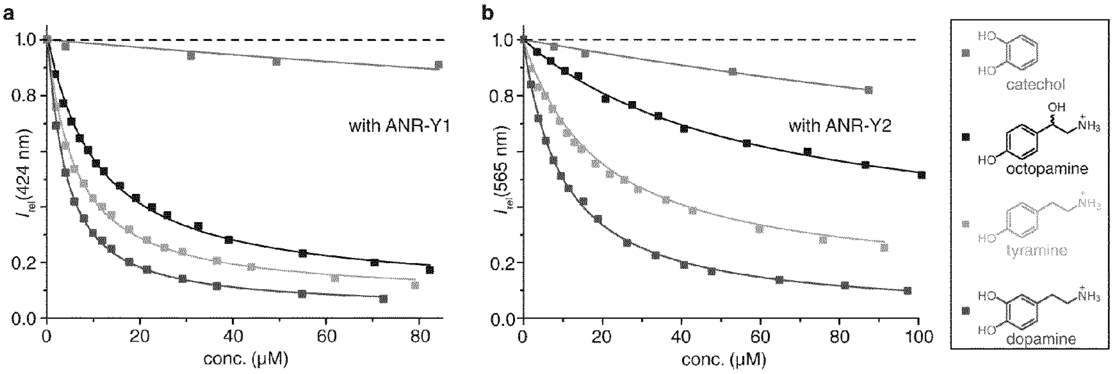


FIGURE 23

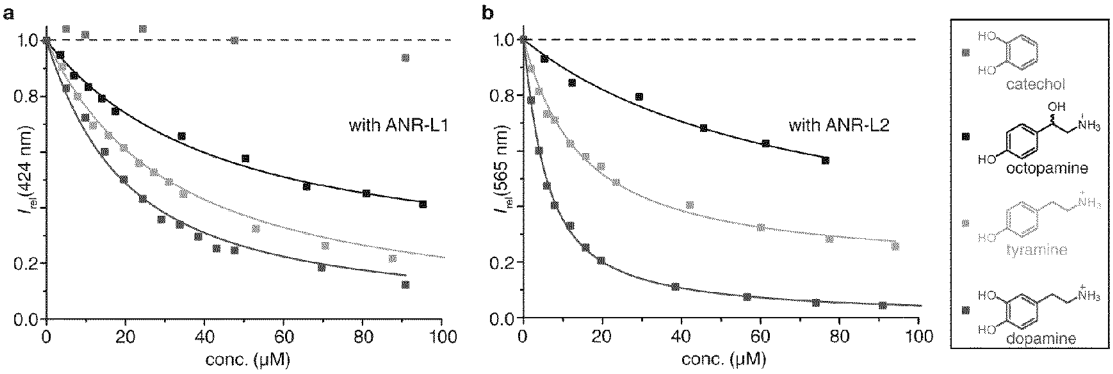


FIGURE 24

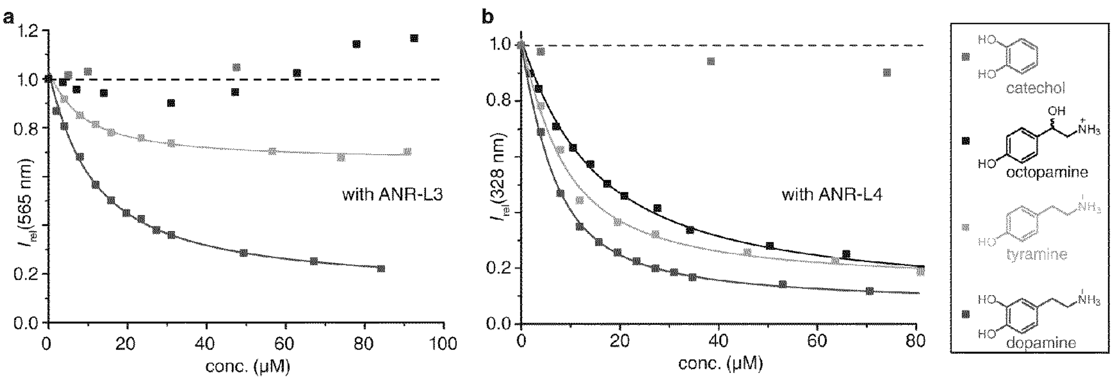


FIGURE 25

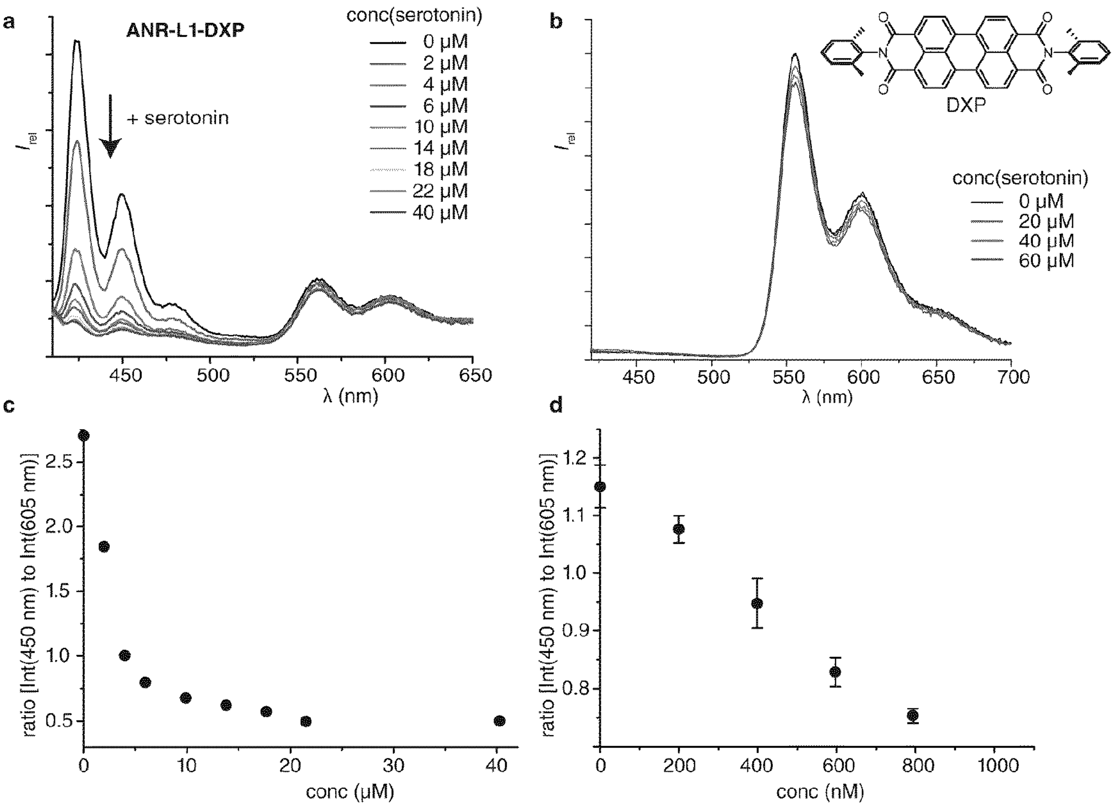


FIGURE 26

a

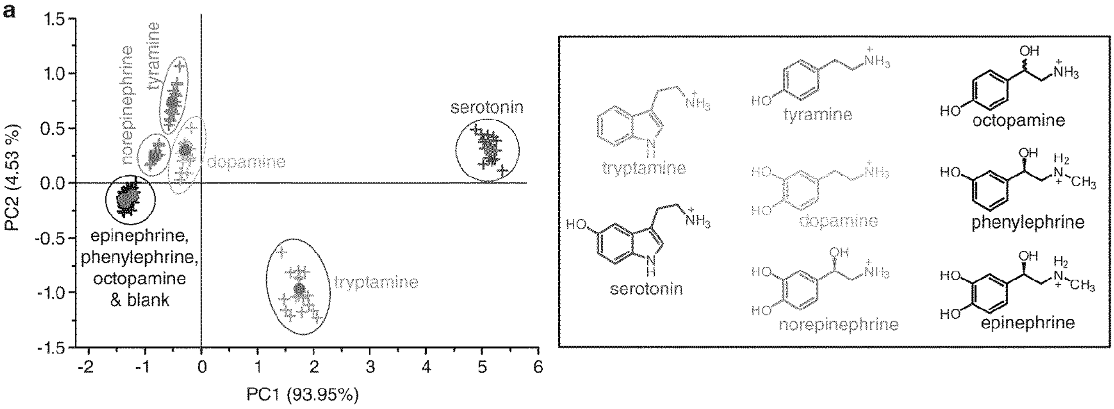


b

| analyte | $K_a$(ANR-L1) $10^3$ $M^{-1}$ | $K_a$(ANR-Y1) $10^3$ $M^{-1}$ | $K_a$(ANR-L2) $10^3$ $M^{-1}$ | $K_a$(ANR-Y2) $10^3$ $M^{-1}$ | $K_a$(ANR-L3) $10^3$ $M^{-1}$ |
|---|---|---|---|---|---|
| serotonin | 304 | 15,100 | 828 | 1,590 | 364 |
| tryptamine | 98 | 6,600 | 630 | 1,010 | 106 |
| dopamine | 63 | 292 | 223 | 105 | 103 |
| tyramine | 37 | 173 | 76 | 52 | 146 |
| norepinephrine | 33 | 95 | 80 | 38 | 69 |
| octopamine | 25 | 95 | 15 | 17 | 1 |
| epinephrine | 1 | 38 | 4 | 26 | 1 |
| phenylephrine | 1 | 18 | 9 | 5 | 1 |
| blank | 1 | 1 | 1 | 1 | 1 |

FIGURE 27

a

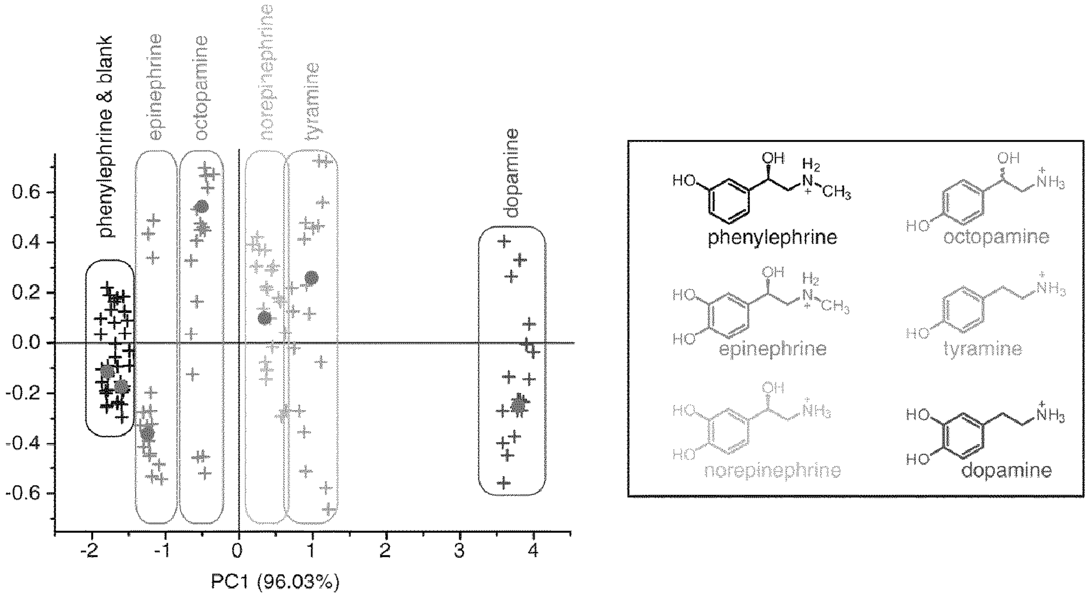


b

| analyte | $K_a$(ANR-L1) $10^3$ $M^{-1}$ | $K_a$(ANR-Y1) $10^3$ $M^{-1}$ | $K_a$(ANR-L2) $10^3$ $M^{-1}$ | $K_a$(ANR-Y2) $10^3$ $M^{-1}$ |
|---|---|---|---|---|
| dopamine | 63 | 292 | 223 | 105 |
| tyramine | 37 | 173 | 76 | 52 |
| norepinephrine | 33 | 95 | 80 | 38 |
| octopamine | 25 | 95 | 15 | 17 |
| epinephrine | 1 | 38 | 4 | 26 |
| phenylephrine | 1 | 18 | 9 | 5 |
| blank | 1 | 1 | 1 | 1 |

FIGURE 28

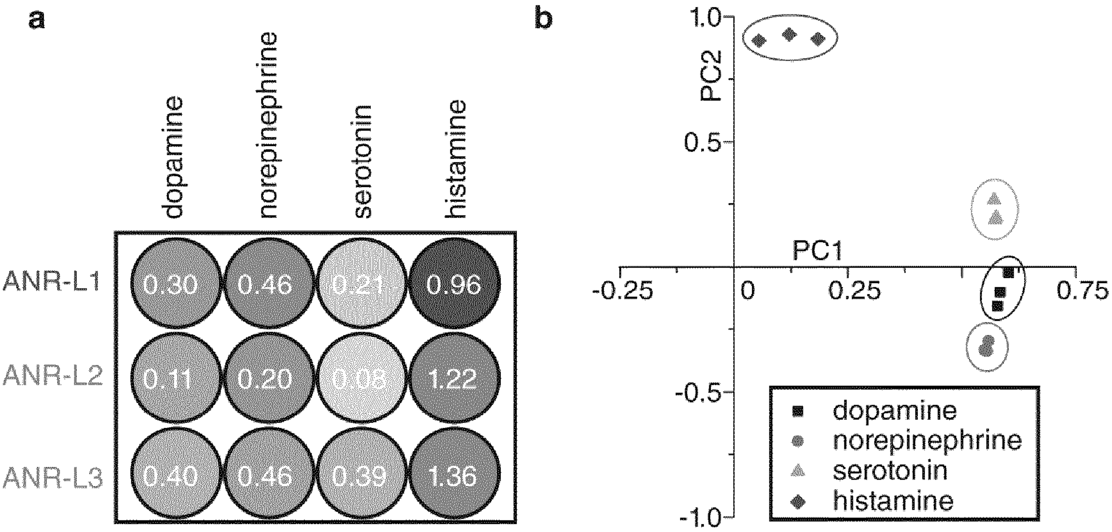


FIGURE 29

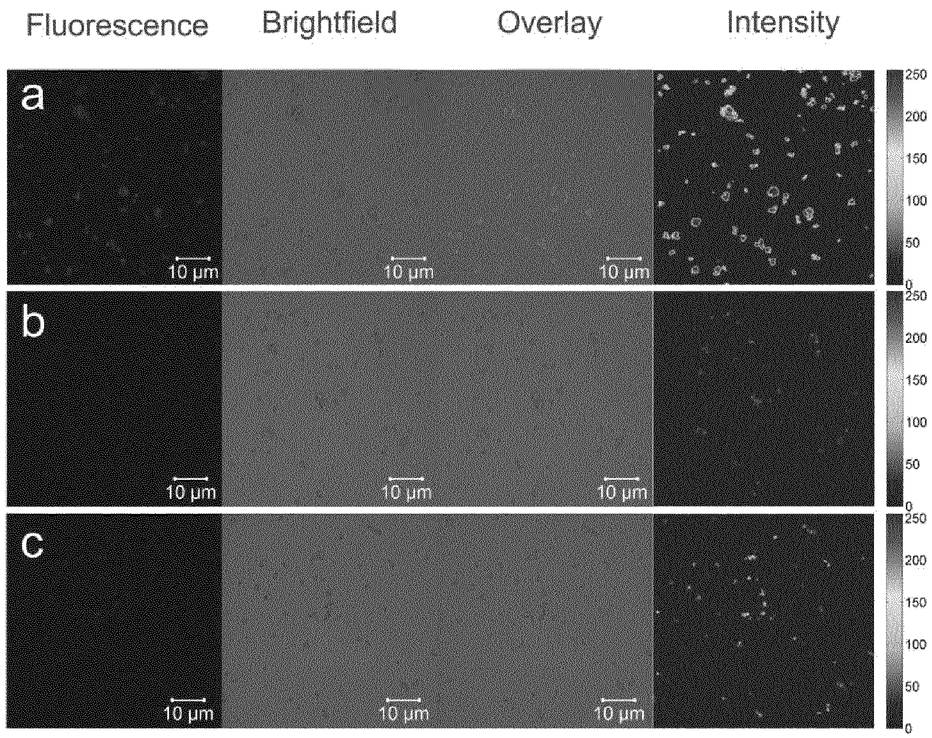


**FIGURE 30**

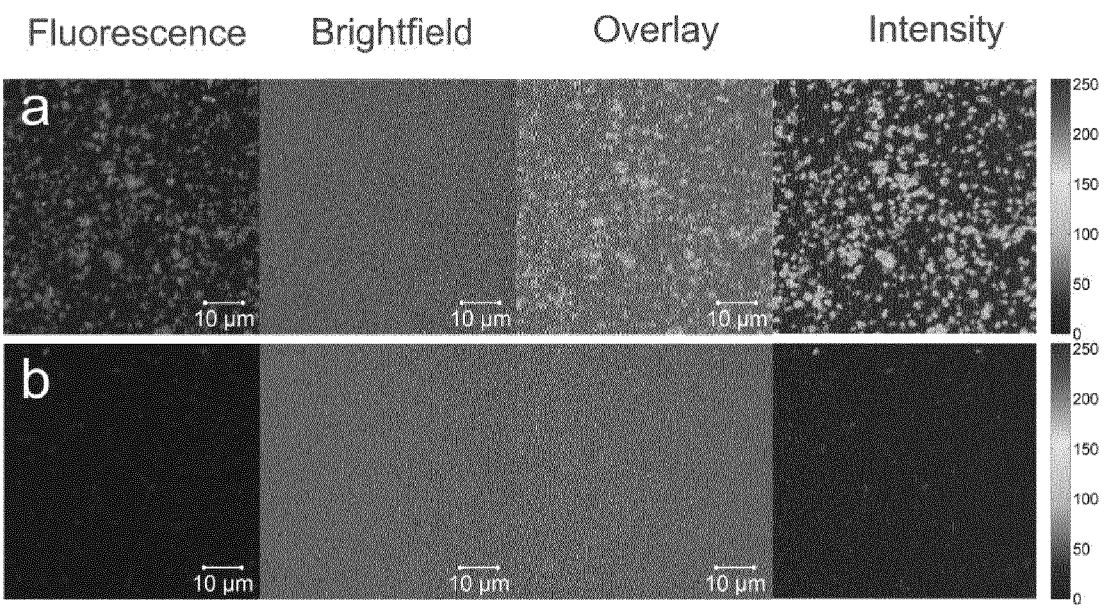


**FIGURE 31**

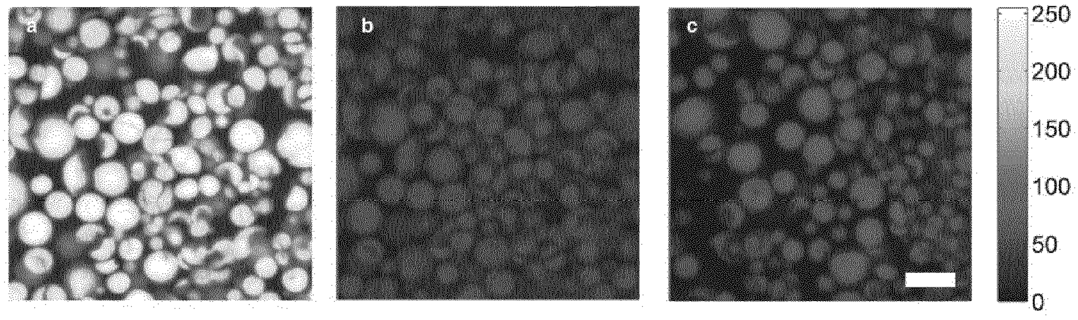


FIGURE 32

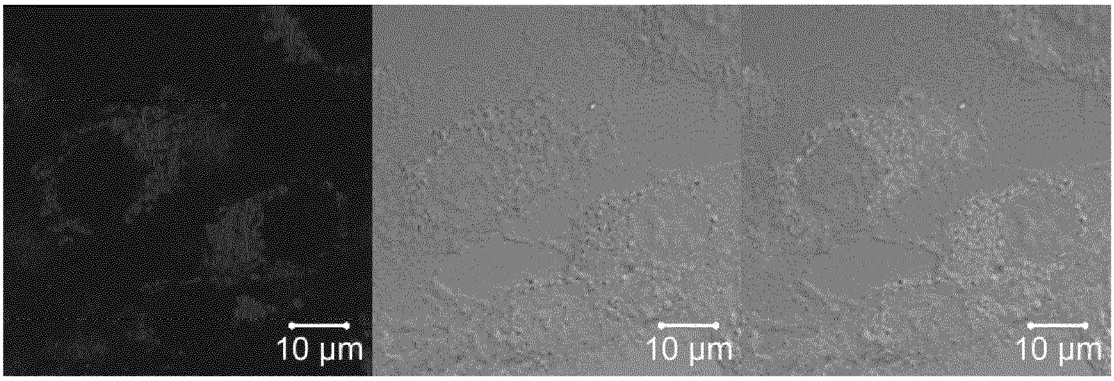


FIGURE 33

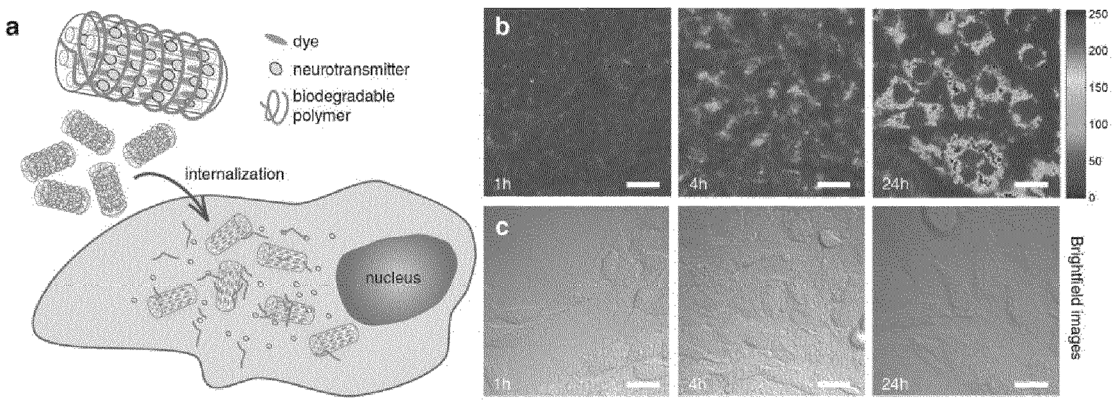


FIGURE 34

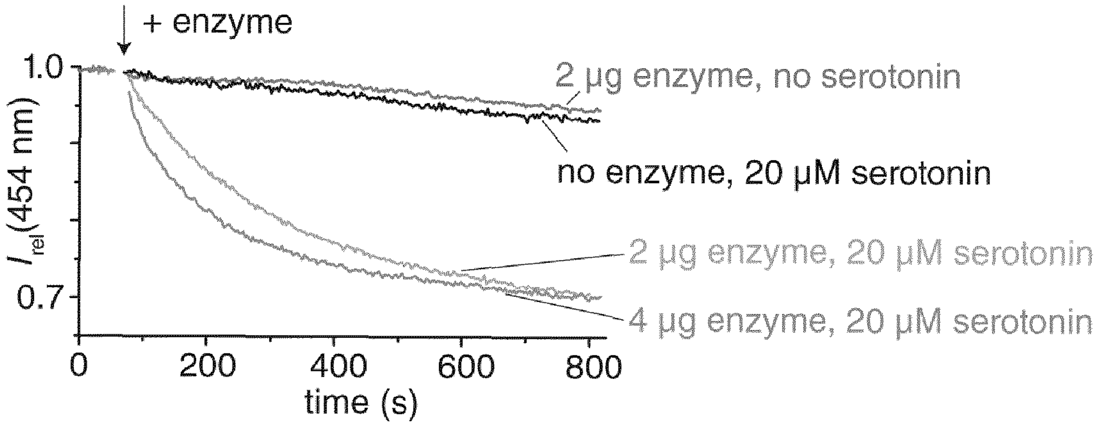


**FIGURE 35**

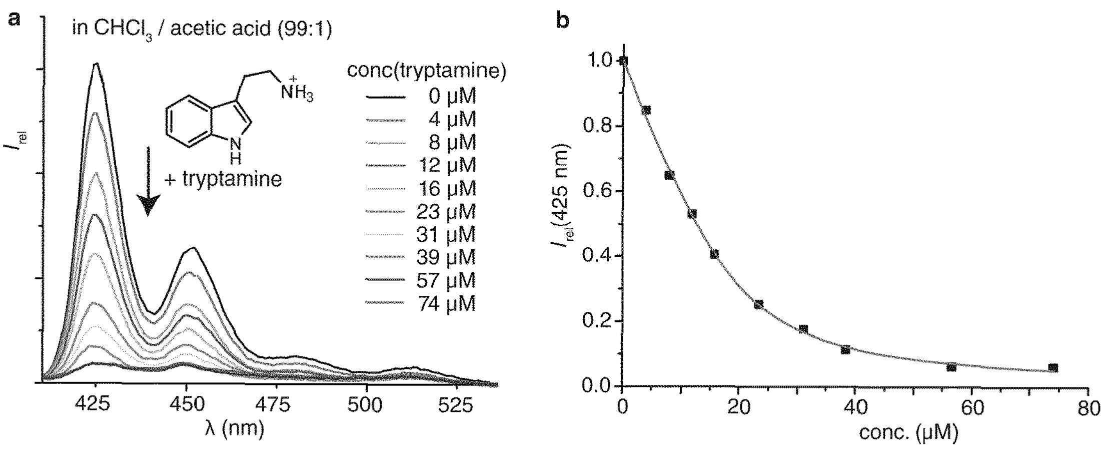


**FIGURE 36**

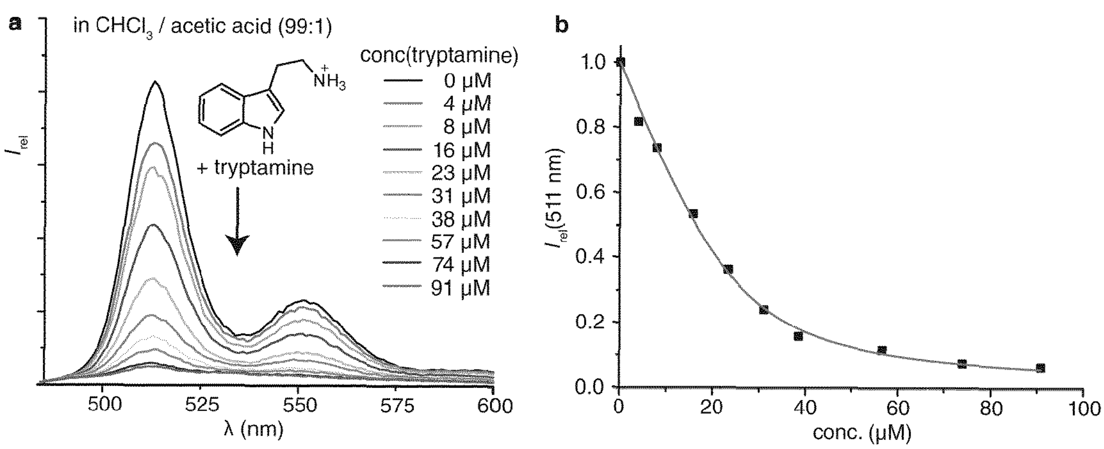


**FIGURE 37**

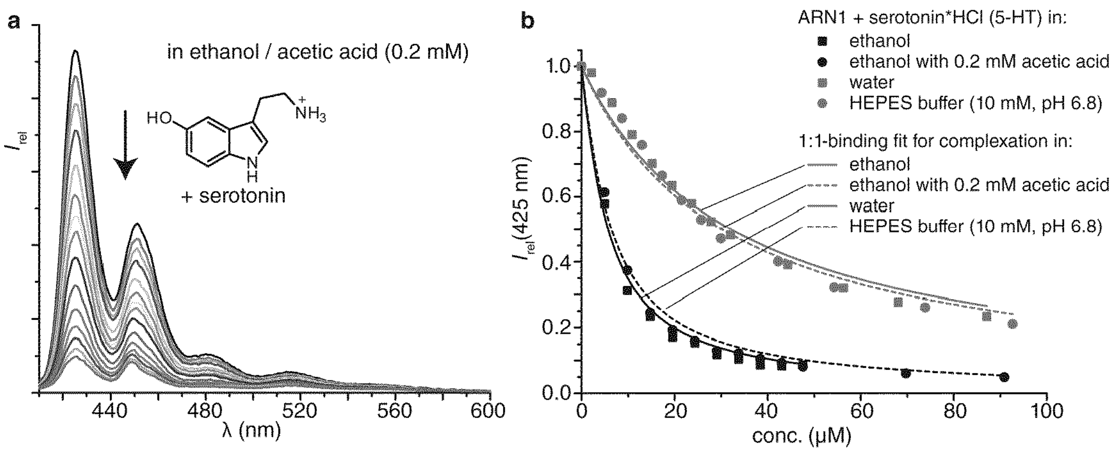


# FIGURE 38

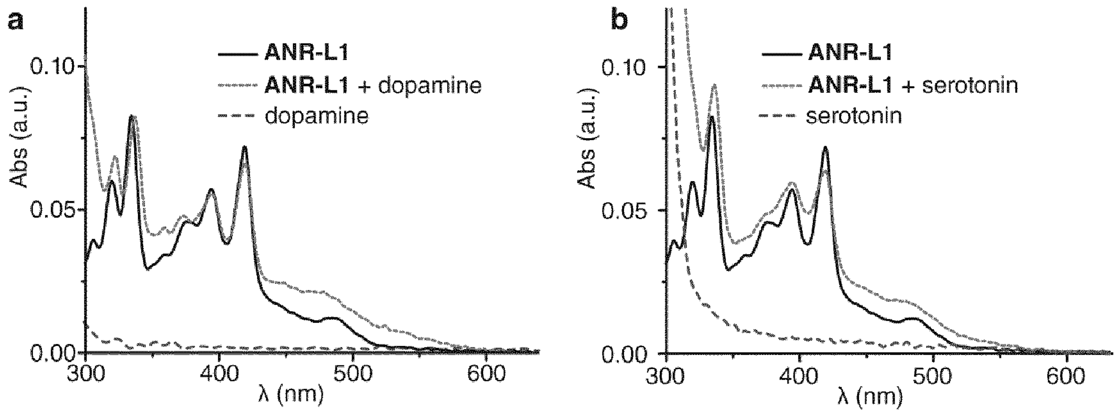


# FIGURE 39

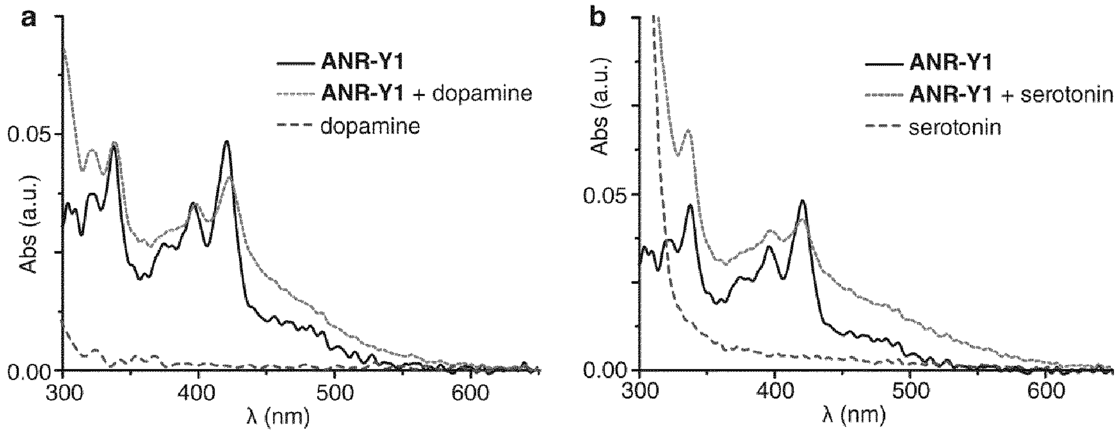


# FIGURE 40

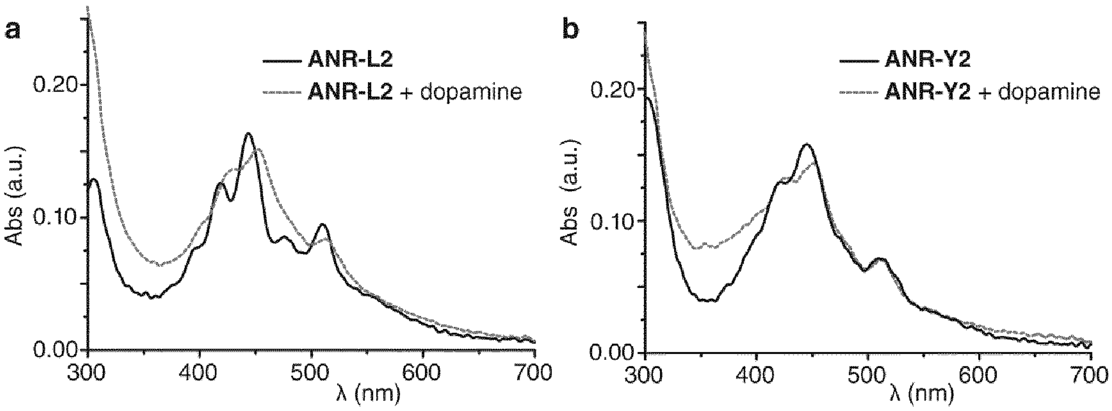


FIGURE 41

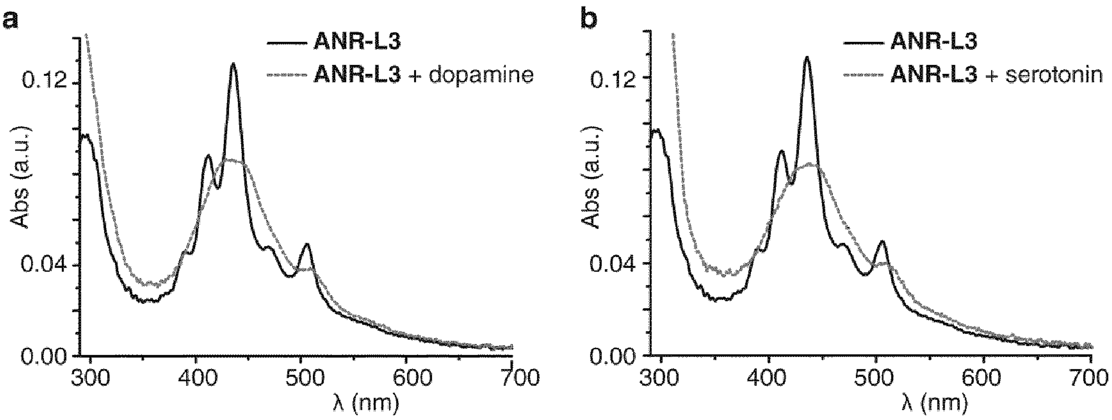


FIGURE 42

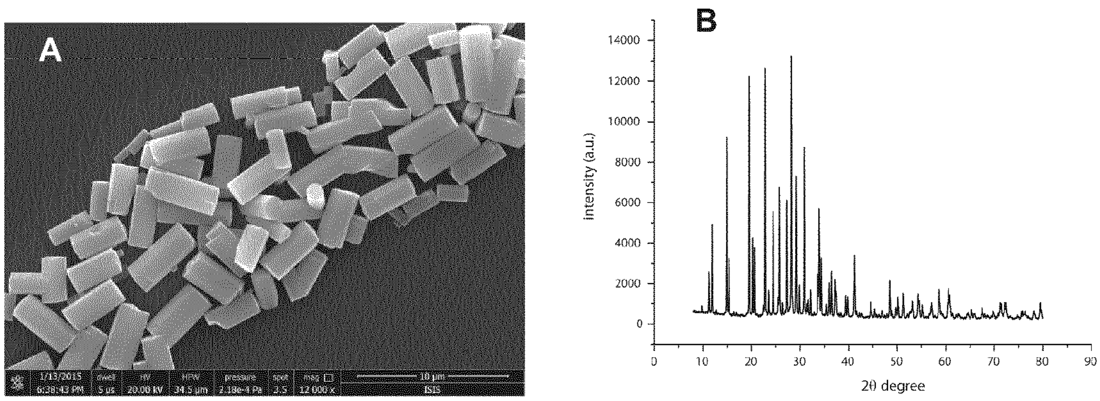


FIGURE 43

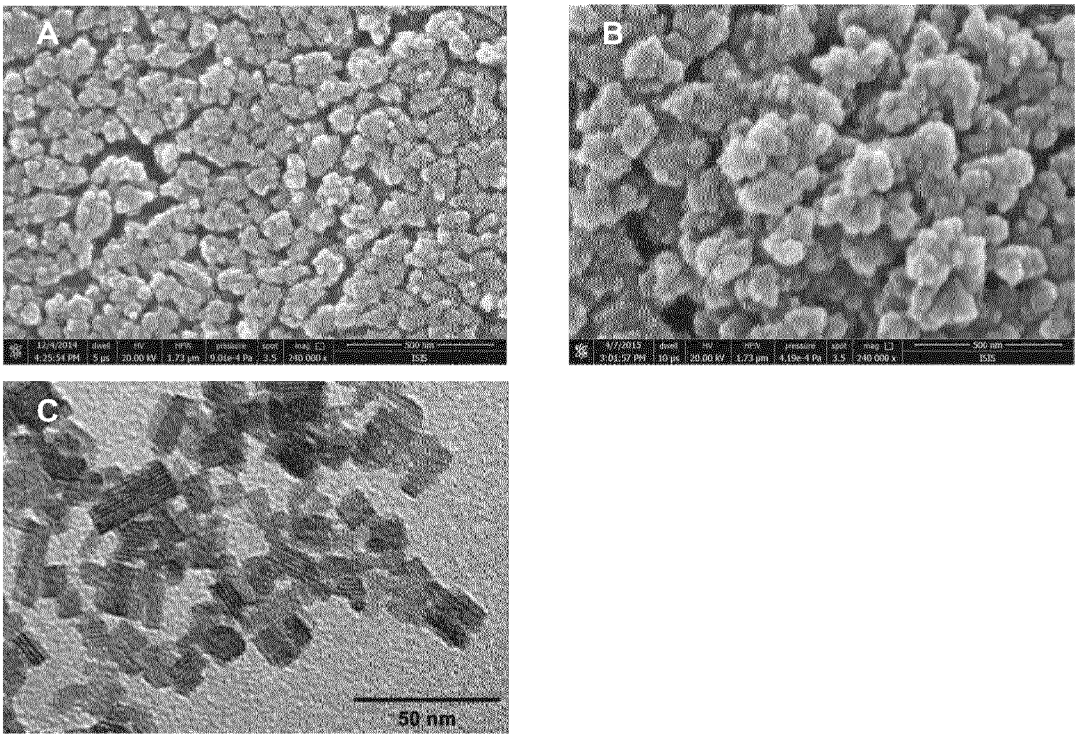


FIGURE 44

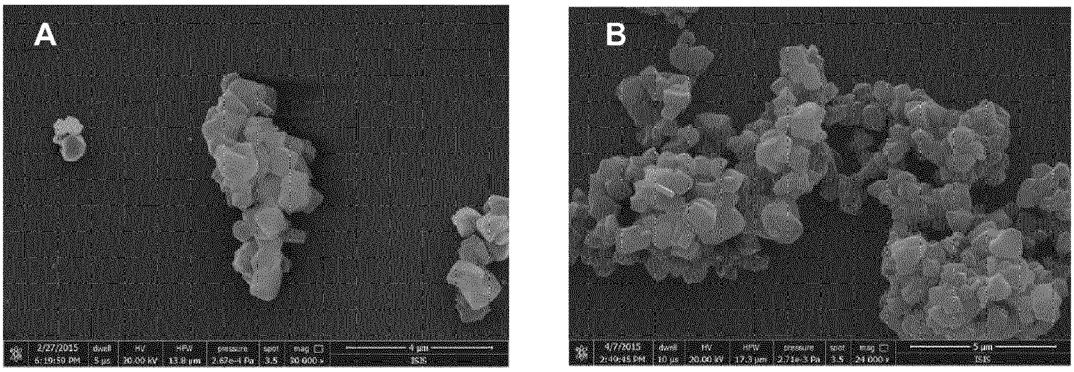


FIGURE 45

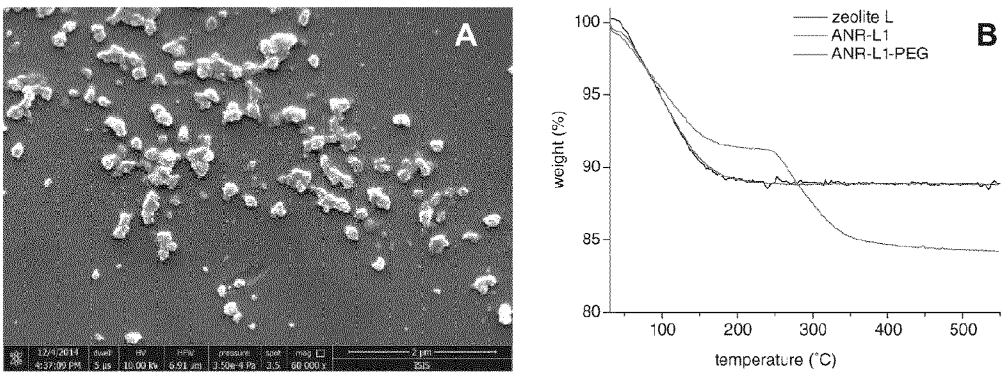


FIGURE 46

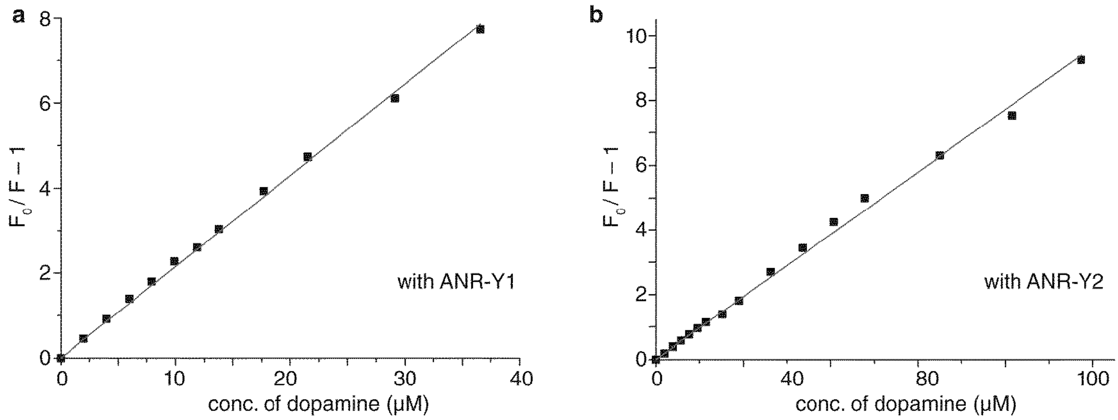


FIGURE 47

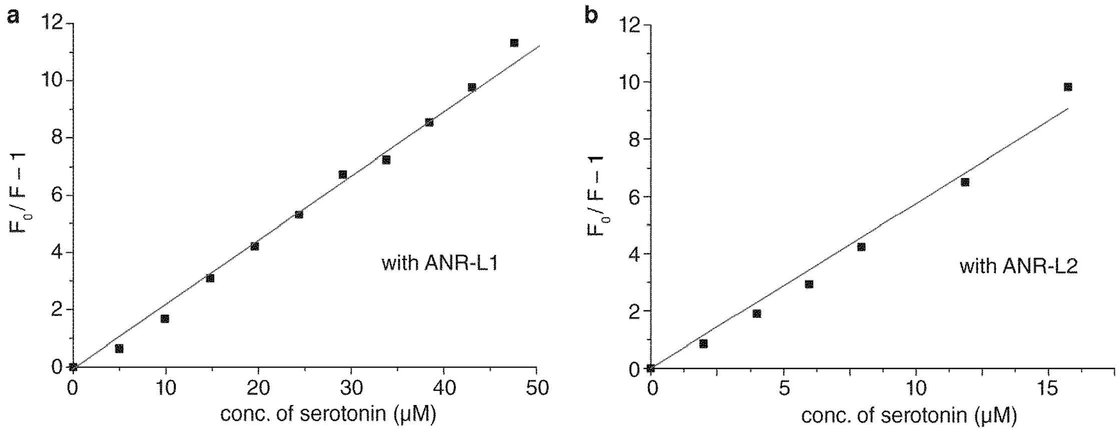


FIGURE 48

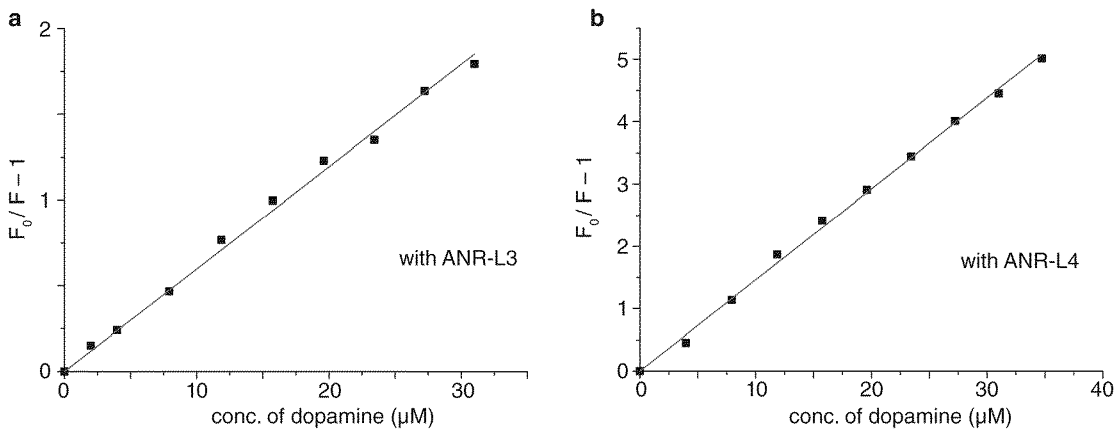


FIGURE 49

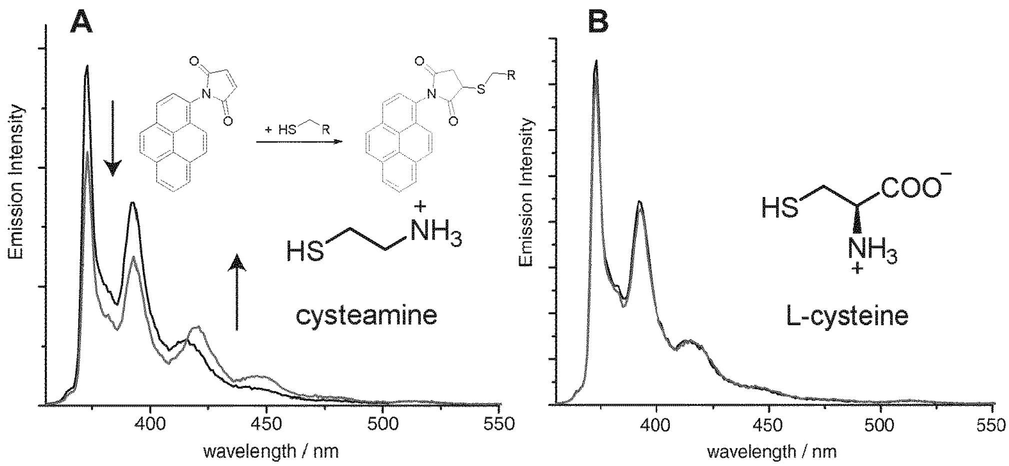


# FIGURE 50

| analyte | biological function | charge | aryl-unit | $K_d$(ANR-L2) μM | $K_d$(ANR-Y2) μM |
|---|---|---|---|---|---|
| serotonin (5-HT) | neurotransmitter | + | 5HO-indole | 1.2 | 0.63 |
| tryptamine | trace amine | + | indole | 1.6 | 0.99 |
| dopamine | neurotransmitter | + | catechol | 4.5 | 9.5 |
| $TrpNH_2$ | - | + | indole | 6.7 | 2.7 |
| norepinephrine | neurotransmitter | + | catechol | 13 | 26 |
| tyramine | trace amine | + | phenol | 13 | 19 |
| octopamine | trace amine | + | phenol | 68 | 58 |
| epinephrine | neurotransmitter | + | catechol | 240 | 39 |
| phenylephrine | drug (decongestant) | + | phenol | 240 | 190 |
| phenethylamine | trace amine | + | benzene | n.d.* | >100* |
| histamine | neurotransmitter | + | imidazole | n.d.* | 56* |
| tryptophan (Trp) | amino acid | + – | indole | >>1000 | >1000 |
| TrpGly | occurs in hypophysis | + – | indole | >>1000 | >1000 |
| 5-hydroxy tryptophan | precursor to 5HT | + – | 5HO-indole | >>1000 | >1000 |

# TABLE 3

| analyte | biological function | charge | aryl-unit | $K_d$(ANR-L3) µM |
|---|---|---|---|---|
| serotonin (5-HT) | neurotransmitter | + | 5HO-indole | 2.7 |
| tryptamine | trace amine | + | indole | 9.4 |
| tyramine | trace amine | + | phenol | 6.9* |
| dopamine | neurotransmitter | + | catechol | 9.7 |
| norepinephrine | neurotransmitter | + | catechol | 15 |
| phenethylamine | trace amine | + | benzene | 190* |
| histamine | neurotransmitter | + | imidazole | 360* |
| epinephrine | neurotransmitter, hormone | + | catechol | >300 |
| L-DOPA | drug (dopamine precursor) | + – | catechol | >>1000 |
| tryptophan (Trp) | amino acid | + – | indole | >>1000 |
| nicotine | stimulant drug, toxin | + | pyridine | >>1000 |
| indole | - | No | indole | >>1000 |
| catechol | - | No | catechol | >>1000 |

# TABLE 4

| analyte | biological function | charge | aryl-unit | $K_d$(ANR-L4) µM |
|---|---|---|---|---|
| dopamine | neurotransmitter | + | catechol | 3.5 |
| norepinephrine | neurotransmitter | + | catechol | 4.5 |
| tyramine | trace amine | + | phenol | 5.7 |
| octopamine | trace amine | + | phenol | 12 |
| phenethylamine | trace amine | + | benzene | 19 |
| epinephrine | neurotransmitter, hormone | + | catechol | 110 |
| phenylephrine | drug (decongestant) | + | phenol | >100 |
| histamine | neurotransmitter | + | imidazole | n.d.* |
| L-DOPA | drug (dopamine precursor) | + – | catechol | >>1000 |
| phenylalanine | amino acid | + – | benzene | >>1000 |
| tyrosine | amino acid | + – | phenol | >>1000 |
| histidine | amino acid | + – | imidazole | >>1000 |
| adenosine | neuromodulator | No | purine | >>1000 |
| catechol | - | No | catechol | >>1000 |

# TABLE 5

| system | $\lambda_{exc}$ (nm) | QY |
|---|---|---|
| **D1** | 336 | 0.43 |
| **ANR-L1** | 336 | 0.52 |
| **ANR-L1** + excess dopamine | 336 | < 0.01 |
| **D1** | 336 | 0.43 |
| **ANR-Y1** | 336 | 0.53 |
| **ANR-Y1** + excess dopamine | 336 | < 0.01 |
| **D2** | 420 | 0.37 |
| **ANR-L2** | 420 | 0.12 |
| **ANR-L2** + excess dopamine | 420 | < 0.01 |
| **D2** | 420 | 0.37 |
| **ANR-L2** | 420 | 0.14 |
| **ANR-L2** + excess dopamine | 420 | < 0.01 |
| **D3** | 440 | 0.04 |
| **ANR-L3** | 440 | < 0.01 |
| **ANR-L3** + excess dopamine | 440 | 0.51 |
| **D4** | 300 | 0.67 |
| **ANR-L4** | 300 | 0.45 |
| **ANR-L4** + excess dopamine | 300 | < 0.01 |

**TABLE 6**

| system | zeta potential (mV) | size by SEM (nm) |
|---|---|---|
| **zeolite Y** | –27 | 900 |
| **ANR-Y1** | –31 | 900 |
| **ANR-Y2** | –28 | 900 |
| **zeolite L** | –32 | 50 |
| **ANR-L1** | –34 | 50 |
| **ANR-L2** | –28 | 50 |
| **ANR-L3** | –31 | 50 |
| **ANR-L4** | –29 | 50 |
| **ANR-L1-PEG** | –15 | 50 |
| **ANR-L1 + polylysine coating** | + 25 | - |

**TABLE 7**

Europäisches Patentamt
European Patent Office
Office européen des brevets

# EUROPEAN SEARCH REPORT

Application Number
EP 16 30 5378

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DONGPENG YAN ET AL: "Tunable Two-color Luminescence and Host-guest Energy Transfer of Fluorescent Chromophores Encapsulated in Metal-Organic Frameworks", SCIENTIFIC REPORTS, vol. 4, 11 March 2014 (2014-03-11), XP055299689, DOI: 10.1038/srep04337 * the whole document * * abstract * * figures 1-8 * ----- | 1-41 | INV. C01B39/02 G01N21/63 G01N33/531 G01N33/58 |
| Y | XU-SHENG WANG ET AL: "An Anion Metal-Organic Framework with Lewis Basic Sites-Rich toward Charge-Exclusive Cationic Dyes Separation and Size-Selective Catalytic Reaction", INORGANIC CHEMISTRY, vol. 55, no. 5, 7 March 2016 (2016-03-07), pages 2641-2649, XP055300257, EASTON, US ISSN: 0020-1669, DOI: 10.1021/acs.inorgchem.6b00019 * the whole document * ----- | 1-41 | TECHNICAL FIELDS SEARCHED (IPC) C01B G01N |
| Y | JIHYUN AN ET AL: "Cation-Triggered Drug Release from a Porous Zinc-Adeninate Metal-Organic Framework", JOURNAL OF THE AMERICAN CHEMICAL SOCIETY, vol. 131, no. 24, 24 June 2009 (2009-06-24), pages 8376-8377, XP055028138, ISSN: 0002-7863, DOI: 10.1021/ja902972w * the whole document * ----- -/-- | 1-41 | |

The present search report has been drawn up for all claims

1

| Place of search | Date of completion of the search | Examiner |
|---|---|---|
| The Hague | 6 September 2016 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 5

Europäisches Patentamt
European Patent Office
Office européen des brevets

# EUROPEAN SEARCH REPORT

Application Number
EP 16 30 5378

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MING-JIE DONG ET AL: "A Luminescent Dye@MOF Platform: Emission Fingerprint Relationships of Volatile Organic Molecules", ANGEWANDTE CHEMIE INTERNATIONAL EDITION, vol. 53, no. 6, 2 January 2014 (2014-01-02), pages 1575-1579, XP055299688, DE ISSN: 1433-7851, DOI: 10.1002/anie.201307331 * the whole document * * figures 1-4 * ----- | 1-41 | |
| Y | ELIDA ILINOIU ET AL: "Simultaneous/Selective Detection of Dopamine and Ascorbic Acid at Synthetic Zeolite-Modified/Graphite-Epoxy Composite Macro/Quasi-Microelectrodes", SENSORS, vol. 13, no. 6, 3 June 2013 (2013-06-03), pages 7296-7307, XP055299406, DOI: 10.3390/s130607296 * the whole document * ----- -/-- | 1-41 | TECHNICAL FIELDS SEARCHED (IPC) |

1

The present search report has been drawn up for all claims

| Place of search | Date of completion of the search | Examiner |
|---|---|---|
| The Hague | 6 September 2016 | Boiangiu, Clara |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches Patentamt
European Patent Office
Office européen des brevets

# EUROPEAN SEARCH REPORT

Application Number
EP 16 30 5378

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MEISSAM NOROOZIFAR ET AL: "Simultaneous and sensitive determination of a quaternary mixture of AA, DA, UA and Trp using a modified GCE by iron ion-doped natrolite zeolite-multiwall carbon nanotube", BIOSENSORS AND BIOELECTRONICS, ELSEVIER BV, NL, vol. 28, no. 1, 28 June 2011 (2011-06-28), pages 56-63, XP028340752, ISSN: 0956-5663, DOI: 10.1016/J.BIOS.2011.06.042 [retrieved on 2011-07-06] * the whole document * ----- | 1-40 | |
| Y | PAUCHARD M ET AL: "DYE-LOADED ZEOLITE L SANDWICHES AS ARTIFICIAL ANTENNA SYSTEMS FOR LIGHT TRANSPORT", CHEMISTRY - A EUROPEAN JOURNAL, WILEY - V C H VERLAG GMBH & CO. KGAA, WEINHEIM, DE, vol. 6, no. 18, 1 January 2000 (2000-01-01), pages 3456-3470, XP001065414, ISSN: 0947-6539, DOI: 10.1002/1521-3765(20000915)6:18<3456::AID-CHEM3456>3.3.CO;2-X * the whole document * ----- | 1-41 | TECHNICAL FIELDS SEARCHED (IPC) |
| Y | US 2015/150981 A1 (GREF RUXANDRA [FR] ET AL) 4 June 2015 (2015-06-04) * the whole document * * paragraphs [0020], [0177] - [0181], [0188] - [193229], [0314] - [0328]; figures 1-28; examples 2, 3 * ----- -/-- | 1-41 | |

The present search report has been drawn up for all claims

1

| Place of search | Date of completion of the search | Examiner |
|---|---|---|
| The Hague | 6 September 2016 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 5

Europäisches Patentamt
European Patent Office
Office européen des brevets

# EUROPEAN SEARCH REPORT

Application Number
EP 16 30 5378

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FR 2 921 660 A1 (CENTRE NAT RECH SCIENT [FR]; UNIV VERSAILLES SAINT QUENTIN [FR]) 3 April 2009 (2009-04-03)<br>* the whole document *<br>* paragraphs [0042] - [0064], [0078] - [0102], [0159] - [0181], [0121] - [0123]; figure 10 *<br>* figures 1-28 *<br>----- | 1-41 | |
| Y | US 2012/202006 A1 (RIMER JEFFREY D [US]) 9 August 2012 (2012-08-09)<br>* the whole document *<br>* paragraphs [0006] - [0014], [0019], [0088], [0115], [0121], [0122], [0128], [0132], [0187] - [0191] *<br>----- | 1-41 | |
| Y | WO 2010/078337 A1 (UNIV CALIFORNIA [US]; BASF SE [DE]; YAGHI OMAR M [US]; CZAJA ALEXANDER) 8 July 2010 (2010-07-08)<br>* the whole document *<br>----- | 1-41 | TECHNICAL FIELDS SEARCHED (IPC) |
| Y | EP 2 141 122 A1 (UNIV MUENSTER WILHELMS [DE]; UNIV TWENTE [NL]) 6 January 2010 (2010-01-06)<br>* the whole document *<br>* paragraphs [0034], [0035], [0046] - [0059], [0061] - [0068]; claims 1-14 *<br>-----<br>-/-- | 1-41 | |

The present search report has been drawn up for all claims

1

| Place of search | Date of completion of the search | Examiner |
|---|---|---|
| The Hague | 6 September 2016 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 5

Europäisches Patentamt
European Patent Office
Office européen des brevets

# EUROPEAN SEARCH REPORT

Application Number
EP 16 30 5378

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MA ET AL: "Fluorene-based Cu(II)-MOF: a visual colorimetric anion sensor and separator based on an anion-exchange approach", CHEMICAL COMMUNICATIONS - CHEMCOM, , vol. 48, no. 24 21 March 2012 (2012-03-21), pages 2946-2948, XP008181506, ISSN: 1359-7345, DOI: 10.1039/C2CC16800F Retrieved from the Internet: URL:http://pubs.rsc.org/en/content/articlepdf/2012/CC/C2CC16800F [retrieved on 2011-12-06] * the whole document * ----- | 1-40 | |
| Y,D | WO 02/36490 A1 (UNIV BERN [CH]; CALZAFERRI GION [CH]) 10 May 2002 (2002-05-10) * the whole document * ----- | 1-41 | |
| Y,D | WO 2008/052603 A1 (ROCHE DIAGNOSTICS GMBH [DE]; HOFFMANN LA ROCHE [CH]; UNIV BERN [CH]; P) 8 May 2008 (2008-05-08) * the whole document * ----- | 1-41 | TECHNICAL FIELDS SEARCHED (IPC) |

The present search report has been drawn up for all claims

1

| Place of search | Date of completion of the search | Examiner |
|---|---|---|
| The Hague | 6 September 2016 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 16 30 5378

This annex lists the patent family members relating to the patent documents cited in the above-mentioned European search report. The members are as contained in the European Patent Office EDP file on
The European Patent Office is in no way liable for these particulars which are merely given for the purpose of information.

06-09-2016

| Patent document cited in search report | Publication date | Patent family member(s) | Publication date |
|---|---|---|---|
| US 2015150981 A1 | 04-06-2015 | CA 2873379 A1 | 05-12-2013 |
| | | CN 104718214 A | 17-06-2015 |
| | | EP 2855490 A1 | 08-04-2015 |
| | | FR 2991325 A1 | 06-12-2013 |
| | | JP 2015521197 A | 27-07-2015 |
| | | KR 20150031243 A | 23-03-2015 |
| | | US 2015150981 A1 | 04-06-2015 |
| | | WO 2013178954 A1 | 05-12-2013 |
| FR 2921660 A1 | 03-04-2009 | CA 2699480 A1 | 25-06-2009 |
| | | EP 2193137 A1 | 09-06-2010 |
| | | ES 2430044 T3 | 18-11-2013 |
| | | FR 2921660 A1 | 03-04-2009 |
| | | JP 5547078 B2 | 09-07-2014 |
| | | JP 2010540600 A | 24-12-2010 |
| | | US 2010209354 A1 | 19-08-2010 |
| | | WO 2009077670 A1 | 25-06-2009 |
| US 2012202006 A1 | 09-08-2012 | US 2012202006 A1 | 09-08-2012 |
| | | WO 2012106675 A2 | 09-08-2012 |
| WO 2010078337 A1 | 08-07-2010 | EP 2356072 A1 | 17-08-2011 |
| | | US 2012028846 A1 | 02-02-2012 |
| | | US 2013295680 A1 | 07-11-2013 |
| | | WO 2010078337 A1 | 08-07-2010 |
| EP 2141122 A1 | 06-01-2010 | EP 2141122 A1 | 06-01-2010 |
| | | GB 2461686 A | 13-01-2010 |
| | | US 2010003188 A1 | 07-01-2010 |
| WO 0236490 A1 | 10-05-2002 | AT 350337 T | 15-01-2007 |
| | | AU 1202302 A | 15-05-2002 |
| | | DE 60125814 T2 | 26-04-2007 |
| | | DK 1335879 T3 | 05-03-2007 |
| | | EP 1335879 A1 | 20-08-2003 |
| | | ES 2278792 T3 | 16-08-2007 |
| | | US 2004026662 A1 | 12-02-2004 |
| | | US 2006001004 A1 | 05-01-2006 |
| | | US 2008272338 A1 | 06-11-2008 |
| | | US 2011253939 A1 | 20-10-2011 |
| | | WO 0236490 A1 | 10-05-2002 |
| WO 2008052603 A1 | 08-05-2008 | AT 485240 T | 15-11-2010 |
| | | CA 2592754 A1 | 02-05-2008 |
| | | CN 101573294 A | 04-11-2009 |
| | | EP 2089320 A1 | 19-08-2009 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 16 30 5378

This annex lists the patent family members relating to the patent documents cited in the above-mentioned European search report. The members are as contained in the European Patent Office EDP file on
The European Patent Office is in no way liable for these particulars which are merely given for the purpose of information.

06-09-2016

| Patent document cited in search report | Publication date | Patent family member(s) | Publication date |
|---|---|---|---|
| | | ES 2353920 T3 | 08-03-2011 |
| | | HK 1128455 A1 | 11-03-2011 |
| | | JP 5059117 B2 | 24-10-2012 |
| | | JP 2010506176 A | 25-02-2010 |
| | | US 2008138914 A1 | 12-06-2008 |
| | | US 2011287408 A1 | 24-11-2011 |
| | | WO 2008052603 A1 | 08-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1335879 A
- EP 2089320 A


### Non-patent literature cited in the description


- **BIEDERMANN et al.** *Angew. Chem. Int. Ed.,* 2014, 5694-5699
- **CALZAFERRI et al.** *Angew Chem Int Ed,* 2003, vol. 42, 3732
- **RUIZ et al.** *Monatshefte Fur Chemie,* 2005, vol. 136, 77
- **MEGELSKI ; CALZAFERRI.** *Adv Funct Mater,* 2001, vol. 11, 277
- **S. SARAVANAMURUGAN et al.** *Microporous Mesoporous Mater.,* 2008, vol. 112, 97
- **Y. SATOH et al.** *Industrial & Engineering Chemistry Research,* 2013, vol. 52, 15293-15297
- **Z. CHEN et al.** *Nanoscale Research Letters,* 2008, vol. 4, 204-209
- **J. TIAN et al.** *J. Am. Chem. Soc.,* 2012, vol. 134, 9581-9584
- **A. KARMAKAR et al.** *Coord. Chem. Rev.,* 2016, vol. 307, 313-341
- **CALZAFERRI et al.** *Chem. Eur. J.,* 2000, vol. 6, 3456
- **A. ZABALA RUIZ ; D. BRUHWILER ; T. BAN ; G. CALZAFERRI.** *Monatshefte fur Chemie,* 2005, vol. 136, 77
- **S. MEGELSKI ; G. CALZAFERRI.** *Adv. Funct. Mater.,* 2001, vol. 11, 277
- Immunoassay. Academic Press, 1996
- **SCHMIDT, A.C. ; WANG, X. ; ZHU, Y. ; SOMBERS, L.A.** Carbon Nanotube Yarn Electrodes for Enhanced Detection of Neurotransmitter Dynamics in Live Brain Tissue. *ACS Nano,* 2013, vol. 7, 7864-7873
- **BOERSMA, A.J. ; BRAIN, K.L. ; BAYLEY, H.** Real-Time Stochastic Detection of Multiple Neurotransmitters with a Protein Nanopore. *ACS Nano,* 2012, vol. 6, 5304-5308
- **KRUSS, S. et al.** Neurotransmitter Detection Using Corona Phase Molecular Recognition on Fluorescent Single-Walled Carbon Nanotube Sensors. *Journal of the American Chemical Society,* 2013, vol. 136, 713-724
- **KASERA, S. ; HERRMANN, L.O. ; BARRIO, J.D. ; BAUMBERG, J.J. ; SCHERMAN, O.A.** Quantitative multiplexing with nano-self-assemblies in SERS. *Sci. Rep.,* 2014, vol. 4
- **BASURAY, A. N. et al.** The Chameleonic Nature of Diazaperopyrenium Recognition Processes. *Angew. Chem. Int. Ed.,* 2012, vol. 51, 11872-11877
- **HARTLIEB, K. J. et al.** Anticancer Activity Expressed by a Library of 2,9-Diazaperopyrenium Dications. *ACS Nano,* 2015, vol. 9, 1461-1470
- **BIEDERMANN, F. et al.** Benzobis(imidazolium)-cucurbit[8]uril complexes for binding and sensing aromatic compounds in aqueous solution. *Chem. Eur. J.,* 2010, vol. 16, 13716-13722
- **CALZAFERRI, G. ; HUBER, S. ; MAAS, H. ; MINKOWSKI, C.** Host-Guest Antenna Materials. *Angew. Chem. Int. Ed.,* 2003, vol. 42, 3732-3758
- **NYRÖNEN, T. et al.** Molecular Mechanism for Agonist-Promoted α2A-Adrenoceptor Activation by Norepinephrine and Epinephrine. *Mol. Pharmacol,* 2001, vol. 59, 1343-1354
- **LANAU, F. ; ZENNER, M.-T. ; CIVELLI, O. ; HARTMAN, D.S.** Epinephrine and Norepinephrine Act as Potent Agonists at the Recombinant Human Dopamine D4 Receptor. *J. Neurochem.,* 1997, vol. 68, 804-812
- **STEWART, S. ; IVY, M.A. ; ANSLYN, E.V.** The use of principal component analysis and discriminant analysis in differential sensing routines. *Chemical Society Reviews,* 2013
- **GARCIA, K.P. et al.** Zwitterionic-Coated "Stealth" Nanoparticles for Biomedical Applications: Recent Advances in Countering Biomolecular Corona Formation and Uptake by the Mononuclear Phagocyte System. *Small,* 2014, vol. 10, 2516-2529
- **BRECK.** Zeolite Molecular Sieves. Wiley, 1974, vol. 752
- **BAERLOCHER et al.** Atlas of Zeolite Framework Types. Elsevier, 2001, vol. 19
- **J. PYUN ; S. JIA ; T. KOWALEWSKI ; G.D. PATTERSON ; K. MATYJASZEWSKI.** *Macromolecules,* 2003, vol. 36, 5094-5104
- **C. SANCHEZ ; B. JULIAN ; P. BELLEVILLE ; M.POPALL.** *J. mater. Chem.,* 2005, vol. 15, 3559-3592
- **S.R. HALL ; S.S. DAVIS ; S. MANN.** *Langmuir,* 2000, vol. 16, 1454-1456

- **INAGAKI, S. ; GUAN, S. ; OHSUNA, T. ; TERASAKI, O.** *Nature,* 2002, vol. 416, 304-307
- **ASEFA, T. ; MACLACHLAN, M. J. ; COOMBS, N. ; OZIN, G. A.** *Nature,* 1999, vol. 402, 867-871
- **BIEDERMANN, F. ; NAU, W. M.** Noncovalent Chirality Sensing Ensembles for the Detection and Reaction Monitoring of Amino Acids, Peptides, Proteins, and Aromatic Drugs. *Angew. Chem. Int. Ed.,* 2014, 5694-5699
- **S. SARAVANAMURUGAN ; SUJANDI, E. ; A. PRASETYANTO ; S.-E. PARK.** Liquid-phase reaction of 2'-hydroxyacetophenone and benzaldehyde over SO3H-SBA-15 catalysts: Influence of microwave and thermal effects. *Microporous Mesoporous Mater.,* 2008, vol. 112, 97
- **Y. SATOH ; Y. YOKOYAMA ; I. OGINO ; S. R. MUKAI.** *Industrial & Engineering Chemistry Research,* 2013, vol. 52, 15293-15297
- **Z. CHEN ; R. XU ; Y. ZHANG ; N. GU.** *Nanoscale Research Letters,* 2008, vol. 4, 204-209
- **J. TIAN ; L. V. SARAF ; B. SCHWENZER ; S. M. TAYLOR ; E. K. BRECHIN ; J. LIU ; S. J. DALGARNO ; P. K. THALLAPALLY.** *J. Am. Chem. Soc.,* 2012, vol. 134, 9581-9584
- **A. KARMAKAR ; A. V. DESAI ; S. K. GHOSH.** *Coord. Chem. Rev.,* 2016, vol. 307, 313-341
- **D. KARAMI ; S. ROHANI.** *Industrial & Engineering Chemistry Research,* 2009, vol. 48, 4837-4843
- **DING, S.-Y. ; WANG, W.** Covalent organic frameworks (COFs): from design to applications. *Chem. Soc. Rev.,* 2013, vol. 42, 548-568
- **SINDELAR, V. et al.** Supramolecular Assembly of 2,7-Dimethyldiazapyrenium and Cucurbit[8]uril: A New Fluorescent Host for Detection of Catechol and Dopamine. *Chemistry - A European Journal,* 2005, vol. 11, 7054-7059